(19) Europäisches Patentamt / European Patent Office / Office européen des brevets

(11) **EP 3 712 242 A1**

(12) **EUROPEAN PATENT APPLICATION**
published in accordance with Art. 153(4) EPC

(43) Date of publication:
**23.09.2020 Bulletin 2020/39**

(51) Int Cl.:
*C12M 1/00* *(2006.01)*    *C12N 15/10* *(2006.01)*
*C12Q 1/686* *(2018.01)*

(21) Application number: **18875894.0**

(22) Date of filing: **13.11.2018**

(86) International application number:
**PCT/JP2018/042044**

(87) International publication number:
**WO 2019/093530 (16.05.2019 Gazette 2019/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 13.11.2017  JP 2017218552
21.11.2017  JP 2017224014
24.11.2017  JP 2017226081
24.11.2017  JP 2017226082
24.11.2017  JP 2017226084
24.11.2017  JP 2017226086

(71) Applicant: **Ricoh Company, Ltd.**
**Tokyo 143-8555 (JP)**

(72) Inventors:
• **KAWASHIMA, Yudai**
  **Tokyo 143-8555 (JP)**
• **HASHIMOTO, Michie**
  **Tokyo 143-8555 (JP)**
• **SUZUKI, Koei**
  **Tokyo 143-8555 (JP)**
• **OSAKI, Yusuke**
  **Tokyo 143-8555 (JP)**
• **UNNO, Hirotaka**
  **Tokyo 143-8555 (JP)**
• **SEO, Manabu**
  **Tokyo 143-8555 (JP)**
• **IZUMI, Satoshi**
  **Tokyo 143-8555 (JP)**

(74) Representative: **Fairbairn, Angus Chisholm**
**Marks & Clerk LLP**
**15 Fetter Lane**
**London EC4A 1BW (GB)**

(54) **DEVICE FOR DETECTION DETERMINATION**

(57)    Provided is a detection determining device used in a detection determining method including, in detection of a testing target in a sample by amplification of the testing target and an amplifiable reagent, determining that the testing target is present and a detection result is positive when the amplifiable reagent is amplified and the testing target is amplified, and determining that the testing target is absent or at least less than a specific copy number of the amplifiable reagent and a detection result is negative when the amplifiable reagent is amplified and the testing target is not amplified, the detection determining device including at least one well, wherein the amplifiable reagent in the at least one well is contained in a specific copy number.

FIG. 3

**Description**

Technical Field

The present invention relates to a detection determining device.

Background Art

In recent years, increased sensitivity of analytical techniques has enabled measurement of measurement targets in unit of copy number, and industrial application of gene detection techniques for detecting trace nucleic acids to foods, environmental audits, and medical care has been demanded. Particularly, detection of pathogens or unapproved genetically modified foods is often intended for confirming absence in analytes, and a detection accuracy of a high level is demanded.

For example, owing to the technological characteristics of polymerase chain reactions (PCR) often used in the field of molecular biology studies, polymerase chain reactions (PCR) are said to be theoretically capable of amplifying a nucleic acid even if there is only one copy of nucleic acid.

In the detection of such trace genes by quantitative analyses, there is a need for using standard reagents. A proposed method dilutes DNA fragments having a specific base sequence by a limiting dilution method and selects a diluted solution including the intended copy number based on the result of real-time PCR of the obtained diluted solutions (for example, see Patent document 1).

Another proposed method introduces DNA fragments by a specific copy number into cells by a gene recombination technique, cultures the cells, and isolates the cultured cells to produce a standard reagent containing the DNA fragments in the intended copy number (for example, see Patent document 2).

Quantitative PCR methods are techniques for appropriately detecting levels of fluorescence corresponding to DNA amplifications in the process of a polymerase chain reaction (PCR), and for indirectly determining the initial quantity of DNA, cDNA, or RNA. Such quantitative analyses need calibration curves indicating relationships between nucleic acid sample series and values measured from the nucleic acid sample series.

In order to obtain a correct quantitative value, it is reported that variation of individual measured values, expressed by the coefficient of variation CV, needs to be 20% or lower, that a series of nucleic acid samples needs to include three or more levels, and that five or more measurements need to be obtained per level (for example, see Non-patent document 1 and Non-patent document 2). Such a series of nucleic acid samples are produced by serial dilution of a nucleic acid sample having a known concentration.

For example, a proposed PCR reaction plate container is obtained by producing a series of nucleic acid samples by the serial dilution method and sealing a plurality of different filling copy number levels of nucleic acid samples in a plurality of sample filling portions of a container provided with the sample filling portions (for example, see Patent document 3).

A technique proposed recently fractionates cells into which a target nucleic acid sequence is introduced, one cell by one cell with a manipulator, to enable measurement and filling of very trace nucleic acid molecules (for example, see Patent document 2).

Polymerase chain reactions (PCR) and quantitative polymerase chain reactions (qPCR) are used for, for example, qualitative evaluation and quantitative evaluation of nucleic acids. Lately, PCR is also used for, for example, negative tests for genetically modified crops or foods (GMO) and negative tests for virus contamination. Therefore, assurance of reliability of the results is needed.

For ensuring the results, assurance of device performance and management of measuring systems have been carried out based on device temperature control management and users' maintenance management.

A series of nucleic acid samples used for qualitative evaluation and quantitative evaluation of nucleic acids are produced by serial dilution of a nucleic acid sample having a known concentration. For example, a proposed method dilutes DNA fragments having a specific base sequence by a limiting dilution method and selects a diluted solution including the intended copy number based on the result of real-time PCR of the obtained diluted solutions (for example, see Patent document 1).

A proposed standard nucleic acid kit is obtained by sealing standard nucleic acid solutions serially diluted to different concentrations in a plurality of sample filling portions (for example, see Patent document 2).

Further, for management of the measuring systems, there is a proposal to ensure the results of measurement based on temperature management during PCR reactions (for example, see Non-patent document 3).

In recent years, real-time polymerase chain reactions (real-time PCR) have become increasingly important in qualitative and quantitative determination in genetic testing. Performance evaluation for real-time polymerase chain reactions, particularly, inter-device or inter-facility performance evaluation is important. Currently, calibration curves based on external standards utilizing absorbance are used. However, these calibration curves do not accurately define the absolute numbers of nucleic acids. Particularly, the failure to accurately define the absolute numbers is considerably influential in the low copy number region.

A series of nucleic acid samples for such calibration curves are produced by serial dilution of a nucleic acid sample having a known concentration. For example, a proposed method dilutes DNA fragments having a specific base sequence by a limiting dilution method and selects a diluted solution including the intended copy number based on the result of real-time PCR of the obtained diluted solutions (for example, see Patent document 1).

A method proposed recently introduces DNA fragments by a specific copy number into cells by a gene recombination technique, cultures the cells, and isolates the cultured cells by manipulation, to prepare a sample containing one copy of DNA having an intended base sequence (for example, see Patent document 2).

In recent years, increased sensitivity of analytical techniques has enabled measurement of measurement targets in unit of the number of molecules, and industrial application of gene detection techniques for detecting trace nucleic acids to foods, environmental audits, and medical care has been demanded. Particularly, detection of pathogens, viruses, or unapproved genetically modified foods is often intended for confirming absence in analyte samples, and high-level detection and determination of the detection result are demanded.

Polymerase chain reaction (PCR) methods are used in detection of pathogens and diagnoses of pathological conditions for infectious diseases, contamination tests for genetically modified crops, and genetic diagnoses in negative tests for viruses. The PCR methods are techniques for amplifying DNA stepwise, and can specifically amplify an arbitrary partial base sequence from an analyte sample. Therefore, the PCR methods are widely used in, for example, genetic testing.

In testing of an analyte sample, when a target nucleic acid is not detected from the sample, the detection result is determined as negative. However, in the case of the negative determination, problematically, it has been impossible to ascertain whether the testing target nucleic acid is actually absent in the analyte sample, i.e., whether the negative determination is correct, or whether the nucleic acid is actually present but erroneously determined as absent (negative) due to failure of identifying, i.e., whether the detection result is false-negative.

Hence, various PCR testing methods have been proposed for avoiding false-negative determinations.

A disclosed microbial detection method performs a PCR reaction by adding two different pairs of primer sets, namely a first pair of primers and a second pair of primers in one reaction system (for example, see Patent document 4).

A disclosed DNA detection method synthesizes DNA by amplification from the same primer as the primer for amplifying a certain portion of intended DNA in a manner that the DNA to be synthesized can be distinguished from DNA of the intended portion by, for example, the base length, in order to overcome, for example, the false-negative problem based on PCR performed by adding the synthesized DNA and PCR performed without adding the synthesized DNA (for example, see Patent document 5).

Particularly, norovirus is a pathogen contained in bivalves such as oyster, and known as the cause of winter gastroenteritis and food poisoning. Norovirus is very strongly transmittable and infectious to other people through feces or vomit excreted by the person infected with winter gastroenteritis or food poisoning. Hence, there is a need for tests for strictly identifying the pathogen, which is the source of infection, in order to prevent spread of infection of winter gastroenteritis and food poisoning.

Polymerase chain reaction (PCR) methods are commonly used for testing norovirus. The PCR methods are techniques for amplifying nucleic acids stepwise, and can specifically amplify an arbitrary partial base sequence from an analyte sample. Therefore, the PCR methods are widely used.

Various methods have been proposed for detecting testing targets in analyte samples using the PCR methods.

A proposed quantitative sensing method quantifies the abundance ratio of gene recombinants in a sample containing a plurality of gene recombinant systems (for example, see Patent document 6).

Another proposed method produces a standard sample containing the same target sequence as a DNA fragment including the target sequence in a specific number of molecules (copies) (for example, see Patent document 2).

Citation List

Patent Document

Patent document 1: Japanese Unexamined Patent Application Publication No. 2014-33658
Patent document 2: Japanese Unexamined Patent Application Publication No. 2015-195735
Patent document 3: Japanese Unexamined Patent Application Publication No. 2008-141965
Patent document 4: Japanese Unexamined Patent Application Publication No. 2011-223940
Patent document 5: Japanese Unexamined Patent Application Publication No. 09-224699
Patent document 6: International Publication No. WO 2002/0349439

Non-Ptent Document

Non-patent document 1: U.S. Food and Drug Administration. "Guidance for Industry: Bioanalytical Method Validation.": <http://www.fda.gov/downloads/Drugs/Guidance Compliance Regulatory Information/Guidances/UCM070107.pdf>, cited 5 September, 2014.
Non-patent document 2: European Medicines Agency. "Guideline on Bioanalytical Method Validation.": <http://www.ema.europa.eu/docs/en_GB/document_library/Scientific_guideline/2011/08/WC 500109686.pdf>, cited 5 September, 2014.
Non-patent document 3: ISO/TS 20836:2005

Summary of Invention

Technical Problem

The present invention has an object to provide a detection determining device that, in detection of a testing target contained in a sample, enables detection determination improved in negative determination accuracy, with a capability of more securely avoiding false-negative causing situations particularly when the copy number of the testing target is low.

Solution to Problem

A detection determining device is used in a detection determining method including a determining step of, in detection of a testing target in a sample by amplification of the testing target and an amplifiable reagent, determining that the testing target is present and a detection result is positive when the amplifiable reagent is amplified and the testing target is amplified, and determining that the testing target is absent or at least less than a specific copy number of the amplifiable reagent and a detection result is negative when the amplifiable reagent is amplified and the testing target is not amplified. The detection determining device includes at least one well, and the amplifiable reagent contained in a specific copy number in the at least one well.

Effects of the Invention

The present invention can provide a detection determining device that, in detection of a testing target contained in a sample, enables detection determination improved in negative determination accuracy, with a capability of more securely avoiding false-negative causing situations particularly when the copy number of the testing target is low.

Brief Description of the Drawings

FIG. 1 is a graph plotting a relationship between a copy number having variation according to a Poisson distribution and a coefficient of variation CV;
FIG. 2 is a perspective view illustrating an example of a device of the present invention;
FIG. 3 is a side view illustrating an example of a device of the present invention;
FIG. 4 is a diagram illustrating an example of positions of wells to be filled with an amplifiable reagent in a device of the present invention;
FIG. 5 a diagram illustrating another example of positions of wells to be filled with an amplifiable reagent in a device of the present invention;
FIG. 6 is a perspective view illustrating an example of a device of the present invention;
FIG. 7 is a perspective view illustrating another example of a device of the present invention;
FIG. 8 is a side view of FIG. 7.
FIG. 9 is a perspective view illustrating another example of a device of the present invention;
FIG. 10 a diagram illustrating an example of positions of wells to be filled with an amplifiable reagent in a device of the present invention;
FIG. 11 is a diagram illustrating another example of positions of wells to be filled with an amplifiable reagent in a device of the present invention;
FIG. 12 is a block diagram illustrating an example hardware configuration of a detection determining device;

FIG. 13 is a diagram illustrating an example functional configuration of a detection determining device;

FIG. 14 is a flowchart illustrating an example of a process performed according to a detection determining program;

FIG. 15 is a graph plotting a relationship between an average specific copy number x and a coefficient of variation CV in specific copy number ranges of less than 100 and 100 or greater;

FIG. 16 is a perspective view illustrating another example of a device of the present invention;

FIG. 17 is a diagram illustrating example positioning of wells to be filled with an amplifiable reagent in a device of the present invention;

FIG. 18 is a diagram illustrating another example positioning of wells to be filled with an amplifiable reagent in a device of the present invention;

FIG. 19 is a diagram illustrating example sequences of nucleic acids to be introduced into cells when introducing a nucleic acid that expresses a norovirus GI genotype and a nucleic acid that expresses a norovirus GII genotype;

FIG. 20 is a plan view illustrating an example of a device of the present invention;

FIG. 21 is a plan view illustrating another example of a device of the present invention;

FIG. 22 is a plan view illustrating another example of a device of the present invention;

FIG. 23 is a plan view illustrating another example of a device of the present invention;

FIG. 24 is a plan view illustrating another example of a device of the present invention;

FIG. 25 is a graph plotting an example calibration curve generated using the device of FIG. 24;

FIG. 26 is a plan view illustrating another example of a device of the present invention;

FIG. 27 is a plan view illustrating another example of a device of the present invention;

FIG. 28 is a plan view illustrating another example of a device of the present invention;

FIG. 29 is a plan view illustrating another example of a device of the present invention;

FIG. 30 is a plan view illustrating another example of a device of the present invention;

FIG. 31 is a block diagram illustrating an example hardware configuration of a preparator's skill evaluating device;

FIG. 32 is a diagram illustrating an example functional configuration of a preparator's skill evaluating device;

FIG. 33 is a flowchart illustrating an example of a process performed according to a preparator's skill evaluating program;

FIG. 34 is a block diagram illustrating an example hardware configuration of a testing device performance evaluating device;

FIG. 35 is a diagram illustrating an example functional configuration of a testing device performance evaluating device;

FIG. 36 is a flowchart illustrating an example of a process performed according to a testing device performance evaluating program;

FIG. 37 is a graph plotting an example of a relationship between the frequency and the fluorescence intensity of cells in which DNA replication has occurred;

FIG. 38A is an exemplary diagram illustrating an example of an electromagnetic valve-type discharging head;

FIG. 38B is an exemplary diagram illustrating an example of a piezo-type discharging head;

FIG. 38C is an exemplary diagram illustrating a modified example of the piezo-type discharging head illustrated in FIG. 38B;

FIG. 39A is an exemplary graph plotting an example of a voltage applied to a piezoelectric element;

FIG. 39B is an exemplary graph plotting another example of a voltage applied to a piezoelectric element;

FIG. 40A is an exemplary diagram illustrating an example of a liquid droplet state;

FIG. 40B is an exemplary diagram illustrating an example of a liquid droplet state;

FIG. 40C is an exemplary diagram illustrating an example of a liquid droplet state;

FIG. 41 is a schematic diagram illustrating an example of a dispensing device configured to land liquid droplets sequentially into wells;

FIG. 42 is an exemplary diagram illustrating an example of a liquid droplet forming device;

FIG. 43 is a diagram illustrating hardware blocks of a control unit of the liquid droplet forming device of FIG. 42;

FIG. 44 is a diagram illustrating functional blocks of a control unit of the liquid droplet forming device of FIG. 42;

FIG. 45 is a flowchart illustrating an example of an operation of a liquid droplet forming device;

FIG. 46 is an exemplary diagram illustrating a modified example of a liquid droplet forming device;

FIG. 47 is an exemplary diagram illustrating another modified example of a liquid droplet forming device;

FIG. 48A is a diagram illustrating a case where two fluorescent particles are contained in a flying liquid droplet;

FIG. 48B is a diagram illustrating a case where two fluorescent particles are contained in a flying liquid droplet;

FIG. 49 is a graph plotting an example of a relationship between a luminance Li when particles do not overlap each other and a luminance Le actually measured;

FIG. 50 is an exemplary diagram illustrating another modified example of a liquid droplet forming device;

FIG. 51 is an exemplary diagram illustrating another example of a liquid droplet forming device;

FIG. 52 is an exemplary diagram illustrating an example of a method for counting cells that have passed through a micro-flow path;

FIG. 53 is an exemplary diagram illustrating an example of a method for capturing an image of a portion near a nozzle portion of a discharging head;

FIG. 54 is a graph plotting a relationship between a probability P (>2) and an average cell number;

FIG. 55 is a graph illustrating an example of amplification conditions;

FIG. 56A is a diagram illustrating a result of agarose electrophoresis of a sample (1) performed after PCR amplification of the sample in a negative test for norovirus in a shellfish in Example 2, where the sample (1) is prepared on a 96-well plate by discharging 10 cells (copies) of 600G yeast by IJ and adding a norovirus-containing sample to the resultant (Example of the present invention);

FIG. 56B is a diagram illustrating a result of agarose electrophoresis of a sample (2) performed after PCR amplification of the sample, where the sample (2) is prepared to contain norovirus only;

FIG. 56C is a diagram illustrating a result of agarose electrophoresis of a sample (3) performed after PCR amplification of the sample in a negative test for norovirus in a shellfish in Example 2, where the sample (3) is prepared on a 96-well plate by diluting 600G plasmid, dispensing the resultant by an amount corresponding to 10 copies per well by a manual operation, and adding a norovirus-containing sample to the resultant (Comparative Example to IJ);

FIG. 57 is a diagram illustrating example positioning of nucleic acid samples in Examples and Comparative Examples of the present invention;

FIG. 58 is a graph plotting results of quantitative PCR in Examples of the present invention;

FIG. 59 is a graph plotting results of quantitative PCR in Comparative Examples of the present invention;

FIG. 60 is a diagram illustrating an example of a temperature control method in real-time PCR;

FIG. 61 is a diagram illustrating an example plate on which real-time PCR is performed;

FIG. 62 is a diagram illustrating an example of results of FIG. 61;

FIG. 63 is a diagram illustrating an example of results of FIG. 61; and

FIG. 64 is a diagram illustrating an example of results of FIG. 61.

Mode for Carrying out the Invention

(Detection determining device)

<First embodiment>

A detection determining device of the present invention is used in a detection determining method including a determining step of, in detection of a testing target in a sample by amplification of the testing target and an amplifiable reagent, determining that the testing target is present and a detection result is positive when the amplifiable reagent is amplified and the testing target is amplified, and determining that the testing target is absent or at least less than a specific copy number of the amplifiable reagent and a detection result is negative when the amplifiable reagent is amplified and the testing target is not amplified. The detection determining device includes at least one well, and the amplifiable reagent contained in the at least one well in a specific copy number, preferably further includes an identifier unit and a base material, and further includes other members as needed.

In the present specification, "the detection determining device" may simply be referred to as, for example, "device".

Existing standard samples, which, in terms of the contents of nucleic acids, may have a Poisson distribution-based random variation that occurs when picking up the analyte from a sample containing the nucleic acid in a low copy number, have not been able to improve the accuracy of testing instruments per se.

Existing low-copy-number nucleic acid standard substances used in accuracy management have not indicated the uncertainty that may have occurred when producing the low-copy-number nucleic acid standard substances, and may not have been able to ensure reliability of the accuracy management.

Use of the device of the present invention makes it possible to evaluate reliability of measurement results when detecting an intended reagent from a sample containing an amplifiable reagent in a low copy number. In the present invention, "a low copy number" means that the copy number of an amplifiable reagent is low.

Patent document 4 intends to ensure success or failure of an experiment process of target gene detection and avoid false-negative determination due to failure of the experiment, by performing the experiment adding two different pairs of primer sets in one reaction system. However, Patent document 4 does not define the copy number of reference DNA used as control. Therefore, only success or failure of the experiment process is ensured, and false-negative due to other causes, for example, the target gene present in less than or equal to the limit of detection of the experiment process (described in detail below), cannot be avoided. That is, the method has not been enough as a testing method that can more securely avoid false-negative causing situations.

For example, when a very trace analyte sample is used (i.e., when the copy number of the testing target contained in the sample is low), it has been impossible to ascertain which of the following cases is pertinent to failure to detect the testing target and determination that "the testing target is absent" (negative). That is, the method of Patent document 4 has not been able to ascertain whether the testing target is absent in the analyte sample (negative) or whether the testing target is present but cannot be identified and is erroneously determined as negative (false-negative).

Patent document 5 synthesizes DNA by amplification from the same pair of primers as the primers of the intended DNA, in a manner that the DNA to be synthesized can be distinguished from the intended DNA by, for example, the base length and the base sequence of the amplified product. The technique intends to avoid false-negative determination by performing PCR with addition of the synthesized DNA and performing PCR without addition of the synthesized DNA. However, Patent document 5 does not define the copy number of the synthesized reference DNA. Hence, the technique is not satisfactory as a testing method that enables avoiding false-negative determination without fail. For example, when a very trace analyte sample is used (i.e., when the copy number of the testing target contained in the sample is low), it has been impossible to ascertain which of the following cases is pertinent to failure to detect the testing target and determination that "the testing target is absent" (negative). That is, the method of Patent document 5 has not been able to ascertain whether the testing target is absent in the analyte sample (negative) or whether the testing target is present but cannot be identified and is erroneously determined as negative (false-negative).

Hence, the present invention provides a device that can be suitably used in a testing method that can more infallibly avoid false-negative causing situations and better improve negative determination accuracy even when a very trace analyte sample is used (i.e., when the copy number of the testing target is low).

The present invention uses a device that includes at least one sample filling well to be filled with an amplifiable reagent by a specific copy number at a certain accuracy, and includes in each well, the amplifiable reagent dispensed at a certain coefficient of variation or higher.

A copy number means the number of target or specific base sequences in an amplifiable reagent contained in the well.

The target base sequence refers to a base sequence including defined base sequences in at least primer and probe regions. Specifically, a base sequence having a defined total length is also referred to as specific base sequence.

A specific copy number refers to the aforementioned copy number that specifies the number of target base sequences at accuracy of a certain level or higher.

This means that the specific copy number is known as the number of target base sequences actually contained in a well. That is, the specific copy number in the present invention is more accurate or reliable as a number than a predetermined copy number (calculated estimated value) obtained according to existing serial dilution methods, and is a controlled value that has no dependency on a Poisson distribution even if the value is within a low copy number region of 1,000 or lower in particular. When it is said that the specific copy number is a controlled value, it is preferable that a coefficient of variation CV expressing uncertainty roughly satisfy either $CV < 1/\sqrt{x}$ with respect to an average copy number x or CV≤20%. Hence, use of a device including wells in which a target base sequence is contained in the specific copy number makes it possible to perform qualitative or quantitative testing of samples containing the target base sequence more accurately than ever.

When the number of target base sequences and the number of nucleic acid molecules including the sequence coincide with each other, "copy number" and "number of molecules" may be associated with each other.

Specifically, for example, in the case of norovirus, when the number of viruses is 1, the number of nucleic acid molecules is 1 and the specific copy number is 1. In the case of yeast at a GI phase, when the number of yeast cells is 1, the number of nucleic acid molecules (the number of same chromosomes) is 1 and the specific copy number is 1. In the case of human cell at a G0/GI phase, when the number of human cells is 1, the number of nucleic acid molecules (the number of same chromosomes) is 2 and the copy number is 2.

Further, in the case of yeast at a GI phase having the target base sequence introduced at two positions, when the number of yeast cells is 1, the number of nucleic acid molecules (the number of same chromosomes) is 1 and the copy number is 2.

In the present invention, a specific copy number of the amplifiable reagent may also be referred to as absolute number of the amplifiable reagent.

The specific copy number of the amplifiable reagent is preferably 1 copy or greater but 1,000 copies or less, preferably 100 copies or less, more preferably 20 copies or less, and yet more preferably 10 copies or less.

It is preferable that the specific copy number of the amplifiable reagent includes two or more different integers.

Examples of combination of specific copy numbers of the amplifiable reagent include a combination of 1, 2, 3, 4, 5, 6, 7, 8, 9, and 10, a combination of 1, 3, 5, 7, and 9, and a combination of 2, 4, 6, 8, and 10.

Combination of specific copy numbers of the amplifiable reagent may be, for example, a combination of four levels, namely, 1, 10, 100, and 1,000. Calibration curves can be generated using the device of the present invention with a combination of a plurality of different specific copy numbers.

When there are a plurality of wells containing the amplifiable reagent, it is at least needed that the amplifiable reagent be contained in the wells in the same specific copy number.

When it is said that the amplifiable reagent is contained in the wells in the same specific copy number, it is meant that the variation in the number of amplifiable reagents is within a tolerable range, where the variation occurs when the amplifiable reagents are filled in the device. Whether the variation in the number of amplifiable reagents is within the tolerable range can be determined based on information on uncertainty described below.

Information on the specific copy number of the amplifiable reagent is not particularly limited and may be appropriately selected depending on the intended purpose so long as the information is information regarding the amplifiable reagent in the device. Examples of the information include information on uncertainty, information on a carrier described below, and information on the amplifiable reagent.

"Uncertainty" is defined in ISO/IEC Guide 99:2007 [International Vocabulary of Metrology-Basics and general concepts and related terms (VIM)] as "a parameter that characterizes measurement result-incidental variation or dispersion of values rationally linkable to the measured quantity".

Here, "values rationally linkable to the measured quantity" means candidates for the true value of the measured quantity. That is, uncertainty means information on the variation of the results of measurement due to operations and devices involved in production of a measurement target. With a greater uncertainty, a greater variation is predicted in the results of measurement.

For example, the uncertainty may be standard deviation obtained from the results of measurement, or a half value of a reliability level, which is expressed as a numerical range in which the true value is contained at a predetermined probability or higher.

The uncertainty may be calculated according to the methods based on, for example, Guide to the Expression of Uncertainty in Measurement (GUM:ISO/IEC Guide 98-3), and Japan Accreditation Board Note 10, Guideline on Uncertainty in Measurement in Test. As the method for calculating the uncertainty, for example, there are two types of applicable methods: a type-A evaluation method using, for example, statistics of the measured values, and a type-B evaluation method using information on uncertainty obtained from, for example, calibration certificate, manufacturer's specification, and information open to the public.

All uncertainties due to factors such as operations and measurement can be expressed by the same reliability level, by conversion of the uncertainties to standard uncertainty. Standard uncertainty indicates variation in the average value of measured values.

In an example method for calculating the uncertainty, for example, factors that may cause uncertainties are extracted, and uncertainties (standard deviations) due to the respective factors are calculated. Then, the calculated uncertainties due to the respective factors are synthesized according to the sum-of-squares method, to calculate a synthesized standard uncertainty. In the calculation of the synthesized standard uncertainty, the sum-of-squares method is used. Therefore, a factor that causes a sufficiently small uncertainty can be ignored, among the factors that cause uncertainties.

In the device of the present invention, a coefficient of variation of the amplifiable reagent filled in the wells may be used as the information on the uncertainty.

The coefficient of variation means a relative value of the variation in the number of cells (or the number of amplifiable reagents) filled in each concave, where the variation occurs when cells are filled in the concave. That is, the coefficient of variation means the filling accuracy in terms of the number of cells (or amplifiable reagents) filled in the concave. The coefficient of variation is a value obtained by dividing standard deviation $\sigma$ by an average value x. Here, the coefficient of variation CV is assumed to be a value obtained by dividing standard deviation $\sigma$ by an average copy number (average number of copies filled) x. In this case, a relational expression represented by Formula 1 below is established.

$$CV = \frac{\sigma}{x} \qquad \cdots \text{Formula 1}$$

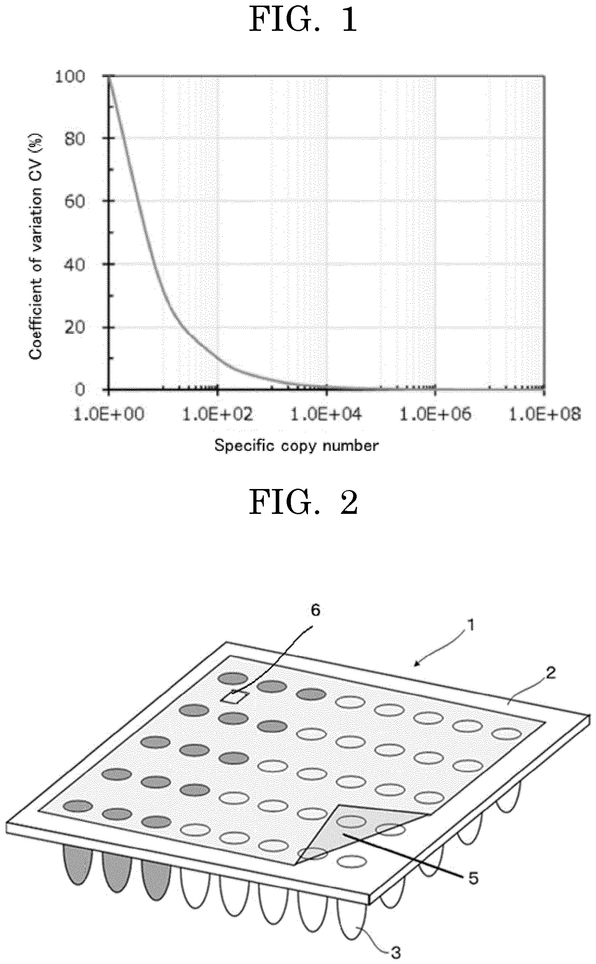

Generally, cells (or amplifiable reagents) have a random distribution state of a Poisson distribution in a dispersion liquid. Therefore, in a random distribution state by a serial dilution method, i.e., of a Poisson distribution, standard deviation $\sigma$ can be regarded as satifying a relational expression represented by Formula 2 below with an average copy number x. Hence, in the case where a dispersion liquid of cells (or amplifiable reagents) is diluted by a serial dilution method, when coefficients of variation CV (CV values) for average copy numbers x are calculated according to Formula 3 below derived from Formula 1 above and Formula 2 based on the standard deviation $\sigma$ and the average copy numbers x, the results are as presented in Table 1 and FIG. 1. The coefficient of variation CV for a copy number having variation according to a Poisson distribution can be obtained from FIG. 1.

$$\sigma = \sqrt{x}$$

$$\cdots \text{Formula 2}$$

$$CV = \frac{1}{\sqrt{x}}$$

$$\cdots \text{Formula 3}$$

Table 1

| Average copy number x | Coefficient of variation CV |
|---|---|
| 1.00E+00 | 100.00% |
| 1.00E+01 | 31.62% |
| 1.00E+02 | 10.00% |
| 1.00E+03 | 3.16% |
| 1.00E+04 | 1.00% |
| 1.00E+05 | 0.32% |
| 1.00E+06 | 0.10% |
| 1.00E+07 | 0.03% |
| 1.00E+08 | 0.01% |

From the results of Table 1 and FIG. 1, it can be understood that when a well is to be filled with, for example, a copy number of 100 of amplifiable reagents according to a serial dilution method, the final copy number of amplifiable reagents to be filled in the reaction solution has a coefficient of variation (CV) of at least 10%, even when other accuracies are ignored.

As regards the copy number of the amplifiable reagent, it is preferable that the coefficient of variation CV and an average specific copy number x of the amplifiable reagent satisfy a relationship: $CV < 1/\sqrt{x}$, and more preferably satisfy $CV < 1/2\sqrt{x}$.

As regards the information on the uncertainty, it is preferable that the device include a plurality of wells in each of which the amplifiable reagent is contained, and that the information on the uncertainty include information on uncertainty of the device as a whole based on the specific copy number of the amplifiable reagent contained in each well.

There are some conceivable factors that cause uncertainties. For example, in a production process of introducing the intended amplifiable reagent into cells and dispensing the cells while counting the number of cells, examples of the conceivable factors include the number of amplifiable reagents in a cell (for example, the cell cycle of the cell), the unit configured to locate the cells in the device (including any outcomes of operations of an inkjet device or each section of the device, such as operation timings of the device, and the number of cells included in a liquid droplet when the cell suspension is formed into the form of a liquid droplet), the frequency at which cells are located at appropriate positions of the device (for example, the number of cells located in a well), and contamination due to destruction of cells in a cell suspension and consequent mixing of the amplifiable reagent into the cell suspension (hereinafter may also be described as mixing of contaminants).

Examples of information on the number of the amplifiable reagent as the information on the amplifiable reagent include information on uncertainty of the number of amplifiable reagents contained in the device.

<Well>

For example, the shape, the number, the volume, the material, and the color of the well are not particularly limited and may be appropriately selected depending on the intended purpose.

The shape of the well is not particularly limited and may be appropriately selected depending on the intended purpose so long as, for example, a nucleic acid can be located in the well. Examples of the shape of the well include: concaves such as a flat bottom, a round bottom, a U bottom, and a V bottom; and sections on a substrate.

The number of wells is at least one, preferably a plural number of 2 or greater, more preferably 5 or greater, and yet more preferably 50 or greater.

Examples of a product in which the number of wells is one include a PCR tube.

For example, a multi-well plate is suitably used as a product in which the number of wells is 2 or greater.

Examples of the multi-well plate include a 24-well, 48-well, 96-well, 384-well, or 1,536-well plate.

The volume of the well is not particularly limited, may be appropriately selected depending on the intended purpose, and is preferably 1 microliter or greater but 1,000 microliters or less in consideration of the amount of a sample used in a common nucleic acid testing device.

The material of the well is not particularly limited and may be appropriately selected depending on the intended purpose. Examples of the material of the well include polystyrene, polypropylene, polyethylene, fluororesins, acrylic resins, polycarbonate, polyurethane, polyvinyl chloride, and polyethylene terephthalate.

Examples of the color of the well include transparent colors, semi-transparent colors, chromatic colors, and complete light-shielding colors.

Wettability of the well is not particularly limited and may be appropriately selected depending on the intended purpose. The wettability of the well is preferably water repellency. When the wettability of the well is water repellency, adsorption of the amplifiable reagent to the internal wall of the well can be reduced. Further, when the wettability of the well is water repellency, the amplifiable reagent, a primer, and an amplifying reagent in the well can be moved in a state of a solution.

The method for imparting water repellency to the internal wall of the well is not particularly limited and may be appropriately selected depending on the intended purpose. Examples of the method include a method of forming a fluororesin coating film, a fluorine plasma treatment, and an embossing treatment. Particularly, by applying a water repellency imparting treatment that imparts a contact angle of 100 degrees or greater, it is possible to suppress reduction of the amplifiable reagent due to spill of the liquid and suppress increase of uncertainty (or coefficient of variation).

<Base material>

The device is preferably a plate-shaped device obtained by providing a well in a base material, but may be linking-type well tubes such as 8-series tubes.

For example, the material, the shape, the size, and the structure of the base material are not particularly limited and may be appropriately selected depending on the intended purpose.

The material of the base material is not particularly limited and may be appropriately selected depending on the intended purpose. Examples of the material of the base material include semiconductors, ceramics, metals, glass, quartz glass, and plastics. Among these materials, plastics are preferable.

Examples of the plastics include polystyrene, polypropylene, polyethylene, fluororesins, acrylic resins, polycarbonate, polyurethane, polyvinyl chloride, and polyethylene terephthalate.

The shape of the base material is not particularly limited and may be appropriately selected depending on the intended purpose. For example, board shapes and plate shapes are preferable.

The structure of the base material is not particularly limited, may be appropriately selected depending on the intended purpose, and may be, for example, a single-layer structure or a multilayered structure.

<Identifier unit>

It is preferable that the device include an identifier unit that enables identifying information on the specific copy number of the amplifiable reagent and the uncertainty of the specific copy number.

The identifier unit is not particularly limited and may be appropriately selected depending on the intended purpose. Examples of the identifier unit include a memory, an IC chip, a barcode, a QR code (registered trademark), a Radio Frequency Identifier (hereinafter may also be referred to as "RFID"), color coding, and printing.

The position at which the identifier unit is provided and the number of identifier units are not particularly limited and may be appropriately selected depending on the intended purpose.

Examples of the information to be stored in the identifier unit include not only the information on the specific copy number of the amplifiable reagent and the uncertainty of the specific copy number, but also results of analyses (for example, activity value and emission intensity), the number of amplifiable reagents (for example, the number of cells), whether cells are alive or dead, a copy number of a specific base sequence, which of a plurality of wells is filled with the amplifiable reagent, the kind of the amplifiable reagent, the measurement date and time, and the name of the person in charge of measurement. The information stored in the identifier unit can be read with various kinds of reading units. For example, when the identifier unit is a barcode, a barcode reader is used as the reading unit.

The method for writing information in the identifier unit is not particularly limited and may be appropriately selected depending on the intended purpose. Examples of the method include manual input, a method of directly writing data through a liquid droplet forming device configured to count the number of amplifiable reagents during dispensing of the amplifiable reagents into the wells, transfer of data stored in a server, and transfer of data stored in a cloud system.

<Other members>

The other members are not particularly limited and may be appropriately selected depending on the intended purpose. Examples of the other members include a sealing member.

*-Sealing member-

It is preferable that the device include a sealing member in order to prevent mixing of foreign matters into the wells and outflow of the filled materials.
It is preferable that the sealing member be configured to be capable of sealing at least one well and separable at a perforation in order to be capable of sealing or opening each one of the wells individually.
The shape of the sealing member is preferably a cap shape matching the inner diameter of a well, or a film shape for covering the well opening.
Examples of the material of the sealing member include polyolefin resins, polyester resins, polystyrene resins, and polyamide resins.
It is preferable that the sealing member have a film shape that can seal all wells at a time. It is also preferable that the sealing member be configured to have different adhesive strengths for wells that need to be reopened and wells that need not, in order that the user can reduce improper use.
It is preferable that the well contain at least any one selected from a primer and an amplifying reagent.
A primer is a synthetic oligonucleotide having a complementary base sequence that includes 18 through 30 bases and is specific to a template DNA of a polymerase chain reaction (PCR). A pair of primers, namely a forward primer and a reverse primer, are set at two positions in a manner to sandwich the region to be amplified.
Examples of the amplifying reagent for, for example, a polymerase chain reaction (PCR) include enzymes such as DNA polymerase, matrices such as the four kinds of bases (dGTP, dCTP, dATP, and dTTP), $Mg^{2+}$ (2 mM magnesium chloride), and a buffer for maintaining the optimum pH (pH of from 7.5 through 9.5).
It is preferable that the device include a negative control well in which the copy number of the amplifiable reagent is 0, and a positive control well in which the copy number of the amplifiable reagent is 10 or greater.
Detection sensed in the negative control and non-detection sensed in the positive control suggest abnormality of the detection system (the reagent or the device). With the negative control and the positive control provided, the user can immediately recognize a problem when the problem occurs, and can stop the measurement and inspect the root of the problem.
The state of the amplifiable reagent, a primer, and an amplifying reagent in the well is not particularly limited and may be appropriately selected depending on the intended purpose. For example, the state of the amplifiable reagent, a primer, and an amplifying reagent may be a state of either a solution or a solid. In terms of convenience of use, the state of the amplifiable reagent, a primer, and an amplifying reagent is particularly preferably a state of a solution. In a state of a solution, a user can use the amplifiable reagent, a primer, and an amplifying reagent for a test immediately. In terms of transportation, the state of the amplifiable reagent, a primer, and an amplifying reagent is particularly preferably a state of a solid and more preferably a dry state. In a solid dry state, a reaction speed at which the amplifiable reagent is decomposed by, for example, a breakdown enzyme, can be reduced, and storage stability of the amplifiable reagent, a primer, and an amplifying reagent can be improved.
It is preferable that the amplifiable reagent, a primer, and an amplifying reagent be filled in appropriate amounts in the device in the solid dry state, in order to make it possible to use the amplifiable reagent, a primer, and an amplifying reagent in the form of a reaction solution immediately by dissolving the amplifiable reagent, a primer, and an amplifying reagent in a buffer or water immediately before use of the device.
The drying method is not particularly limited and may be appropriately selected depending on the intended purpose. Examples of the drying method include freeze drying, heating drying, hot-air drying, vacuum drying, steam drying, suction drying, infrared drying, barrel drying, and spin drying.

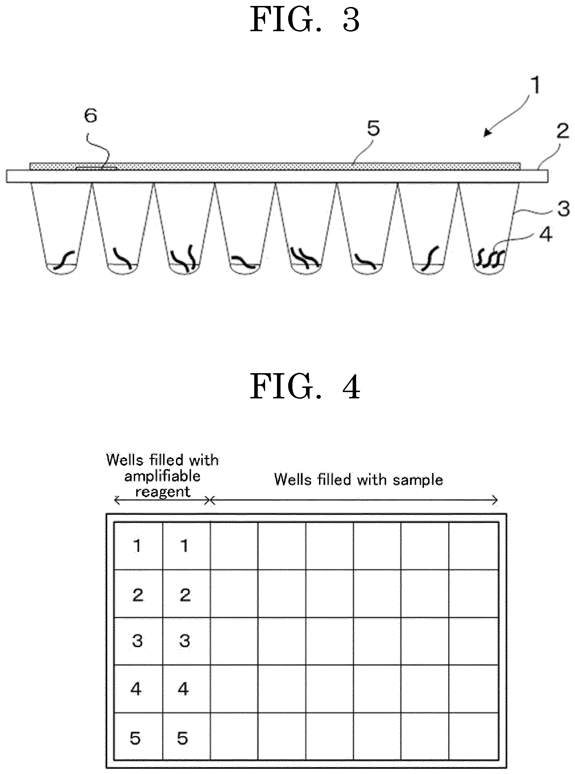

FIG. 2 is a perspective view illustrating an example of the device 1 of the present invention. FIG. 3 is a side view of the device 1 of FIG. 2. In the device 1, a plurality of wells 3 are provided in a base material 2, and a nucleic acid 4 serving as the amplifiable reagent is filled in specific copy numbers in the wells 3 (internal spatial regions enclosed by the well wall surfaces constituting the wells). Information on the specific copy numbers of the amplifiable reagent and the uncertainty of the specific copy numbers of the amplifiable reagent is associated with this device 1. FIG. 2 and FIG. 3 illustrate an example in which the openings of the wells 3 of the device 1 are covered with a sealing member 5.
For example, as illustrated in FIG. 2 and FIG. 3, an IC chip or a barcode (identifier unit 6) storing the information on the number of the reagent filled in each well 3 and the uncertainty (or certainty) of the number, or information associated with these kinds of information is placed at a position that is between the sealing member 5 and the base material 2 and does not overlap the openings of the wells. This is suitable for preventing, for example, unintentional alteration of the identifier unit.

With the identifier unit, the device can be distinguished from a common well plate that does not have an identifier unit. Therefore, confusion or mistake can be prevented.

FIG. 4 is a diagram illustrating an example of the positions of the wells to be filled with the amplifiable reagent in the device of the present invention. The numerals in the wells in FIG. 4 indicate the specific copy numbers in which the amplifiable reagent is contained. The wells with no numerals in FIG. 4 are wells for samples and control measurement.

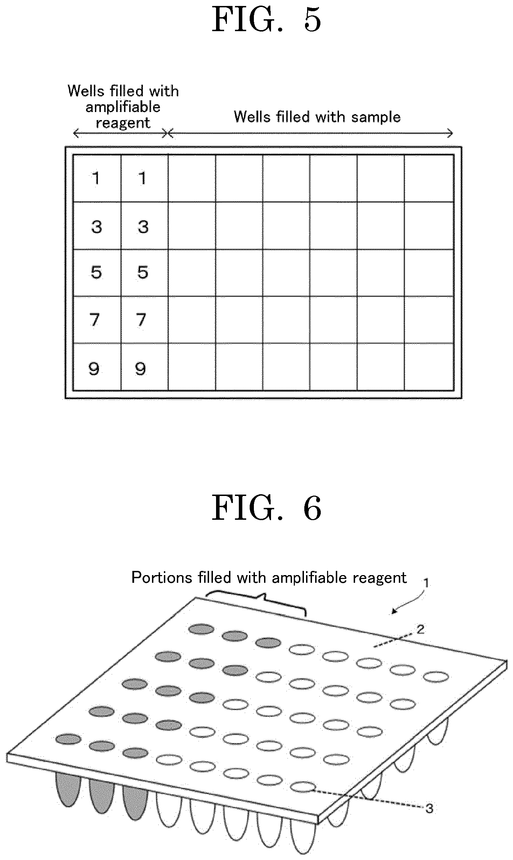

FIG. 5 is a diagram illustrating another example of the positions of the wells to be filled with the amplifiable reagent in the device of the present invention. The numerals in the wells in FIG. 5 indicate the specific copy numbers in which the amplifiable reagent is contained. The wells with no numerals in FIG. 5 are wells for samples and control measurement.

It is preferable that the amplifiable reagent be a nucleic acid. It is preferable that the nucleic acid be incorporated into the nucleus of a cell.

-Nucleic acid-

A nucleic acid means a polymeric organic compound in which a nitrogen-containing base derived from purine or pyrimidine, sugar, and phosphoric acid are bonded with one another regularly. Examples of the nucleic acid also include a fragment of a nucleic acid or an analog of a nucleic acid or of a fragment of a nucleic acid.

The nucleic acid is not particularly limited and may be appropriately selected depending on the intended purpose. Examples of the nucleic acid include DNA, RNA, and cDNA.

The nucleic acid or nucleic acid fragment may be a natural product obtained from a living thing, or a processed product of the natural product, or a product produced by utilizing a genetic recombination technique, or a chemically synthesized artificially synthesized nucleic acid. One of these nucleic acids may be used alone or two or more of these nucleic acids may be used in combination. With an artificially synthesized nucleic acid, it is possible to reduce impurities and set the molecular weight to a low level. This makes it possible to improve the initial reaction efficiency.

An artificially synthesized nucleic acid means an artificially synthesized nucleic acid produced to have the same constituent components (base, deoxyribose, and phosphoric acid) as naturally existent DNA or RNA. Examples of the artificially synthesized nucleic acid include not only a nucleic acid having a base sequence coding a protein, but also a nucleic acid having an arbitrary base sequence.

Examples of the analog of a nucleic acid or a nucleic acid fragment include a nucleic acid or a nucleic acid fragment bonded with a non-nucleic acid component, a nucleic acid or a nucleic acid fragment labeled with a labeling agent such as a fluorescent dye or an isotope (e.g., a primer or a probe labeled with a fluorescent dye or a radioisotope), and an artificial nucleic acid, which is a nucleic acid or a nucleic acid fragment in which the chemical structure of some of the constituent nucleotides is changed (e.g., PNA, BNA, and LNA).

The form of the nucleic acid is not particularly limited and may be appropriately selected depending on the intended purpose. Examples of the form of the nucleic acid include double-strand nucleic acid, single-strand nucleic acid, and partially double-strand or single-strand nucleic acid. Cyclic or straight-chain plasmids can also be used.

The nucleic acid may be modified or mutated.

It is preferable that the nucleic acid have a target base sequence. The term "specific" means "particularly specified".

The target base sequence is not particularly limited and may be appropriately selected depending on the intended purpose. Examples of the target base sequence include base sequences used for infectious disease testing, naturally non-existent non-natural base sequences, animal cell-derived base sequences, plant cell-derived base sequences, fungal cell-derived base sequences, bacterium-derived base sequences, and virus-derived base sequences. One of these base sequences may be used alone or two or more of these base sequences may be used in combination.

When using a non-natural base sequence, the target base sequence preferably has a GC content of 30% or higher but 70% or lower, and preferably has a constant GC content (for example, see SEQ ID NO. 1).

The base length of the target base sequence is not particularly limited, may be appropriately selected depending on the intended purpose, and may be, for example, a base length of 20 base pairs (or mer) or greater but 10,000 base pairs (or mer).

When using a base sequence used for infectious disease testing, the base sequence is not particularly limited and may be appropriately selected depending on the intended purpose so long as the base sequence includes a base sequence specific to the intended infectious disease. It is preferable that the base sequence include a base sequence designated in official analytical methods or officially announced methods (for example, see SEQ ID NOS. 2 and 3).

The nucleic acid may be a nucleic acid derived from the cells to be used, or a nucleic acid introduced by transgenesis. When a nucleic acid introduced by transgenesis and a plasmid are used as the nucleic acid, it is preferable to confirm that one copy of the nucleic acid is introduced per cell. The method for confirming that one copy of the nucleic acid is introduced is not particularly limited and may be appropriately selected depending on the intended purpose. Examples of the method include a sequencer, a PCR method, and a Southern blotting method.

One kind or two or more kinds of nucleic acids having specific base sequences may be introduced by transgenesis. Also in the case of introducing only one kind of a nucleic acid by transgenesis, base sequences of the same kind may be introduced in tandem depending on the intended purpose.

The method for transgenesis is not particularly limited and may be appropriately selected depending on the intended purpose so long as the method can introduce an intended copy number of specific nucleic acid sequences at an intended position. Examples of the method include homologous recombination, CRISPR/Cas9, CRISPR/Cpf1, TALEN, Zinc finger nuclease, Flip-in, and Jump-in. In the case of yeast fungi, homologous recombination is preferable among these methods in terms of a high efficiency and ease of controlling.

-Carrier-

It is preferable to handle the amplifiable reagent in a state of being carried on a carrier. When the amplifiable reagent is a nucleic acid, a preferable form is the nucleic acid being carried (or more preferably encapsulated) by the carrier having a particle shape (carrier particles).

The carrier is not particularly limited and may be appropriately selected depending on the intended purpose. Examples of the carrier include a cell, a resin, liposome, and microcapsule.

-Cells--

A cell means a structural, functional unit that includes an amplifiable reagent (for example, a nucleic acid) and forms an organism.

The cells are not particularly limited and may be appropriately selected depending on the intended purpose. All kinds of cells can be used regardless of whether the cells are eukaryotic cells, prokaryotic cells, multicellular organism cells, and unicellular organism cells. One of these kinds of cells may be used alone or two or more of these kinds of cells may be used in combination.

The eukaryotic cells are not particularly limited and may be appropriately selected depending on the intended purpose. Examples of the eukaryotic cells include animal cells, insect cells, plant cells, fungi, algae, and protozoans. One of these kinds of eukaryotic cells may be used alone or two or more of these kinds of eukaryotic cells may be used in combination. Among these eukaryotic cells, animal cells and fungi are preferable.

Adherent cells may be primary cells directly taken from tissues or organs, or may be cells obtained by passaging primary cells directly taken from tissues or organs a few times. Adherent cells may be appropriately selected depending on the intended purpose. Examples of adherent cells include differentiated cells and undifferentiated cells.

Differentiated cells are not particularly limited and may be appropriately selected depending on the intended purpose. Examples of differentiated cells include: hepatocytes, which are parenchymal cells of a liver; stellate cells; Kupffer cells; endothelial cells such as vascular endothelial cells, sinusoidal endothelial cells, and corneal endothelial cells; fibroblasts; osteoblasts; osteoclasts; periodontal ligament-derived cells; epidermal cells such as epidermal keratinocytes; epithelial cells such as tracheal epithelial cells, intestinal epithelial cells, cervical epithelial cells, and corneal epithelial cells; mammary glandular cells; pericytes; muscle cells such as smooth muscle cells and myocardial cells; renal cells; pancreatic islet cells; nerve cells such as peripheral nerve cells and optic nerve cells; chondrocytes; and bone cells.

Undifferentiated cells are not particularly limited and may be appropriately selected depending on the intended purpose. Examples of undifferentiated cells include: pluripotent stem cells such as embryotic stem cells, which are undifferentiated cells, and mesenchymal stem cells having pluripotency; unipotent stem cells such as vascular endothelial progenitor cells having unipotency; and iPS cells.

Fungi are not particularly limited and may be appropriately selected depending on the intended purpose. Examples of fungi include molds and yeast fungi. One of these kinds of fungi may be used alone or two or more of these kinds of fungi may be used in combination. Among these kinds of fungi, yeast fungi are preferable because the cell cycles are adjustable and monoploids can be used.

The cell cycle means a cell proliferation process in which cells undergo cell division and cells (daughter cells) generated by the cell division become cells (mother cells) that undergo another cell division to generate new daughter cells.

Yeast fungi are not particularly limited and may be appropriately selected depending on the intended purpose. For example, yeast fungi that are synchronously cultured to synchronize at a G0/G1 phase, and fixed at a G1 phase are preferable.

Further, for example, as yeast fungi, Bar1-deficient yeasts with enhanced sensitivity to a pheromone (sex hormone) that controls the cell cycle at a G1 phase are preferable. When yeast fungi are Bar1-deficient yeasts, the abundance ratio of yeast fungi with uncontrolled cell cycles can be reduced. This makes it possible to, for example, prevent a specific nucleic acid from increasing in number in the cells contained in a well.

The prokaryotic cells are not particularly limited and may be appropriately selected depending on the intended purpose. Examples of the prokaryotic cells include eubacteria and archaea. One of these kinds of prokaryotic cells may be used alone or two or more of these kinds of prokaryotic cells may be used in combination.

As the cells, dead cells are preferable. With dead cells, it is possible to prevent occurrence of cell division after fractionation.

As the cells, cells that can emit light upon reception of light are preferable. With cells that can emit light upon reception of light, it is possible to land the cells into wells while having a highly accurate control on the number of cells.

Reception of light means receiving of light.

An optical sensor means a passive sensor configured to collect, with a lens, any light in the range from visible light rays visible by human eyes to near infrared rays, short-wavelength infrared rays, and thermal infrared rays that have longer wavelengths than the visible light rays, to obtain, for example, shapes of target cells in the form of image data.

--Cells that can emit light upon reception of light--

The cells that can emit light upon reception of light are not particularly limited and may be appropriately selected depending on the intended purpose so long as the cells can emit light upon reception of light. Examples of the cells include cells stained with a fluorescent dye, cells expressing a fluorescent protein, and cells labeled with a fluorescent-labeled antibody.

A cellular site stained with a fluorescent dye, expressing a fluorescent protein, or labeled with a fluorescent-labeled antibody is not particularly limited. Examples of the cellular site include a whole cell, a cell nucleus, and a cellular membrane.

--Fluorescent dye--

Examples of the fluorescent dye include fluoresceins, azo dyes, rhodamines, coumarins, pyrenes, cyanines. One of these fluorescent dyes may be used alone or two or more of these fluorescent dyes may be used in combination. Among these fluorescent dyes, fluoresceins, azo dyes, and rhodamines are preferable, and eosin, Evans blue, trypan blue, rhodamine 6G, rhodamine B, and rhodamine 123 are more preferable.

As the fluorescent dye, a commercially available product may be used. Examples of the commercially available product include product name: EOSIN Y (available from Wako Pure Chemical Industries, Ltd.), product name: EVANS BLUE (available from Wako Pure Chemical Industries, Ltd.), product name: TRYPAN BLUE (available from Wako Pure Chemical Industries, Ltd.), product name: RHODAMINE 6G (available from Wako Pure Chemical Industries, Ltd.), product name: RHODAMINE B (available from Wako Pure Chemical Industries, Ltd.), and product name: RHODAMINE 123 (available from Wako Pure Chemical Industries, Ltd.).

--Fluorescent protein--

Examples of the fluorescent protein include Sirius, EBFP, ECFP, mTurquoise, TagCFP, AmCyan, mTFP1, MidoriishiCyan, CFP, TurboGFP, AcGFP, TagGFP, Azami-Green, ZsGreen, EmGFP, EGFP, GFP2, HyPer, TagYFP, EYFP, Venus, YFP, PhiYFP, PhiYFP-m, TurboYFP, ZsYellow, mBanana, KusabiraOrange, mOrange, TurboRFP, DsRed-Express, DsRed2, TagRFP, DsRed-Monomer, AsRed2, mStrawberry, TurboFP602, mRFP1, JRed, KillerRed, mCherry, mPlum, PS-CFP, Dendra2, Kaede, EosFP, and KikumeGR. One of these fluorescent proteins may be used alone or two or more of these fluorescent proteins may be used in combination.

--Fluorescent-labeled antibody--

The fluorescent-labeled antibody is not particularly limited and may be appropriately selected depending on the intended purpose so long as the fluorescent-labeled antibody is fluorescent-labeled. Examples of the fluorescent-labeled antibody include CD4-FITC and CD8-PE. One of these fluorescent-labeled antibodies may be used alone or two or more of these fluorescent-labeled antibodies may be used in combination.

The volume average particle diameter of the cells is preferably 30 micrometers or less, more preferably 10 micrometers or less, and particularly preferably 7 micrometers or less in a free state. When the volume average particle diameter of the cells is 30 micrometers or less, the cells can be suitably used in an inkjet method or a liquid droplet discharging unit such as a cell sorter.

The volume average particle diameter of the cells can be measured by, for example, a measuring method described below.

Ten microliters is extracted from a produced stained yeast dispersion liquid and poured onto a plastic slide formed of PMMA. Then, with an automated cell counter (product name: COUNTESS AUTOMATED CELL COUNTER, available from Invitrogen), the volume average particle diameter of the cells can be measured. The cell number can be obtained by a similar measuring method.

The concentration of the cells in a cell suspension is not particularly limited, may be appropriately selected depending on the intended purpose, and is preferably $5 \times 10^4$ cells/mL or higher but $5 \times 10^8$ cells/mL or lower and more preferably $5 \times 10^4$ cells/mL or higher but $5 \times 10^7$ cells/mL or lower. When the cell number is $5 \times 10^4$ cells/mL or higher but $5 \times 10^8$ cells/mL or lower, it can be ensured that cells be contained in a discharged liquid droplet without fail. The cell number can be measured with an automated cell counter (product name: COUNTESS AUTOMATED CELL COUNTER, available from Invitrogen) in the same manner as measuring the volume average particle diameter.

The cell number of cells including a nucleic acid is not particularly limited and may be appropriately selected depending on the intended purpose so long as the cell number is a plural number.

-Resin-

The material, the shape, the size, and the structure of the resin are not particularly limited and may be appropriately selected depending on the intended purpose so long as the resin can carry the amplifiable reagent (for example, a nucleic acid).

- Liposome-

A liposome is a lipid vesicle formed of a lipid bilayer containing lipid molecules. Specifically, the liposome means a lipid-containing closed vesicle including a space separated from the external environment by a lipid bilayer produced based on the polarities of a hydrophobic group and a hydrophilic group of lipid molecules.

The liposome is a closed vesicle formed of a lipid bilayer using a lipid, and contains an aqueous phase (internal aqueous phase) in the space in the closed vesicle. The internal aqueous phase contains, for example, water. The liposome may be single-lamellar (single-layer lamellar or unilamellar with a single bilayer) or multilayer lamellar (multilamellar, with an onion-like structure including multiple bilayers, with the individual layers separated by watery layers).

As the liposome, a liposome that can encapsulate an amplifiable reagent (for example, a nucleic acid) is preferable. The form of encapsulation is not particularly limited. "Encapsulation" means a form of a nucleic acid being contained in the internal aqueous phase and the layer of the liposome. Examples of the form include a form of encapsulating a nucleic acid in the closed space formed of the layer, a form of encapsulating a nucleic acid in the layer per se, and a combination of these forms.

The size (average particle diameter) of the liposome is not particularly limited so long as the liposome can encapsulate an amplifiable reagent (for example, a nucleic acid). It is preferable that the liposome have a spherical form or a form close to the spherical form.

The component (layer component) constituting the lipid bilayer of the liposome is selected from lipids. As the lipid, an arbitrary lipid that can dissolve in a mixture solvent of a water-soluble organic solvent and an ester-based organic solvent can be used. Specific examples of the lipid include phospholipids, lipids other than phospholipids, cholesterols, and derivatives of these lipids. These components may be formed of a single kind of a component or a plurality of kinds of components.

-Microcapsule-

A microcapsule means a minute particle having a wall material and a hollow structure, and can encapsulate an amplifiable reagent (for example, a nucleic acid) in the hollow structure.

The microcapsule is not particularly limited, and, for example, the wall material and the size of the microcapsule may be appropriately selected depending on the intended purpose.

Examples of the wall material of the microcapsule include polyurethane resins, polyurea, polyurea-polyurethane resins, urea-formaldehyde resins, melamine-formaldehyde resins, polyamide, polyester, polysulfone amide, polycarbonate, polysulfinate, epoxyr, acrylic acid ester, methacrylic acid ester, vinyl acetate, and gelatin. One of these wall materials may be used alone or two or more of these wall materials may be used in combination.

The size of the microcapsule is not particularly limited and may be appropriately selected depending on the intended purpose so long as the microcapsule can encapsulate an amplifiable reagent (for example, a nucleic acid).

The method for producing the microcapsule is not particularly limited and may be appropriately selected depending on the intended purpose. Examples of the method include an in-situ method, an interfacial polymerization method, and a coacervation method.

It is preferable that the sample filling well contain at least any one of a primer and an amplifying reagent.

A primer is a synthetic oligonucleotide having a complementary base sequence that includes 18 through 30 bases and is specific to a template DNA of a polymerase chain reaction (PCR). A pair of primers, namely a forward primer and a reverse primer, are set at two positions in a manner to sandwich the region to be amplified.

Examples of the amplifying reagent for, for example, a polymerase chain reaction (PCR) include enzymes such as DNA polymerase, matrices such as the four kinds of bases (dGTP, dCTP, dATP, and dTTP), $Mg^{2+}$ (2 mM magnesium chloride), and a buffer for maintaining the optimum pH (pH of from 7.5 through 9.5).

The base sequence of the amplifiable reagent may be different from the base sequence of the testing target. This is a preferable mode for performing amplification reactions of the testing target and the amplifiable reagent in the same well.

Because the base sequence of the amplifiable reagent and the base sequence of the testing target are different from each other, a preferable mode is a mode in which a pair of primers for amplifying the testing target and a pair of primers for amplifying the amplifiable reagent are introduced into the sample filling well.

The device of the present invention has a mode in which a positive control having the same bas sequence as the base sequence of the testing target is filled in a predetermined amount in a different well from the sample filling well. Here, the predetermined amount needs at least to be a sufficiently detectable amount. With a positive control filled in a different well, it can be ensured more reliably that the determinations in the case of (1) and (2) in Table 2 are correct, provided that amplification of the positive control is observed.

The device of the present invention includes at least one sample filling well, preferably includes an identifier unit and a base material, and further includes other members as needed.

In the present invention, a well to be filled with a positive control may be provided in a plate in addition to a well used for sample filling. In the following, general description of wells including the sample filling well will be provided.

The detection determining device of the present invention is suitably used particularly for genetic testing in which the testing target is a virus, a bacterium, or animal species identification of edible meat.

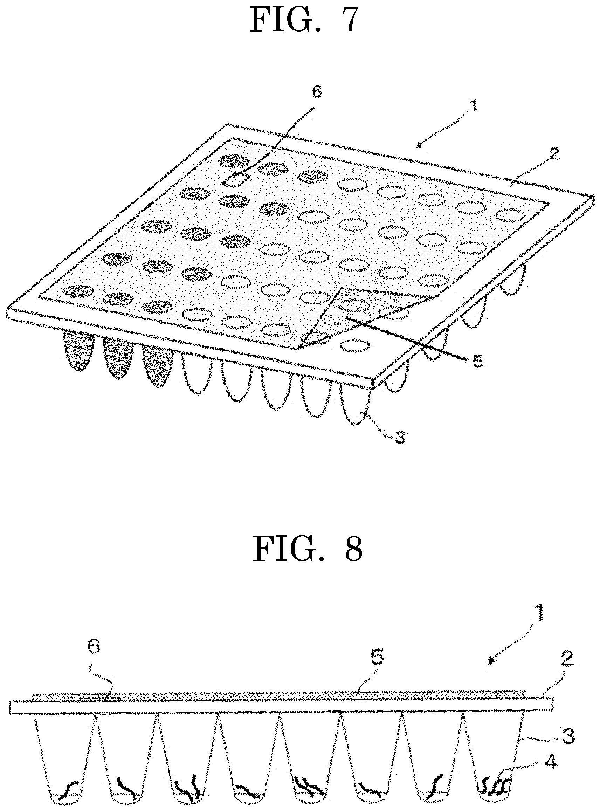

FIG. 6 is a perspective view illustrating an example of the device of the present invention. FIG. 7 is a perspective view illustrating another example of the device of the present invention. FIG. 8 is a side view of the device of FIG. 7.

In the device 1, a plurality of wells 3 are provided in a base material 2, and a nucleic acid 4 serving as the amplifiable reagent is filled in specific copy numbers in the wells 3 (internal spatial regions enclosed by the well wall surfaces constituting the wells). Information on the absolute numbers of amplifiable reagents and the uncertainty of the absolute numbers of amplifiable reagents is associated with this device 1. FIG. 7 and FIG. 8 illustrate an example in which the openings of the wells 3 of the device 1 are covered with a sealing member 5.

For example, as illustrated in FIG. 7 and FIG. 8, an IC chip or a barcode (identifier unit 6) storing the information on the number of the reagent filled in each well 3 and the uncertainty (or certainty) of the number, or information associated with these kinds of information is placed at a position that is between the sealing member 5 and the base material 2 and does not overlap the openings of the wells. This is suitable for preventing, for example, unintentional alteration of the identifier unit.

With the identifier unit, the device can be distinguished from a common well plate that does not have an identifier unit. Therefore, confusion or mistake can be prevented.

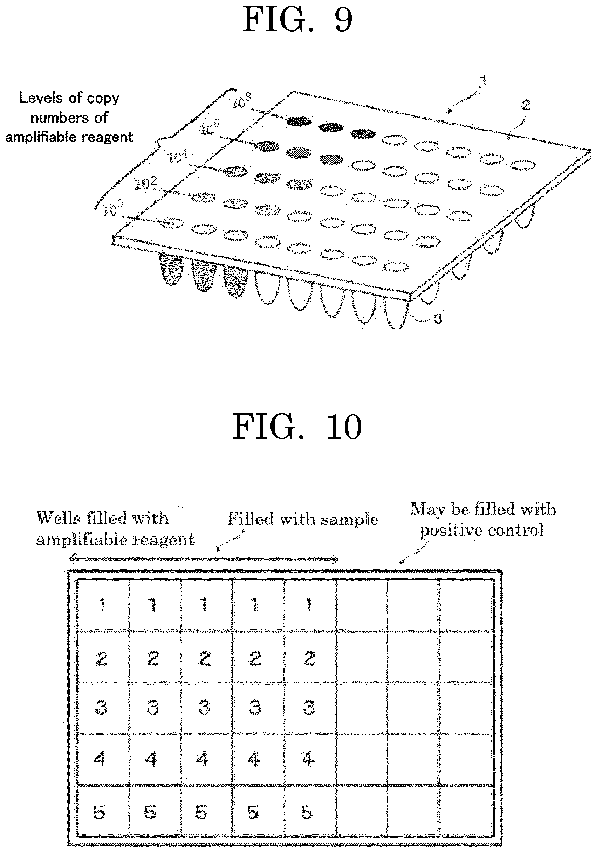

FIG. 9 is a perspective view illustrating another example of the device of the present invention. The device of FIG. 9 is provided with five levels of 1, 2, 3, 4, and 5 as the levels of the specific copy number of the amplifiable reagent.

FIG. 10 is a diagram illustrating an example of the positions of the wells to be filled with the amplifiable reagent in the device of the present invention. The numerals in the wells in FIG. 10 indicate the specific copy numbers in which the amplifiable reagent is contained. The wells with no numerals in FIG. 10 may be filled with, for example, a positive control.

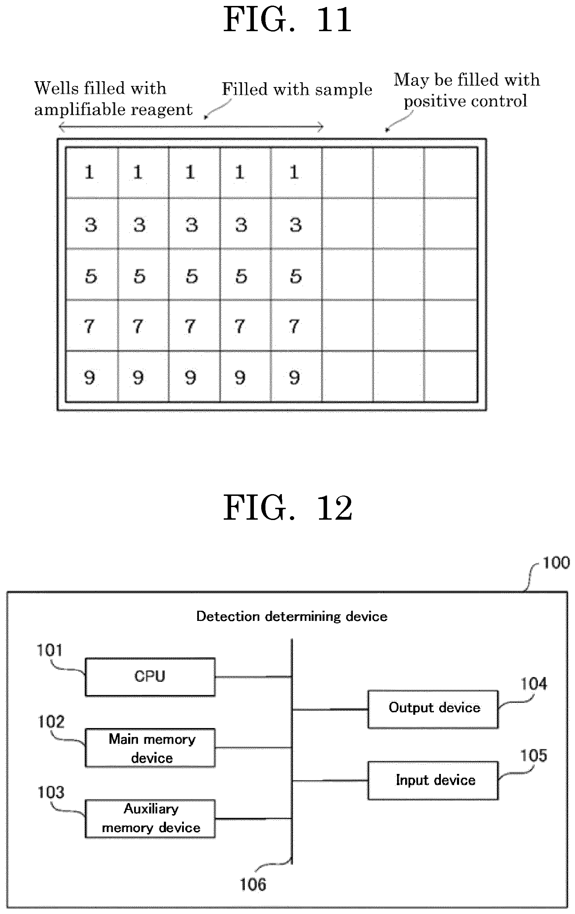

FIG. 11 is a diagram illustrating another example of the positions of the wells to be filled with the amplifiable reagent in the device of the present invention. The numerals in the wells in FIG. 11 indicate the specific copy numbers in which the amplifiable reagent is contained. The wells with no numerals in FIG. 11 may be filled with, for example, a positive control.

A detection determining method using the detection determining device of the present invention, and the detection determining device and a detection determining program that can be suitably used in the detection determining method will be described in detail.

[Detection determining method, detection determining device, and detection determining program]

The detection determining method is a detection determining method used in detection of a testing target in a sample by amplification of the testing target and an amplifiable reagent. The amplifiable reagent is provided in a specific copy number. The detection determining method includes a determining step of determining that the testing target is present and a detection result is positive when the amplifiable reagent is amplified and the testing target is amplified, and determining that the testing target is absent or at least less than the specific copy number of the amplifiable reagent and a detection result is negative when the amplifiable reagent is amplified and the testing target is not amplified, preferably includes an obtaining step of obtaining a result of amplification of the amplifiable reagent and a result of amplification of the testing target and an analyzing step of analyzing the result of amplification of the amplifiable reagent and the result of amplification of the testing target, and further includes other steps as needed.

The detection determining device is a detection determining device used in detection of a testing target in a sample by amplification of the testing target and an amplifiable reagent. The amplifiable reagent is provided in a specific copy number. The detection determining device includes a determining unit configured to determine that the testing target is present and a detection result is positive when the amplifiable reagent is amplified and the testing target is amplified, and determine that the testing target is absent or at least less than the specific copy number of the amplifiable reagent and a detection result is negative when the amplifiable reagent is amplified and the testing target is not amplified, preferably includes an obtaining unit configured to obtain a result of amplification of the amplifiable reagent and a result of amplification of the testing target and an analyzing unit configured to analyze the result of amplification of the amplifiable reagent and the result of amplification of the testing target, and further includes other units as needed.

The detection determining program is a detection determining program used in detection of a testing target in a sample by amplification of the testing target and an amplifiable reagent. The amplifiable reagent is provided in a specific copy number. The detection determining program preferably causes a computer to execute a process including determining that the testing target is present and a detection result is positive when the amplifiable reagent is amplified and the testing target is amplified, and determining that the testing target is absent or at least less than the specific copy number of the amplifiable reagent and a detection result is negative when the amplifiable reagent is amplified and the testing target is not amplified.

Control being performed by, for example, a control unit of the detection determining device has the same meaning as the detection determining method being carried out. Therefore, details of the detection determining method will also be specified through description of the detection determining device. Further, the detection determining program realizes the detection determining device with the use of, for example, computers as hardware resources. Therefore, details of the detection determining program will also be specified through description of the detection determining device.

The detection determining method, the detection determining device, and the detection determining program are based on the premise that the present invention uses a device including wells into which an amplifiable reagent in a specific copy number is dispensed at a certain accuracy and with a coefficient of variation higher than or equal to a certain level. Detailed description of the device will be provided below.

Detection of a testing target in a sample using the device of the present invention makes it possible to more securely avoid a false-negative determination in the detection of the testing target contained in the sample, particularly when the copy number of the testing target is low.

When a detection result is negative, the present invention ensures that the testing target, even if present, is at least less than the specific copy number of the amplifiable reagent. That is, the present invention provides assurance from a quantitative point of view to an ambiguous "negative" determination result that the testing target is absent.

The detection determination of the present invention can work more effectively for a sample containing a testing target in a low copy number. For example, the specific copy number of the testing target is preferably 200 or less, more preferably 100 or less, and particularly preferably 10 or less. That is, the specific copy number of the testing target of 1, 2, 3, 4, 5, 6, 7, 8, 9, or 10 is particularly preferable. As the testing target, a nucleic acid is preferable because a nucleic acid can be amplified with existing techniques.

-Determining step and determining unit-

The determining step is a step of using an amplifiable reagent in a specific copy number, determining that a testing target is present and a detection result is positive when the amplifiable reagent is amplified and the testing target is amplified, and determining that the testing target is absent or at least less than the specific copy number of the amplifiable reagent and a detection result is negative when the amplifiable reagent is amplified and the testing target is not amplified, and is performed by the determining unit.

When the copy number of reference DNA used as control is not specified as in Japanese Unexamined Patent Application Publication No. 2011-223940 or Japanese Unexamined Patent Application Publication No. 09-224699 mentioned above, for example, determination about detection of the testing target made based on the result of amplification of the testing target and the result of amplification of the amplifiable reagent will result as presented in Table 2 below.

Table 2

| | | Amplifiable reagent, of which copy number is not specified | |
|---|---|---|---|
| | | + | - |
| Testing target in sample | + | Positive (highly probable) | Reconsideration of PCR reaction system and reconsideration of copy number of amplifiable reagent are needed |
| | - | Negative or false-negative (impossible to detect whether negative or false-negative) | |

As presented in Table 2, amplification reaction results include four patterns, namely (1) a case where amplification is observed in both of the nucleic acid in the sample and the reference nucleic acid serving as the amplifiable reagent, (2) a case where amplification is observed in the reference nucleic acid serving as the amplifiable reagent, but amplification is not observed in the nucleic acid in the sample, (3) a case where amplification is observed in the nucleic acid in the sample, but amplification is not observed in the reference nucleic acid serving as the amplifiable reagent, and (4) a case where amplification is observed in neither the nucleic acid in the sample nor the reference nucleic acid serving as the amplifiable reagent.

When the copy number of the amplifiable reagent is not specified as in Table 2, the results (1) to (4) described above can be determined as follows.

In the case of (1), it is possible to confirm that the experiment by PCR reaction has been successful because amplification is observed in the reference nucleic acid serving as the amplifiable reagent. Further, it is possible to confirm that the testing target nucleic acid is present in the sample because amplification is observed in the nucleic acid in the sample.

In the case of (2), it is possible to confirm that the experiment by PCR reaction has been successful because amplification is observed in the reference nucleic acid serving as the amplifiable reagent. However, because amplification is not observed in the nucleic acid in the sample, it is generally determined that the testing target nucleic acid is absent in the sample. However, because the copy number of the reference nucleic acid serving as the amplifiable reagent is not specified, it is impossible to specify which of the following cases is pertinent, namely a case where the testing target nucleic acid is truly absent in the sample (negative) and a case where the testing target nucleic acid is present in the sample but in a trace amount, and cannot be experimentally identified and was erroneously determined as negative (false-negative). Particularly, when the copy number of the nucleic acid is a low copy number, the determination of whether negative or false-negative is more difficult.

In the case of (3) and (4), because amplification is not observed in the reference nucleic acid serving as the amplifiable reagent, for example, it is estimated that the PCR reaction has not progressed due to some causes (for example, reaction temperature conditions, preparation of the amplifiable reagent, a thermal cycler, and settings of the real-time PCR device), or that the copy number of the amplifiable reagent is insufficient with respect to the limit of detection, and it is determined that "reconsideration of the PCR reaction system and the copy number of the amplifiable reagent is needed". When the copy number of the amplifiable reagent is not specified, the copy number has a large variation, and the probability that the copy number is higher than or equal to the limit of detection is low. This inevitably increases the frequency that the test results of (3) and (4) will be obtained. Therefore, when the copy number of the amplifiable reagent is not specified, there is a need for performing a test at a copy number that is twice or three times higher than the limit of detection.

On the other hand, when the copy number of the reference nucleic acid serving as the amplifiable reagent is specified as in the present invention, i.e., when the copy number is a specific copy number, for example, determination about detection of the testing target made based on the result of amplification of the testing target and the result of amplification of the amplifiable reagent will result as presented in Table 3 below.

Table 3

| | | Amplifiable reagent in a specific copy number | |
|---|---|---|---|
| | | + | - |
| Testing target in sample | + | Positive (ascertained) | Reconsideration of PCR reaction system and reconsideration of copy number of amplifiable reagent are needed |
| | - | Negative or at least less than specific copy number | |

When the copy number of the reference nucleic acid serving as the amplifiable reagent is specified as presented in Table 3, the results (1) to (4) described above can be determined as follows.

In the case of (1), it is possible to determine definitely that the experiment by PCR reaction has been successful because amplification is observed in the reference nucleic acid serving as the amplifiable reagent. Further, it is possible to determine definitely that the testing target nucleic acid is present in the sample because amplification is observed in the nucleic acid in the sample. Even though the copy number of the nucleic acid is a low copy number, the "positive" determination result can be ensured.

In the case of (2), it is possible to confirm that the experiment by PCR reaction has been successful because amplification is observed in the reference nucleic acid serving as the amplifiable reagent. However, because amplification is not observed in the nucleic acid in the sample, it is possible to determine definitely that the nucleic acid in the sample is at least less than the specific copy number of the amplifiable reagent. That is, it is possible to determine that the testing target nucleic acid is absent in the sample or at least less than the specific copy number of the amplifiable reagent, and that the detection result is "negative" meaning that the testing target is absent, or "at least less than the specific copy number". In the case of (2), it is impossible to specify whether negative or false-negative according to Table 3, whereas it is possible to conclude that the result is "negative" or "at least less than the specific copy number" as described above according to Table 3 of the present invention because the copy number of the reference nucleic acid serving as the amplifiable reagent is specified.

The present invention makes it possible to more securely exclude false-negative and improve the accuracy for negative determination. The present invention can reduce false-negative and ensure a "negative" determination result based on the reasoning that the testing target nucleic acid is at least less than the specific copy number of the amplifiable reagent.

In the case of (3) and (4), because amplification is not observed in the reference nucleic acid serving as the amplifiable reagent, for example, it is estimated that the PCR reaction has not progressed due to some causes (for example, reaction temperature conditions, preparation of the amplifiable reagent, a thermal cycler, and settings of the real-time PCR device), or that the copy number of the amplifiable reagent is insufficient with respect to the limit of detection, and it is determined that "reconsideration of the PCR reaction system and the copy number of the amplifiable reagent is needed".

In the detection determining method of the present invention, when there is a limit of detection by the copy number, it is preferable that the limit of detection of the testing target nucleic acid be comparable to the limit of detection of the nucleic acid serving as the amplifiable reagent.

This makes it possible to regard a limit of detection obtained based on a result of amplification of the reference nucleic acid serving as the amplifiable reagent as a limit of detection of the testing target nucleic acid.

In the detection determining method, it is preferable to perform amplification reactions of the testing target nucleic acid and the nucleic acid serving as the amplifiable reagent, using a device described below.

The device includes at least one sample filling well to be filled with a sample. The sample filling well further includes an amplifiable reagent in a specific copy number. The specific copy number of the amplifiable reagent is a specific natural number.

That is, it is more preferable to fill the amplifiable reagent in the sample filling well to be filled with a sample and perform amplification reactions of the testing target and the amplifiable reagent in the same sample filling well, using the device. By performing amplification reactions of the testing target and the amplifiable reagent in the same well, it is possible to suppress variations of reaction conditions and increase reliability of the results of amplification.

In the detection determining method, it is preferable to perform amplification reactions of the testing target and the amplifiable reagent, using nucleic acids having different base sequences from each other as the testing target and the amplifiable reagent.

Further, in the detection determining method, it is preferable to perform amplification reactions, by filling a positive control having the same base sequence as the testing target base sequence in a certain amount in a different well from the sample filling well. Here, the certain amount needs at least to be a sufficiently detectable amount.

With a positive control filled in a different well, it is ensured more reliably that the determinations in the case of (1) and (2) in Table 3 are correct, provided that amplification of the positive control is observed.

The device used in the detection determining method will be described in more detail below.

-Detection result obtaining step and detection result obtaining unit-

A detection result obtaining step is a step of obtaining a result of amplification of the nucleic acid serving as the amplifiable reagent and a result of amplification of the testing target nucleic acid, and is performed by a detection result obtaining unit.

A detection result obtaining unit 131 is configured to obtain a result of amplification of the nucleic acid serving as the amplifiable reagent and a result of amplification of the testing target nucleic acid obtained from PCR reactions. The data of the obtained results of amplification is stored in a detection result database 141.

-Detection result analyzing step and detection result analyzing unit-

A detection result analyzing step is a step of analyzing the obtained result of amplification of the nucleic acid serving as the amplifiable reagent and the obtained result of amplification of the testing target nucleic acid, and is performed by a detection result amplifying unit.

A detection result analyzing unit 132 is configured to obtain the data of the results of amplification stored in the detection result database 141, and based on the data, analyze whether amplification is observed in the nucleic acid serving as the amplifiable reagent and whether amplification is observed in the testing target nucleic acid.

The procedure of the detection determining program can be executed using a computer including a control unit constituting a detection determining device.

The hardware configuration and the functional configuration of the detection determining device will be described below.

-Hardware configuration of detection determining device-

FIG. 12 is a block diagram illustrating an example of the hardware configuration of a detection determining device 100.

As illustrated in FIG. 12, the detection determining device 100 includes units such as a CPU (Central Processing Unit) 101, a main memory device 102, an auxiliary memory device 103, an output device 104, and an input device 105. These units are coupled to one another through a bus 106.

The CPU 101 is a processing device configured to execute various controls and operations. The CPU 101 realizes various functions by executing OS (Operating System) and programs stored in, for example, the main memory device 102. That is, in the present example, the CPU 101 functions as a control unit 130 of the detection determining device 100 by executing the detection determining program.

The CPU 101 also controls the operation of the entire detection determining device 100. In the present example, the CPU 101 is used as the device configured to control the operation of the entire detection determining device 100. However, this is non-limiting. For example, FPGA (Field Programmable Gate Array) may be used.

The detection determining program and various databases need not indispensably be stored in, for example, the main memory device 102 and the auxiliary memory device 103. The detection determining program and various databases may be stored in, for example, another information processing device that is coupled to the detection determining device 100 through, for example, the Internet, a LAN (Local Area Network), and a WAN (Wide Area Network). The detection determining device 100 may receive the detection determining program and various databases from such another information processing device and execute the program and databases.

The main memory device 102 is configured to store various programs and store, for example, data needed for execution of the various programs.

The main memory device 102 includes a ROM (Read Only Memory) and a RAM (Random Access Memory) that are not illustrated.

The ROM is configured to store various programs such as BIOS (Basic Input/Output System).

The RAM functions as a work area to be developed when the various programs stored in the ROM are executed by the CPU 101. The RAM is not particularly limited and may be appropriately selected depending on the intended purpose. Examples of the RAM include a DRAM (Dynamic Random Access Memory) and a SRAM (Static Random Access Memory).

The auxiliary memory device 103 is not particularly limited and may be appropriately selected depending on the intended purpose so long as the auxiliary memory device 103 can store various information. Examples of the auxiliary memory device 103 include portable memory devices such as a CD (Compact Disc) drive, a DVD (Digital Versatile Disc) drive, and a BD (Blue-ray (registered trademark) Disc) drive.

For example, a display or a speaker can be used as the output device 104. The display is not particularly limited and a known display can be appropriately used. Examples of the display include a liquid crystal display and an organic EL display.

The input device 105 is not particularly limited and a known input device can be appropriately used so long as the input device can receive various requests to the detection determining device 100. Examples of the input device include a keyboard, a mouse, and a touch panel.

The hardware configuration as described above can realize the process functions of the detection determining device 100.

-Functional configuration of detection determining device-

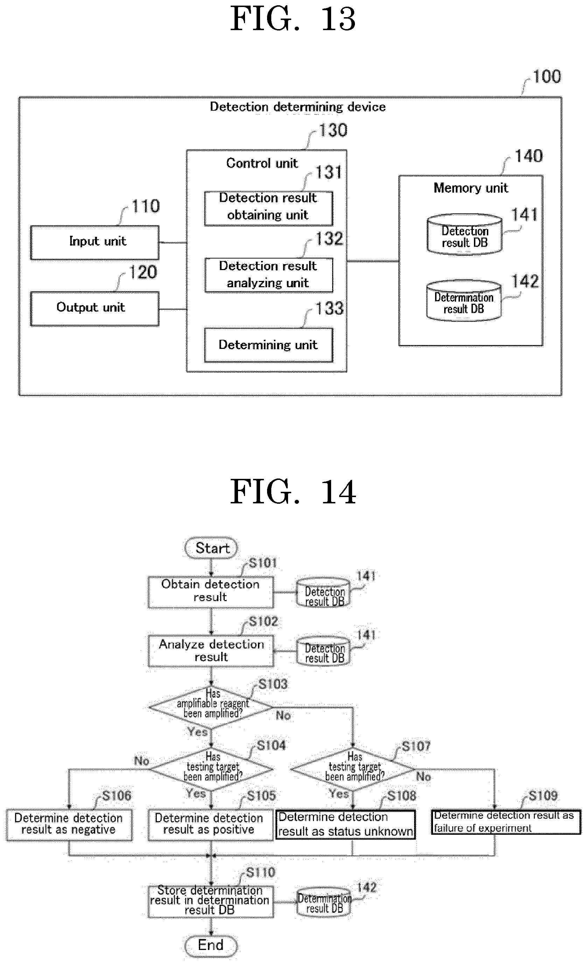

FIG. 13 is a diagram illustrating an example of the functional configuration of the detection determining device 10.

As illustrated in FIG. 13, the detection determining device 100 includes an input unit 110, an output unit 120, the control unit 130, and a memory unit 140.

The control unit 130 includes the detection result obtaining unit 131, the detection result analyzing unit 132, and a determining unit 133. The control unit 130 is configured to control the entire detection determining device 100.

The memory unit 140 includes the detection result database 141 and a determination result database 142. Hereinafter, "database" may be referred to as "DB".

The detection result obtaining unit 131 is configured to obtain a result of amplification of a nucleic acid serving as an amplifiable reagent and a result of amplification of a testing target nucleic acid obtained from PCR reactions. The control unit 130 is configured to store data of the obtained results of amplification in the detection result DB 141.

The detection result analyzing unit 132 is configured to analyze the result of amplification of the nucleic acid serving as the amplifiable reagent and the result of amplification of the testing target nucleic acid, using the data of the results of amplification stored in the detection result DB 141 of the memory unit 140.

The determining unit 133 is configured to determine "positive" and "negative" when the classifications described below are applicable, based on the results of the analyses of the detection result analyzing unit 132.

(1) When the amplifiable reagent is amplified and the testing target is amplified, it is determined that the testing target is present and the detection result is positive.
(2) When the amplifiable reagent is amplified and the testing target is not amplified, it is determined that the testing target is absent or at least less than the specific copy number of the amplifiable reagent and the detection result is negative.

In addition to the determinations of (1) and (2) above, the determining unit 133 may make a determination of, for example, failure of experiment when the cases of (3) and (4) in Table 3 are applicable.

The control unit 130 is configured to store the determination result of the determining unit 133 in the determination result DB 142.

Next, the process procedure of the detection determining program will be described. FIG. 14 is a flowchart illustrating an example of the process procedure of the detection determining program by the control unit 130 of the detection determining device 100.

In the step S101, the detection result obtaining unit 131 of the control unit 130 of the detection determining device 100 obtains a result of amplification of a nucleic acid serving as an amplifiable reagent and a result of amplification of a testing target nucleic acid obtained from PCR reactions, and moves the flow to the step S102. In the step S101, the control unit 130 stores the data of the results of amplification obtained by the detection result obtaining unit 131 in the detection result DB 141 of the memory unit 140.

In the step S102, the detection result analyzing unit 132 of the control unit 130 of the detection determining device 100 obtains the data of the results of amplification stored in the detection result DB 141. Then, the detection result analyzing unit 132 analyzes the respective results as to whether amplification is observed in the nucleic acid serving as the amplifiable reagent and whether amplification is observed in the testing target nucleic acid, and moves the flow to the step S103.

In the step S103, the determining unit 133 of the control unit 130 of the detection determining device 100 moves the flow to the step S104 when amplification is observed in the nucleic acid serving as the amplifiable reagent, based on the result of the analysis by the detection result analyzing unit 132. On the other hand, the determining unit 133 moves the flow to the step S107 when amplification is not observed in the nucleic acid serving as the amplifiable reagent.

In the step S104, the determining unit 133 moves the flow to the step S105 when amplification is observed in the testing target nucleic acid, based on the result of the analysis by the detection result analyzing unit 132. On the other hand, the determining unit 133 moves the flow to step S106 when amplification is not observed in the testing target nucleic acid.

In the step S105, the determining unit 133 determines that the testing target is present and the detection result is positive, based on the results that the nucleic acid serving as the amplifiable reagent is amplified and that the testing target nucleic acid is amplified, and moves the flow to the step S110.

In the step S106, the determining unit 133 determines that the testing target is absent or at least less than the specific copy number of the amplifiable reagent and the detection result is negative, based on the results that the nucleic acid serving as the amplifiable reagent is amplified and that the testing target nucleic acid is not amplified, and moves the flow to the step S110.

In the step S107, the determining unit 133 moves the flow to the step S108 when amplification is observed in the testing target nucleic acid, based on the result of the analysis by the detection result analyzing unit 132. On the other hand, the determining unit 133 moves the flow to step S109 when amplification is not observed in the testing target nucleic acid.

In the step S108, the determining unit 133 determines that the detection result is failure of the experiment (status unknown), based on the results that the nucleic acid serving as the amplifiable reagent is not amplified and that the testing target nucleic acid is amplified, and moves the flow to the step S110.

In the step S109, the determining unit 133 determines that the detection result is failure of the experiment, based on the results that the nucleic acid serving as the amplifiable reagent is not amplified and that the testing target nucleic acid is not amplified, and moves the flow to the step S110.

In the step S110, the control unit 130 stores the determination result made by the determining unit 133 in the determination result DB 142 of the memory unit 140 and terminates the flow.

In the present invention, it is at least needed to make the determination in the step S105 or the step S106, and a mode in which the flow is terminated without moving to the step S107 is possible when amplification is not observed in the nucleic acid serving as the amplifiable reagent.

<Second embodiment>

In another mode of the first embodiment, the device of the present invention may include a well in which the specific copy number of the amplifiable reagent is less than 100, and a well in which the specific copy number of the amplifiable reagent is 100 or greater.

The second embodiment is based on a finding that quantification of an unknown concentration sample using a calibration curve based on a serial dilution of a nucleic acid sample results in a considerably poor accuracy of quantification of a very trace nucleic acid.

As for the coefficient of variation CV of the wells in which the specific copy number of the amplifiable reagent is less than 100, a formula: $CV<1/\sqrt{x}$, or preferably, $CV<1/2\sqrt{x}$ is established between the coefficient of variation CV for the specific copy number and an average specific copy number x of the amplifiable reagent. Further, regardless of the value taken by the average specific copy number, it is preferable that the coefficient of variation CV of the wells in which the specific copy number of the amplifiable reagent is less than 100 be 20% or lower, or more preferably 10% or lower. In this range, it is possible to fill the amplifiable reagent at a high accuracy, when the specific copy number is less than 100.

It is preferable that the coefficient of variation CV of the wells in which the specific copy number of the amplifiable reagent is 100 or greater be 20% or lower. In this range, it is possible to fill the amplifiable reagent at a high accuracy, when the specific copy number is 100 or greater.

For the specific copy number of the amplifiable reagent of less than 100, it is preferable that a formula: $CV<1/\sqrt{x}$ be established between the coefficient of variation CV for the specific copy number and the average specific copy number x of the amplifiable reagent.

It is preferable that the coefficient of variation CV of the wells in which the specific copy number of the amplifiable reagent is 100 or greater be 20% or lower.

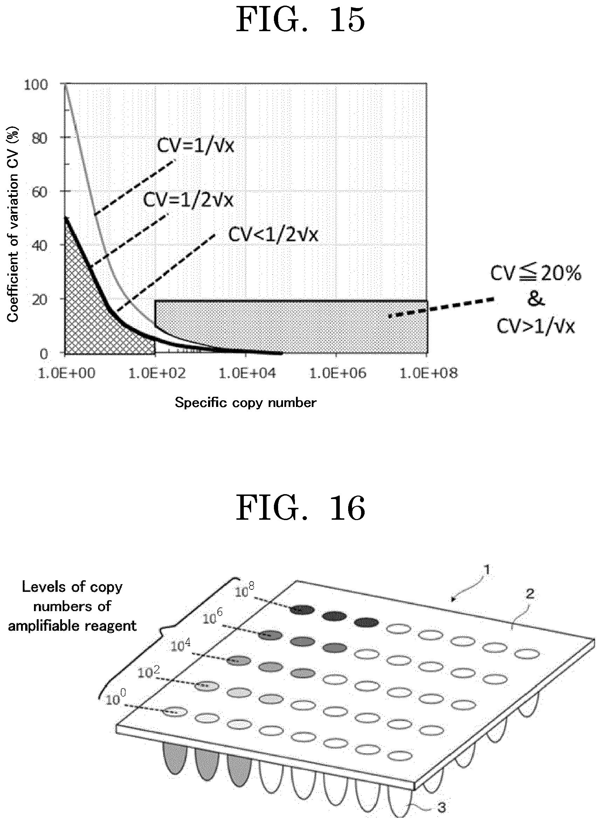

As a result, the device satisfies the relationship plotted in FIG. 15.

FIG. 15 is a graph plotting the relationship between the specific copy number (the copy number of nucleic acids filled in a well) and the coefficient of variation. FIG. 15 presents the following relational expressions: $CV=1/\sqrt{x}$ and $CV=1/2\sqrt{x}$, between the average specific copy number x and the coefficient of variation CV, a specific copy number of 100, and a CV value of 20%. From FIG. 15, it is possible to know (1) a region in which the specific copy number of the amplifiable reagent is less than 100, and a formula: $CV<1/\sqrt{x}$ is established between the coefficient of variation CV for that specific copy number and the average specific copy number x of the amplifiable reagent, and (2) a region in which the average specific copy number of the amplifiable reagent is 100 or greater, a formula: $CV>1/\sqrt{x}$ is satisfied between the coefficient of variation CV for that specific copy number and the average specific copy number x of the amplifiable reagent, and $CV\leq20\%$ is satisfied. Hence, the device enables a highly accurate measurement in a wide range varying from a low copy number to a high copy number.

It is preferable that the number of wells be 2 or greater, and that the specific copy number of the amplifiable reagent in one well and the specific copy number of the amplifiable reagent in any other well be of two or more levels different from each other. Examples of a combination of specific copy numbers include a combination of 1, 2, 3, 4, 5, 6, 7, 8, 9, and 10, a combination of 1, 3, 5, 7, and 9, and a combination of 2, 4, 6, 8, and 10.

It is preferable that the number of wells be 2 or greater, that the specific copy number of the amplifiable reagent in one well be $10^{N1}$, and that the specific copy number of the amplifiable reagent in any other well be $10^{N2}$ (where N1 and N2 are continuous integers). Examples of a combination of specific copy numbers for such a case include a combination of 1, 10, 100, and 1,000, and a combination of 100, 1,000, 10,000, 100,000, and 1,000,000. Hence, the device facilitates generation of a calibration curve for a wide range varying from a low copy number to a high copy number.

FIG. 16 is a perspective view illustrating another example of the device of the present invention. In the device of FIG. 16, levels of the copy number of the amplifiable reagent include the following five levels: $10^0$, $10^2$, $10^4$, $10^6$, and $10^8$.

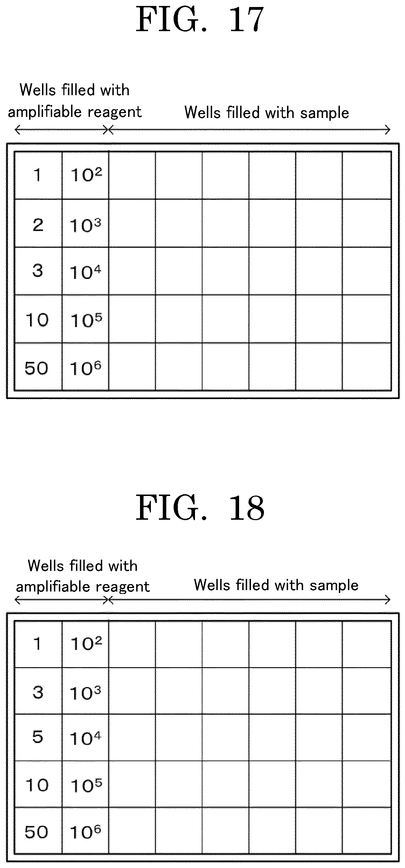

FIG. 17 is a diagram illustrating an example of the positions of the wells to be filled with the amplifiable reagent in the device of the present invention. The numerals in the wells in FIG. 17 indicate the specific copy numbers of the amplifiable reagent. There are provided wells in which the specific copy number is less than 100, namely 1, 2, 3, 10, and 50, and wells in which the specific copy number is 100 or greater, namely $10^2$, $10^3$, $10^4$, $10^5$, and $10^6$. The wells with no numerals in FIG. 17 are wells for a sample or control measurement.

FIG. 18 is a diagram illustrating another example of the positions of the wells to be filled with the amplifiable reagent in the device of the present invention. The numerals in the wells in FIG. 18 indicate the specific copy numbers of the amplifiable reagent. There are provided wells in which the specific copy number is less than 100, namely 1, 3, 5, 10, and 50, and wells in which the specific copy number is 100 or greater, namely $10^2$, $10^3$, $10^4$, $10^5$, and $10^6$. The wells with no numerals in FIG. 18 are wells for a sample or control measurement.

The method for producing a device according to the second embodiment includes "Preparation of amplifiable reagent by diluting method" and "Preparation of amplifiable reagent by discharging method". Both may be performed simultaneously or separately in order in one plate.

[Preparation of amplifiable reagent by diluting method]

It is preferable to prepare the amplifiable reagent by a diluting method when the specific copy number of the amplifiable reagent in one well is 100 or greater. In this case, the specific copy number of the amplifiable reagent is 100 or greater and preferably from 100 through $10^{10}$.

Examples of the diluting method include a method of producing serial dilutions according to a sample preparing method.

Examples of the sample preparing method include a manual operation using a pipette, a micropipetter (available from Eppendorf AG), and a pipetman (available from Eppendorf AG).

<<Preparation of amplifiable reagent by discharging method>>

It is preferable to prepare the amplifiable reagent by a discharging method when the specific copy number of the amplifiable reagent in one well is less than 100. In this case, the specific copy number of the amplifiable reagent is less than 100, preferably 50 or less, more preferably 10 or less, and yet more preferably 5 or less.

Examples of the discharging method include an inkjet discharging method, a cell sorter, and a flow cytometer. The same method as the method in "Device producing method" described below can be used for preparation of the amplifiable reagent by the discharging method.

By employing the device of the present invention as the second embodiment, it is possible to provide a device that enables highly accurate measurement in a wide range varying from a low copy number to a high copy number.

<Third embodiment>

In another mode of the first embodiment, the device of the present invention may include at least one well in which the amplifiable reagent is changed to a partial nucleic acid of the base sequence of the norovirus genome.

The third embodiment is based on a finding that existing devices in which the copy number of the nucleic acid of a norovirus contained in the wells is not specified have a low reliability of the result of amplification of the nucleic acid of the norovirus when subjected to an amplification reaction.

In the device of the present invention, the nucleic acid of the norovirus is located in a specific copy number in each well at a certain accuracy and at a filling accuracy higher than or equal to a certain level.

The device of the present invention can more infallibly avoid false-negative determinations and can be used in qualitative testings with a more improved negative determination accuracy, because the copy number of the nucleic acid of the norovirus contained in the wells of the device is a specific number. That is, the device of the present invention can be used in qualitative testings with accuracy for positivity or negativity. The device of the present invention can also be used in accurate quantitative testings of the norovirus contained in a sample, because the copy number of the nucleic acid of the norovirus contained in the wells of the device is a specific number.

«Nucleic acid of norovirus»

A well contains a nucleic acid of a norovirus in a specific copy number.

It is preferable that the nucleic acid of a norovirus be contained in a carrier.

Examples of the carrier include cells, phages, and viruses.

Examples of the cells include yeast fungi, animals, and plants.

As the cells, the content described in the following section "-Cells-" can be referred to.

The norovirus may be a norovirus collected from a living thing, or a norovirus collected from, for example, feces and vomit excreted by a person infected with the norovirus.

Examples of the living thing include clams.

Examples of the clams include oysters, Manila clams, and basket clams.

As the noroviruses, GI type, GII type, GIII type, GIV type, and GV type have been known. It has been known that the GI type and the GII type have the possibility of infecting humans. From this fact, as the norovirus, it is preferable that the nucleic acid to be contained in a specific copy number in the well be at least any one selected from a nucleic acid that expresses the GI type and a nucleic acid that expresses the GII type.

The norovirus has a nucleic acid formed of a positive single strand RNA as a gene sequence. The nucleic acid of the norovirus may be modified or mutated.

The nucleic acid may be in a bared state in a well or may be carried in a carrier in a well. The state of being carried in a carrier is preferable.

The carrier is not particularly limited and may be appropriately selected depending on the intended purpose as long as the carrier can carry a nucleic acid. Examples of the carrier include cells, liposomes, microcapsules, phages, and viruses. Among these carriers, cells are preferable.

After parts of base sequences extracted from nucleic acids of the norovirus are introduced by transgenesis as RNA and DNA into nucleic acids intrinsic to the cells, the number by which the base sequence of the nucleic acid of the norovirus is present can be obtained by measuring the number of carriers into which RNA and DNA have been introduced by transgenesis, since one nucleic acid is present per carrier.

When the nucleic acid of the norovirus contained in a specific copy number in a well includes both of a nucleic acid that expresses the GI type and a nucleic acid that expresses the GII type, the order of these nucleic acids is not particularly limited and may be appropriately selected depending on the intended purpose. Examples of the order include concatenating a nucleic acid that expresses the GII type directly after a nucleic acid that expresses the GI type, concatenating a nucleic acid that expresses the GI type directly after a nucleic acid that expresses the GII type, providing an arbitrary base sequence between a nucleic acid that expresses the GI type and a nucleic acid that expresses the GII type, and providing arbitrary base sequences before and after a nucleic acid that expresses the GI type and before and after a nucleic acid that expresses the GII type.

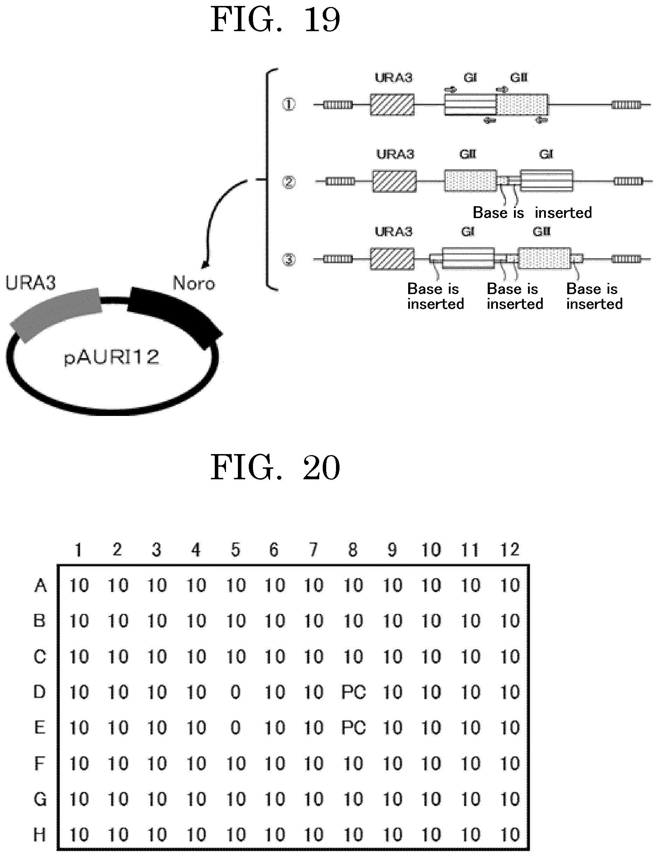

An example of, for example, sequences when a nucleic acid that expresses the GI type and a nucleic acid that expresses the GII type are introduced into a cell is illustrated in FIG. 19.

When a PCR method is used for amplification reactions, it is preferable that the primers include base sequences specified in a notice (Food Safety Inspection issue No. 1105001) of the director of Inspection and Safety Division, Department of Food Safety, Pharmaceutical and Food Safety Bureau of Ministry of Health, Labour and Welfare.

It is preferable that the nucleic acid of the norovirus include at least any one selected from a base sequence for detecting a nucleic acid that expresses the GI type, and a base sequence for detecting a nucleic acid that expresses the GII type.

The base sequence for detecting a nucleic acid that expresses the GI type is not particularly limited and may be appropriately selected depending on the intended purpose. Examples of the base sequence include SEQ ID NO. 8 and SEQ ID NO. 9.

The base sequence for detecting a nucleic acid that expresses the GII type is not particularly limited and may be appropriately selected depending on the intended purpose. Examples of the base sequence include SEQ ID NO. 10 and SEQ ID NO. 11.

It is preferable that the nucleic acid of the norovirus include two or more base sequences selected from SEQ ID NOS. 8, 9, 10, and 11.

It is preferable that the nucleic acid of the norovirus include a base sequence having a total length of 50 bases or more.

It is preferable that the nucleic acid of the norovirus include at least any one selected from a base sequence of a nucleic acid that expresses the GI type and a base sequence of a nucleic acid that expresses the GII type.

The base sequence of a nucleic acid that expresses the GI type is not particularly limited and may be appropriately selected depending on the intended purpose. Examples of the base sequence include SEQ ID NO. 4.

The base sequence of a nucleic acid that expresses the GII type is not particularly limited and may be appropriately selected depending on the intended purpose. Examples of the base sequence include SEQ ID NO. 5.

It is preferable that the nucleic acid of the norovirus include at least any one selected from a base sequence having a homology of 80% or higher with respect to the base sequence of SEQ ID NO. 4, and a base sequence having a homology of 80% or higher with respect to the base sequence of SEQ ID NO. 5.

The order of the base sequence having a homology of 80% or higher with respect to the base sequence of SEQ ID NO. 4 and the base sequence having a homology of 80% or higher with respect to the base sequence of SEQ ID NO. 5 is not particularly limited and may be appropriately selected depending on the intended purpose. Examples of the order include an order of a base sequence having a homology of 80% or higher with respect to the base sequence of SEQ ID NO. 4 to a base sequence having a homology of 80% or higher with respect to the base sequence of SEQ ID NO. 5 from a 5' terminal side, and an order of a base sequence having a homology of 80% or higher with respect to the base sequence of SEQ ID NO. 5 to a base sequence having a homology of 80% or higher with respect to the base sequence of SEQ ID NO. 4 from a 5' terminal side.

It is preferable that the nucleic acid of the norovirus include at least any base sequence selected from (i) a base sequence X including: a base sequence of SEQ ID NO. 4; and at a 5' terminal side or a 3' terminal side, a base sequence having an arbitrary length less than or equal to 1,000 bases, and a base sequence having a homology of 80% or higher with respect to the base sequence X, and (ii) a base sequence Y including: a base sequence of SEQ ID NO. 5; and at a 5' terminal side or a 3' terminal side, a base sequence having an arbitrary length less than or equal to 1,000 bases, and a base sequence having a homology of 80% or higher with respect to the base sequence Y.

The base sequence X including: a base sequence of SEQ ID NO. 4; and at a 5' terminal side or a 3' terminal side, a base sequence having an arbitrary length less than or equal to 1,000 bases is not particularly limited and may be appropriately selected depending on the intended purpose. Examples of the base sequence X include SEQ ID NO. 6.

The base sequence Y including: a base sequence of SEQ ID NO. 5; and at a 5' terminal side or a 3' terminal side, a base sequence having an arbitrary length less than or equal to 1,000 bases is not particularly limited and may be appropriately selected depending on the intended purpose. Examples of the base sequence Y include SEQ ID NO. 7.

The order of the base sequence of (i) and the base sequence of (ii) is not particularly limited and may be appropriately selected depending on the intended purpose. Examples of the order include an order of the base sequence of (i) to the base sequence of (ii) from a 5' terminal side, and an order of the base sequence of (ii) to the base sequence of (i) from a 5' terminal side.

It is preferable to confirm that one copy (one molecule) of the nucleic acid of the norovirus is introduced per cell by transgenesis. When the copy number of the nucleic acid of the norovirus (i.e., a specific base sequence) coincides with the number of nucleic acid molecules including that sequence, "copy number" and "number of molecules" may be associated with each other.

The method for confirming that one copy (one molecule) of the nucleic acid of the norovirus is introduced is not particularly limited and may be appropriately selected depending on the intended purpose. Examples of the method include DNA sequencing, a PCR method, and a Southern blotting method.

The number of kinds of the nucleic acid of the norovirus to be introduced by transgenesis may be one, or two or more. Also in the case of introducing only one kind of a nucleic acid by transgenesis, base sequences of the same kind may be introduced in tandem depending on the intended purpose.

The method for transgenesis is not particularly limited and may be appropriately selected depending on the intended purpose as long as the method can introduce the nucleic acid of the norovirus by an intended copy number at an intended position. Examples of the method include homologous recombination, CRISPR/Cas9, CRISPR/Cpf1, TALEN, Zinc finger nuclease, Flip-in, and Jump-in. When the carrier is a yeast fungus, homologous recombination is preferable among these methods in terms of a high transgenesis efficiency and ease of controlling.

Two or more of the wells in the device contain the nucleic acid of the norovirus in the specific copy number. It is preferable that the specific copy number of the nucleic acid of the norovirus in one of the wells be different from the specific copy number of the nucleic acid of the norovirus in another of the wells.

The device of the present invention includes wells in which a nucleic acid of a testing target norovirus is to be located, in addition to the wells in which the nucleic acid of the norovirus is located in the specific copy number. The wells in which the nucleic acid of the testing target norovirus is to be located may be filled with a predetermined amount of an amplifiable reagent different from the nucleic acid of the testing target norovirus. The predetermined amount needs at least to be a sufficiently detectable amount. When amplification of the amplifiable reagent has occurred, it can be confirmed that the amplification reaction is successful in the well in which the amplifiable reagent is located. Hence, the result of amplification of the testing target norovirus in the same well in which the amplifiable reagent is located is ensured a better reliability.

<Fourth embodiment>

In another mode of the first embodiment, the device of the present invention may include a plurality of wells, and two or more groups into which the wells are divided to be varied in composition in the wells.

It is preferable that the device of the fourth embodiment include a plurality of wells in each of which a reagent composition containing an amplifiable reagent in a specific copy number is located, and two or more groups into which the wells are divided to have the amplifiable reagent located in the same specific copy number but to be varied in composition of the reagent composition except for the specific copy number. It is preferable that the device of the fourth embodiment include a plurality of wells in each of which an amplifiable reagent is located in a specific copy number, and two or more groups into which the wells are divided to be varied in the specific copy number of the amplifiable reagent.

The fourth embodiment is based on the following finding. Existing diluting methods may probabilistically result in failure to fill some portions with copies or may result in failure to locate a copy as designed when the absolute number to be located is only one copy. Therefore, the performance of a measuring system cannot be evaluated with a high accuracy.

The device of the present invention is also based on the following finding. With existing standard nucleic acid kits, it has been impossible to overcome a problem that probabilistic variation of sample nucleic acids in a low copy number region is not taken into consideration.

The device of the present invention is also based on the following finding. Existing methods for evaluating measuring systems have evaluated the measuring systems only based on temperature, which is an indirect factor, and have not been able to appropriately evaluate influences on the measuring systems due to manual operations.

The device of the present invention including two or more groups of wells between which the specific copy number of the amplifiable reagent contained in the reagent composition is the same but the composition of the reagent composition is varied except for the specific copy number can be used for appropriately evaluating the skill of a preparator who prepares a reagent composition.

The fourth embodiment is based on the following finding. Existing diluting methods may probabilistically result in failure to fill some portions with copies or may result in failure to locate a copy as designed when the absolute number to be located is only one copy. Therefore, existing diluting methods cannot be used for evaluation of an in-plane property of a testing device and quantitative evaluation.

The device of the present invention is also based on the following finding. Existing methods employing manipulation are techniques relating to the method for preparing a sample solution. Performance evaluation for testing devices is neither described nor suggested. Besides, these techniques are problematic in throughput.

The device of the present invention including two or more groups into which a plurality of wells in each of which an amplifiable reagent is located in a specific copy number are divided such that the specific copy number of the amplifiable reagent is varied between the groups makes it possible to appropriately evaluate in-plane uniformity of a testing device, inter-testing device performance, and inter-testing facility performance.

The device of the fourth embodiment includes a plurality of wells, and a reagent composition containing an amplifiable reagent in a specific copy number is located in each of the plurality of wells.

A specific copy number (number of specific sequences) means that the copy number of the specific sequence of the amplifiable reagent contained in a well is specified at accuracy of a certain level or higher (with a low uncertainty). This means that the specific copy number is known as the number of specific sequences actually contained in a well. That is, the specific copy number in the present invention is more accurate or reliable as a number than a predetermined copy number (calculated estimated value) obtained according to existing concentration dilution, and is a controlled value that has no dependency on a Poisson distribution even if the value is within a low copy number region of 1,000 or lower in particular. Hence, use of a device including wells in which a specific sequence is contained in the specific copy number makes it possible to perform qualitative or quantitative testing of the amplifiable reagent more accurately than ever.

When the copy number of the nucleic acid (specific base sequence) of the amplifiable reagent and the number of nucleic acid molecules including the sequence coincide with each other, "copy number" and "number of molecules" may be associated with each other.

In the present invention, a specific copy number of the amplifiable reagent may also be referred to as absolute number of the amplifiable reagent.

In addition to the amplifiable reagent in the specific copy number, the reagent composition contains components needed for amplifying the amplifiable reagent (for example, a nucleic acid), and, for example, contains a primer and an amplifying reagent.

A primer is a synthetic oligonucleotide having a complementary base sequence that includes 18 or more but 30 or less bases and is specific to a template DNA of a polymerase chain reaction (PCR). A pair of primers, namely a forward primer and a reverse primer, are set at two positions in a manner to sandwich the region to be amplified.

Examples of the amplifying reagent for, for example, a polymerase chain reaction (PCR) include enzymes such as DNA polymerase, matrices such as the four kinds of bases (dGTP, dCTP, dATP, and dTTP), $Mg^{2+}$ (2 mM magnesium chloride), and a buffer for maintaining the optimum pH (pH of from 7.5 through 9.5).

The device includes two or more groups of wells between which the specific copy number of the amplifiable reagent is the same but the composition of the reagent composition is varied except for the specific copy number. For example, when the base material of the device is a plate including a plurality of wells, a "region" of each group is formed on the plate by each group. Two or more regions between which the specific copy number of the amplifiable reagent in the reagent composition is varied may adjoin each other or may be apart from each other.

For example, being varied in composition except for the specific copy number of the amplifiable reagent means that it is possible to provide combinations that may or may not contain any other primer or amplifying reagent than the nucleic acid serving as the amplifiable reagent. For example, a first group of the device includes (1) a composition containing a nucleic acid, a primer, and an amplifying reagent, a second group of the device includes (2) a composition containing a nucleic acid and a primer, and a third group of the device includes (3) a composition containing only a nucleic acid. A preparator adds at least any one of a primer and an amplifying reagent to the compositions of (2) and (3) by a manual operation in preparation of the reagent composition, and use the reagent composition for a PCR reaction. Hence, the device enables, for example, evaluation of the skill of a preparator who prepares the reagent composition.

When a nucleic acid is used as the amplifiable reagent, examples of the skill of the preparator include a pipetting operation, an amount of the amplifying reagent to be prepared, and addition of a reagent to a well plate.

In preparation, the preparator may have a previous knowledge of the kinds, amounts, and concentrations of nucleic acids, primers (or primer sets including a plurality of kinds of primers), and amplifying reagents to be contained in the respective groups of the device used. This makes it also possible for the preparator to prepare the reagent composition in a manner to suit to the kinds, amounts, and concentrations in the respective groups, and use the reagent composition for a PCR reaction. This enables more appropriate evaluation of the skill of the preparator.

Further, it is possible to employ a mode in which the device includes a plurality of wells, and reagent compositions located in the plurality of wells and each containing at least a composition selected from the group consisting of an amplifiable reagent, a primer, and an amplifying reagent, wherein the reagent compositions include two or more groups varied in the composition.

This configuration makes it possible to evaluate the skill of a preparator who prepares arbitrary compositions.

Furthermore, it is preferable that the device include groups varied from each other in the specific copy number of the amplifiable reagent. That is, it is preferable that there be two or more specific copy numbers of the amplifiable reagent varied between one well and any other well(s). Examples of the combination of the two or more specific copy numbers include a combination of 1, 2, 3, 4, 5, 6, 7, 8, 9, and 10, a combination of 1, 3, 5, 7, and 9, and a combination of 2, 4, 6, 8, and 10.

In the device of the present invention, it is preferable that the specific copy number of the amplifiable reagent in one well be $10^{N1}$, and that the specific copy number of the amplifiable reagent in another well be $10^{N2}$ (where N1 and N2 are mutually continuous integers). Examples of the combination of such specific copy numbers include a combination of 1, 10, 100, and 1,000, and a combination of 100, 1,000, 10,000, 100,000, and 1,000,000. Hence, a calibration curve for a wide range ranging from a low copy number to a high copy number can be easily generated with the device of the present invention.

The device of the present invention includes two or more groups of wells varied in the specific copy number of the amplifiable reagent. For example, when the base material of the device is a plate including a plurality of wells, a "region" of each group is formed on the plate by each group. In the "regions" formed by two or more groups between which the specific copy number of the amplifiable reagent is varied, wells may adjoin each other or may be apart from each other.

Hence, when real-time PCR, which is one kind of evaluation of the performance of a testing device, is performed using the device of the present invention, and the device turns out to include a well (an unqualified well) unsuitable for use as compared with another well present at a different position and sharing the same specific copy number, it is possible to judge whether to calibrate the testing device by real-time PCR again or to exclude the unqualified well from actual sample application. Furthermore, by measuring a testing device on a regular basis using the device of the present invention, it is possible to obtain information on temporal changes of Ct value at an in-plane position of the testing device and evaluate an in-plane property of the testing device based on the information. Moreover, use of devices on which the same specific copy number is located enables comparison between a testing device subjected to measurement and another testing device.

It is preferable that at least one group of the groups of wells be a group in which the specific copy number of the amplifiable reagent is close to the limit of detection.

The limit of detection (LOD) represents the least copy number of amplifiable reagent detectable by a method that can detect an amplifiable reagent (for example, a nucleic acid). The limit of detection is not particularly limited, may be appropriately selected depending on a measuring method, and may be, for example, average value + 3σ.

In the present specification, when there are sample sets varied in the specific copy number and each including 21 samples with the same specific copy number, and any of the sample sets result(s) in non-detection from one sample among the 21 samples (corresponding to 2σ judged by a probability of 95%), the least copy number among the specific copy numbers of such sample sets may be used as the limit of detection.

Being close to the limit of detection means a copy number in a range of ± 1 from the limit of detection copy number.

It is assumed that the device of the present invention includes at least a group in which the specific copy number of the amplifiable reagent is close to the limit of detection. For example, it is assumed that the device of the present invention includes groups with the specific copy number of the amplifiable reagent of "1", "2", "3", "4", and "5", respectively. In this case, when a testing device subjected to performance evaluation using the device of the present invention turns out to be able to amplify the amplifiable reagent in the groups with the specific copy number of "3" or greater but unable to amplify the amplifiable reagent in the groups with the specific copy number of "2" or less, it can be revealed that the lower limit value representing the limit of detection of the specific copy number of the amplifiable reagent in the testing device is "3". Furthermore, when another similar testing device subjected to performance evaluation using the device of the present invention turns out to be able to amplify the amplifiable reagent in the groups with the specific copy number of "4" or greater but unable to amplify the amplifiable reagent in the groups with the specific copy number of "3" or less, it can be revealed that the lower limit value representing the limit of detection of the specific copy number of the amplifiable reagent in the testing device is "4". Hence, it is possible to determine the least specific copy number of the amplifiable reagent detectable by a testing device.

It is preferable that at least one group among the groups in which the amplifiable reagent is located in specific copy numbers be a group in which the specific copy number of the amplifiable reagent is a copy number greater than a limit of quantification.

The limit of quantification (LOQ) indicates the least copy number of amplifiable reagent quantifiable by a method that can quantify an amplifiable reagent (for example, a nucleic acid), where "quantifiable" means that the result of quantification can be sufficiently reliable. The limit of quantification is not particularly limited and may be appropriately selected depending on a measuring method.

In the present specification, a value representing a copy number deviating from linearity of a calibration curve generated using samples formed of a plurality of molecular species (for example, a plurality of nucleic acid samples with different specific copy numbers) may be used as the limit of quantification. Alternatively, uncertainty of a calibration curve may be expressed by CV value. In a graph plotting CV value by representing copy number on a horizontal axis and Ct value on a vertical axis, for example, a value (a copy number) with a CV value of lower than 5% or 10% may be used as the limit of quantification.

In a quantitative evaluation, not a Ct value per se, but a copy number (or a concentration) corresponding to a Ct value can be obtained from a calibration curve and PCR efficiency. Therefore, the limit of quantification may be set based on CV value converted to a copy number (or a concentration).

When the device of the present invention includes at least a group in which the specific copy number of the amplifiable reagent (for example, a nucleic acid) is a copy number greater than the limit of quantification, for example, it is possible to determine the least specific copy number of the amplifiable reagent that can be ensured quantitative detection by a testing device, like it is possible to find a testing device to be capable of ensuring quantitative detection when the specific copy number of the amplifiable reagent is 10 or greater, and find another testing device to be capable of ensuring quantitative detection when the specific coy number of nucleic acid is 20 or greater.

In the device of the present invention, it is preferable that at least one group of the groups of wells be a negative control group in which the specific copy number of the amplifiable reagent is 0.

With at least one group of the groups of wells provided as a negative control group in which the specific copy number of the amplifiable reagent is 0 in the device of the present invention, any detection of an amplifiable reagent from the negative control group suggests abnormality of the detection system (the reagent or the device). With the negative control group provided in the device, the user can immediately recognize a problem when the problem occurs, and can stop the measurement and inspect the root of the problem.

In the device of the present invention, it is preferable that at least one group of the groups of wells be a positive control group in which the specific copy number of the amplifiable reagent is 100 or greater.

With at least one group of the groups of wells provided as a positive control group in which the specific copy number of the amplifiable reagent is 100 or greater in the device of the present invention, any non-detection of an amplifiable reagent from the positive control group suggests abnormality of the detection system (the reagent or the device). With the positive control group provided in the device, the user can immediately recognize a problem when the problem occurs, and can stop the measurement and inspect the root of the problem.

In the device of the present invention, it is further preferable that at least one group of the groups of wells be a group (a group with the least copy number) having the least copy number except for the negative control group, and that the group with the least copy number be positioned at least at wells that are approximately at the periphery of the device.

Wells that are approximately at the periphery means wells on the outermost periphery and wells on some lines that are inward from the outermost periphery among the wells arranged two-dimensionally on the device. Examples of the wells on the outermost periphery and the wells on some lines that are inward from the outermost periphery include wells on the first line or a line with a greater ordinal number but on the 47th line or a line with a less ordinal number.

Unlike the wells positioned near the center of the device, the wells positioned on the outermost periphery of the device have no other wells on the outer side and define the boundary between the device and the outside of the device. Therefore, (1) the wells on the outermost periphery physically have uneven thermal conduction on the device, and (2) the wells on the outermost periphery are susceptible to temperature fluctuating factors of a PCR device because the wells are set on the periphery of a temperature controlling member, which is a component constituting the PCR device. Therefore, by at least one group of the groups of wells being provided in the device of the present invention as a group (a group with the least copy number) having the least copy number except for the negative control group and by the group with the least copy number being positioned at least at the wells that are approximately at the periphery of the device, it is possible to detect failures of the PCR device with a higher sensitivity.

FIG. 20 is a plan view illustrating an example of the device of the present invention. In the device of FIG. 20, nucleic acid in a specific copy number of 10 copies and a primer are located almost all over the surface of a 96-well plate. In the center, negative control (NTC) with 0 copies is located at two positions, and positive control (PC) with 1,000 copies of nucleic acid and with a primer and an amplifying reagent is located at two positions.

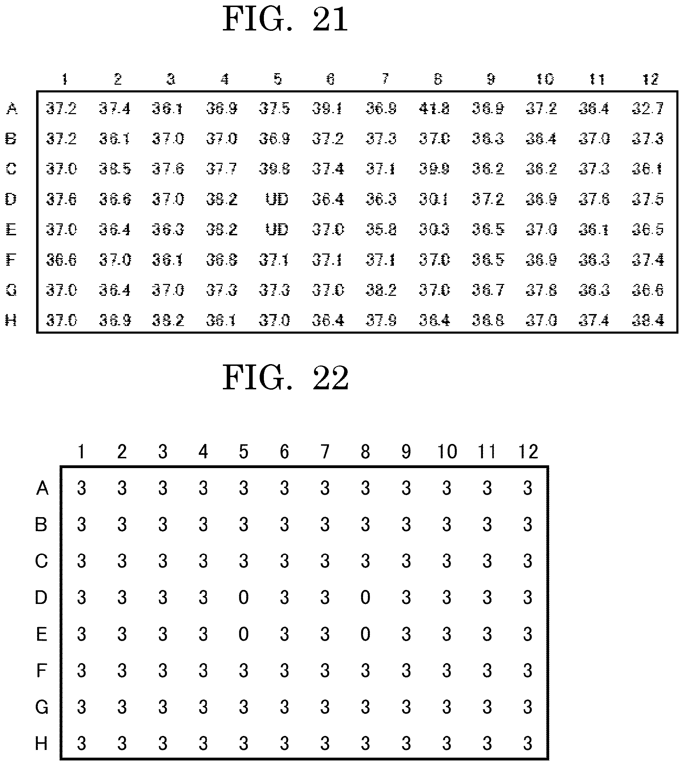

Real-time PCR is performed using the device of FIG. 20. The measured results of Ct (Threshold Cycle) values are presented in FIG. 21. The average Ct value is 37.1. The standard deviation of the Ct values is 1.00. The CV value [(standard deviation/average Ct value)×100] is 2.70. In FIG. 21, "UD" means that a Ct value is not detected.

For example, in the real-time PCR, first, a master mix (available from Thermo Fisher Scientific Inc., TAQMAN UNIVERSAL PCR MASTER MIX) (1 microliter), a forward primer and a reverse primer for amplifying a specific base sequence (0.5 nmol each), and a probe (0.4 nmol) are added to each sample in the device. Subsequently, amplification and detection can be performed with a real-time PCR device (available from Thermo Fisher Scientific Inc., QUANTSTUDIO 7 FLEX).

Ct value indicates the cycle number at a timing when a fluorescence signal of a reaction crosses Threshold Line. Because Ct value is linearly decreased to the initial amount of the target, the initial copy number of DNA can be calculated based on Ct value.

Threshold Line indicates the signal level at which a statistically significant increase from a calculated baseline signal is observed, and means the threshold of a real-time PCR reaction.

Hence, an in-plane property of the device can be evaluated based on, for example, the average Ct value, the standard deviation of the Ct values, the CV value [(standard deviation/average Ct value)×100], and [(Ct value (Max)-Ct value (min))/2·average Ct value]×100.

In the device of FIG. 22, nucleic acid in a specific copy number of 3 copies, a primer, and an amplifying reagent are located almost all over the surface of a 96-well plate. In the center of the plate, negative control (NTC) with 0 copies is located at four positions.

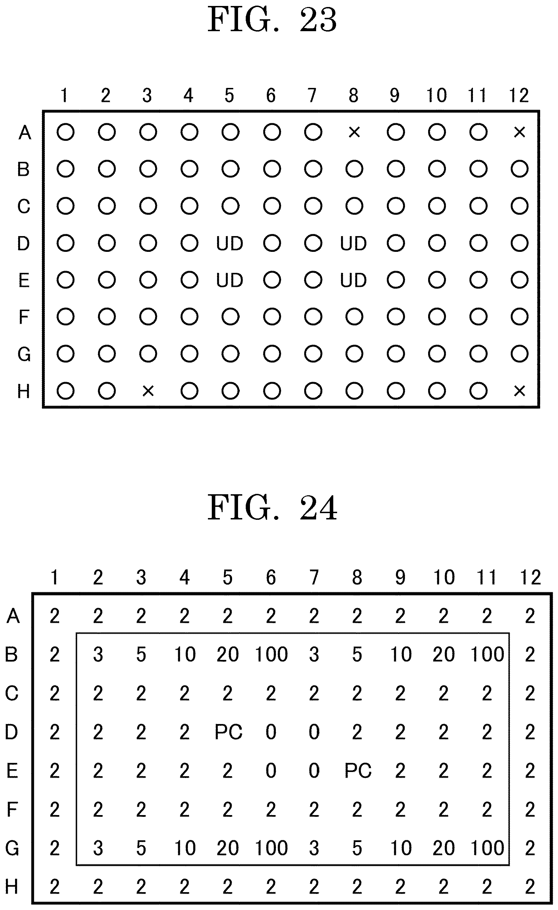

Real-time PCR is performed using the device of FIG. 22, to measure Ct values and calculate the average Ct value. When a well with the specific copy number (3 copies) has a Ct value that is within 10% of the average Ct value, the well is indicated as "○". When a well with the specific copy number (3 copies) has a Ct value that is greater than 10% of the average Ct value, the well is indicated as "×". The results are presented in FIG. 23. In FIG. 23, "UD" means that a Ct value is not detected.

As a result, the Ct value is greater than 10% of the average Ct value at four wells on the periphery of the 96-well plate. It is revealed that these four wells on the periphery are unsuitable for use at a low copy number. Hence, it is possible to judge whether to perform calibration by real-time PCR again or not to use these four wells on the periphery with actual samples.

For example, by performing measurement for a certain period of time using the devices described with reference to FIG. 20 to FIG. 23, it is possible to obtain temporal changes of the Ct values. Hence, like an in-plane property of a testing device, when a value greater than 10% of the average Ct value is obtained as a Ct value of a well, it is possible to adopt a measure of calibrating the testing device or not using the measured position. Furthermore, because the specific copy number of the copies located is an absolute value, use of devices with copies located in the same specific copy number enables comparison of the performance between testing devices.

In a 96-well plate illustrated in FIG. 24, nucleic acid in specific copy numbers of 2 copies, 3 copies, 5 copies, 10 copies, 20 copies, 50 copies, and 100 copies, a primer, and an amplifying reagent are located, and negative control (NTC) with 0 copies and positive control (PC) with 500 copies of nucleic acid and a primer are located in the center.

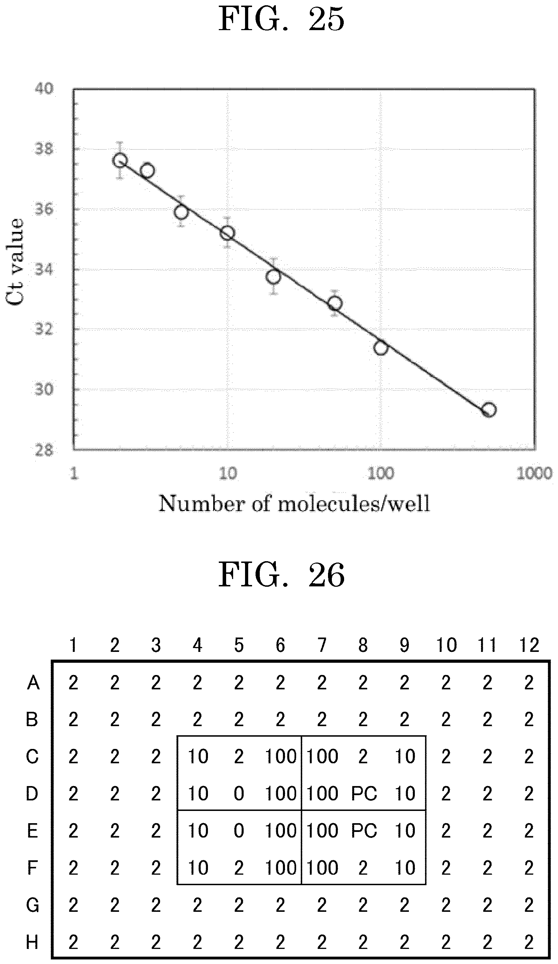

Real-time PCR is performed using the device of FIG. 24, to measure Ct values. A calibration curve plotting the Ct values on the vertical axis and the copy number on the horizontal axis is presented in FIG. 25. A correlation coefficient ($R^2$) is used as an indicator of the performance of a testing device. In this case, the correlation coefficient is 0.99. The correlation coefficient is a value indicating at what degree data coincides with a calibration curve, and reflects the linearity of the calibration curve. The least copy number (in this case, 2 copies) located on the outermost periphery can serve as an indicator of in-plane uniformity.

In the device of FIG. 26, nucleic acid in specific copy numbers of 2 copies, 10 copies, and 100 copies, a primer, and an amplifying reagent are located in the center of a 96-well plate, and negative control (NTC) with 0 copies and positive control (PC) with 500 copies of nucleic acid and a primer are located in the center. The least copy number (in this case, 2 copies) located on the outermost periphery and on the approximate periphery including one horizontal line and two vertical lines inward from the outermost periphery can serve as an indicator of in-plane uniformity.

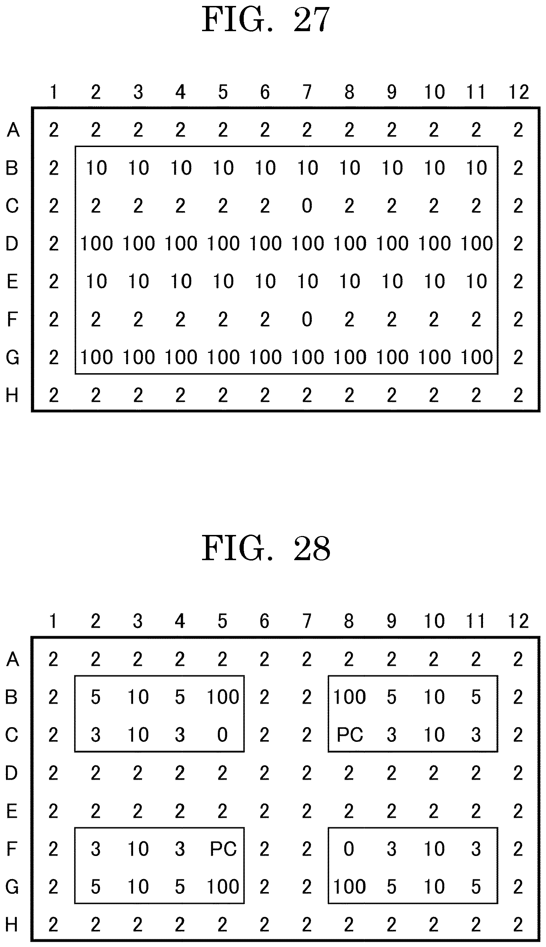

In the device of FIG. 27, nucleic acid in specific copy numbers of 2 copies, 10 copies, and 100 copies, a primer, and an amplifying reagent are located in 20 wells per specific copy number in a 96-well plate, the least copy number (in this case, 2 copies) is located on the outermost periphery, and the wells with the specific copy number of 100 copies also serve as positive control (PC). The least copy number (in this case, 2 copies) located on the outermost periphery can serve as an indicator of in-plane uniformity.

In the device of FIG. 28, nucleic acid in specific copy numbers of 3 copies, 5 copies, 10 copies, and 100 copies and a primer are located in a 96-well plate, and the least copy number (in this case, 2 copies) is located on the outermost periphery and in a cross formation. Negative control (NTC) with 0 copies and positive control (PC) with 500 copies of nucleic acid, a primer, and an amplifying reagent are located.

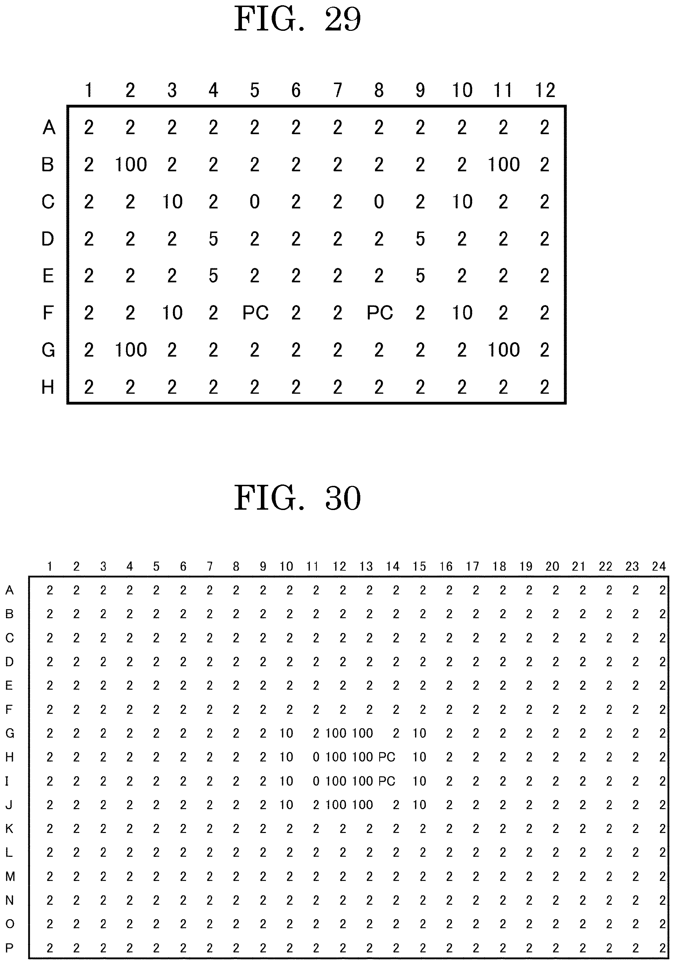

In the device of FIG. 29, nucleic acid in specific copy numbers of 2 copies, 5 copies, 10 copies, and 100 copies and a primer are located in a 96-well plate, and the least copy number (in this case, 2 copies) is located on the outermost periphery. Negative control (NTC) with 0 copies and positive control (PC) with 500 copies of nucleic acid and a primer are located in the center.

In the device of FIG. 30, nucleic acid in specific copy numbers of 2 copies, 10 copies, and 100 copies, a primer, and an amplifying reagent are located in the center of a 384-well plate, and the least copy number (in this case, 2 copies) is located in the rest of the plate. The least copy number (in this case, 2 copies) located on the outermost periphery and on the approximate periphery including five horizontal lines and eight vertical lines inward from the outermost periphery can serve as an indicator of in-plane uniformity.

By performing measurement for a certain period of time using the devices described with reference to FIG. 24 and FIG. 26 to FIG. 30, it is possible to obtain temporal changes of the Ct values. Hence, like an in-plane property of a testing device, when a value deviating from a quality control value is obtained, it is possible to adopt a measure such as calibrating the testing device or not using the measured position. Furthermore, because the copy number of the copies located is an absolute value, use of devices with copies located in the same copy number enables comparison of the performance between testing devices.

A preparator's skill evaluating method, a preparator's skill evaluating device, and a preparator's skill evaluating program using the device of the fourth embodiment including two or more groups of wells between which the specific copy number of the amplifiable reagent contained in the reagent composition is the same but the composition of the reagent composition is varied except for the specific copy number will be described.

[Preparator's skill evaluating method, preparator's skill evaluating device, and preparator's skill evaluating program]

A preparator's skill evaluating method is a preparator's skill evaluating method for evaluating the skill of a preparator who prepares a reagent composition, includes a Ct value information obtaining step of obtaining information on a Ct value in the device of the present invention using the device of the present invention and a skill evaluating step of evaluating the skill of a preparator based on the obtained information on the Ct value, and further includes other steps as needed.

A preparator's skill evaluating device of the present invention is a preparator's skill evaluating device configured to evaluate the skill of a preparator who prepares a reagent composition, includes a Ct value information obtaining unit configured to obtain information on a Ct value in the device of the present invention using the device of the present invention and a skill evaluating unit configured to evaluate the skill of a preparator based on the obtained information on the Ct value, and further includes other units as needed.

A preparator's skill evaluating program is a preparator's skill evaluating program for evaluating the skill of a preparator who prepares a reagent composition, and causes a computer to execute a process including obtaining information on a Ct value in the device of the present invention using the device of the present invention, and evaluating the skill of a preparator based on the obtained information on the Ct value.

Control being performed by, for example, a control unit of the preparator's skill evaluating device has the same meaning as the preparator's skill evaluating method being carried out. Therefore, details of the preparator's skill evaluating method will also be specified through description of the preparator's skill evaluating device. Further, the preparator's skill evaluating program realizes the preparator's skill evaluating device with the use of, for example, computers as hardware resources. Therefore, details of the preparator's skill evaluating program will also be specified through description of the preparator's skill evaluating device.

<Ct value information obtaining step and Ct value information obtaining unit>

The Ct value information obtaining step is a step of obtaining information on a Ct value in the device of the present invention using the device of the present invention, and is performed by the Ct value information obtaining unit.

Ct value indicates the cycle number at a timing when a fluorescence signal of a reaction crosses Threshold Line. Because Ct value is linearly decreased to the initial amount of the target, the initial copy number of DNA can be calculated based on Ct value.

Threshold Line indicates the signal level at which a statistically significant increase from a calculated baseline signal is observed, and means the threshold of a real-time PCR reaction.

Baseline means the signal level in PCR initial cycles during which the fluorescence signal does not almost change.

Examples of the information on the Ct value include a Ct value, average Ct, standard deviation, CV value [(standard deviation/average Ct)$\times$100], and [(Ct(Max)-Ct(min))/2·average Ct]$\times$100.

<Skill evaluating step and skill evaluating unit>

The skill evaluating step is a step of evaluating the skill of a preparator based on the information on the Ct value, and is performed by the skill evaluating unit.

Examples of the skill of the preparator include a skill of the preparator to prepare a reagent composition by a manual operation as regards the composition other than a nucleic acid in the device.

It is possible to evaluate the skill of the preparator, based on a PCR reaction of a prepared reagent composition performed using the device of the present invention, and comparison of information on Ct values to be obtained.

For evaluation of the skill of the preparator, the preparator may prepare samples on the same device on which the standard samples with which the samples are to be compared are provided, or may prepare samples on a different device. As the method for evaluating the skill of the preparator by letting the preparator prepare samples on the same device on which the standard samples with which the samples are to be compared are provided, for example, an automatic dispensing device or a certified skill holder prepares reagents on wells, and the preparator to be evaluated prepares reagents on different wells on the same device. Then, Ct values obtained from PCR reactions in the respective wells are compared with each other. In this way, it is possible to evaluate the skill of the preparator. As the method for evaluating the skill of the preparator by letting the preparator prepare samples on a device different from the device on which the standard samples with which the samples are to be compared are provided, for example, an automatic dispensing device or a certified skill holder prepares reagents on a first device, and the preparator to be evaluated prepares reagents on a second device. Then, Ct values obtained from PCR reactions using the prepared first and second devices are compared with each other. In this way, it is possible to evaluate the skill of the preparator to be evaluated.

<Other steps and other units>

The other steps and other units are not particularly limited and may be appropriately selected depending on the intended purpose. Examples of the other steps and other units include a displaying step and a display unit.

The process according to the preparator's skill evaluating program of the present invention can be executed using a computer including a control unit constituting the preparator's skill evaluating device.

The hardware configuration and the functional configuration of the preparator's skill evaluating device will be described below.

<Hardware configuration of preparator's skill evaluating device>

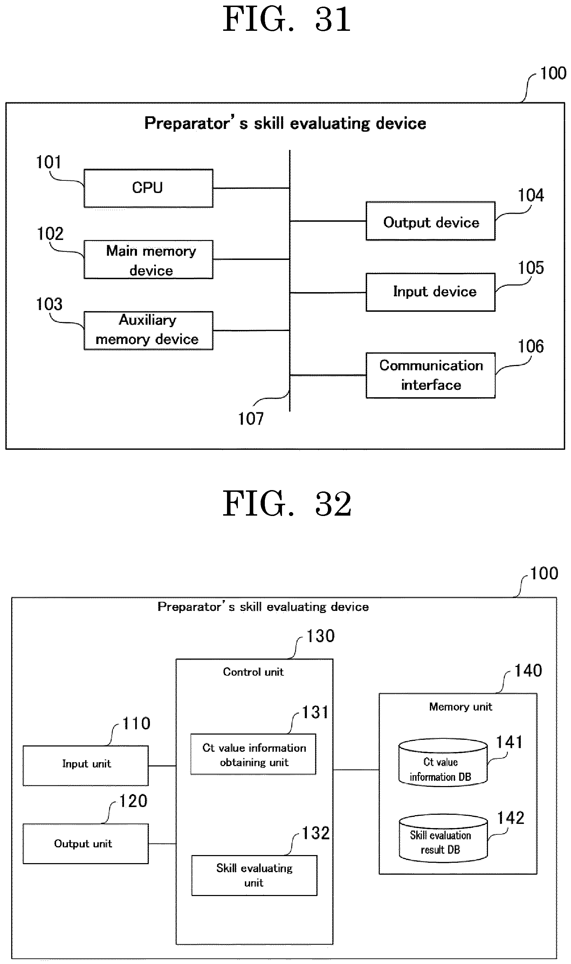

FIG. 31 is a block diagram illustrating an example of a hardware configuration of a preparator's skill evaluating device 100.

As illustrated in FIG. 31, the preparator's skill evaluating device 100 includes a CPU (Central Processing Unit) 101, a main memory device 102, an auxiliary memory device 103, an output device 104, an input device 105, and a communication interface (communication I/F) 106. These units are coupled to one another via a bus 107.

The CPU 101 is a processing device configured to execute various controls and operations. The CPU 101 realizes various functions by executing an OS (Operating System) and programs stored in, for example, the main memory device 102. That is, in the present example, the CPU 101 functions as a control unit 130 of the preparator's skill evaluating device 100 by executing the preparator's skill evaluating program.

The CPU 101 also controls the operation of the preparator's skill evaluating device 100 on the whole. In the present example, the CPU 101 is used as the device configured to control the operation of the preparator's skill evaluating device 100 on the whole. However, this is non-limiting. For example, a FPGA (Field Programmable Gate Array) may be used.

The preparator's skill evaluating program and various databases need not be stored in, for example, the main memory device 102 or the auxiliary memory device 103. The preparator's skill evaluating program and various databases may be stored in, for example, any other information processing device that is coupled to the preparator's skill evaluating device 100 via, for example, the Internet, a LAN (Local Area Network), and a WAN (Wide Area Network). The preparator's skill evaluating device 100 may be configured to receive the preparator's skill evaluating program and various databases from such another information processing device and execute the preparator's skill evaluating program and various databases.

The main memory device 102 is configured to store various programs and store, for example, data needed for execution of the various programs.

The main memory device 102 includes a ROM (Read Only Memory) and a RAM (Random Access Memory) unillustrated.

The ROM stores, for example, various programs such as BIOS (Basic Input/Output System).

The RAM functions as a work area to be developed when the various programs stored in the ROM are executed by the CPU 101. The RAM is not particularly limited and may be appropriately selected depending on the intended purpose. Examples of the RAM include a DRAM (Dynamic Random Access Memory) and an SRAM (Static Random Access Memory).

The auxiliary memory device 103 is not particularly limited and may be appropriately selected depending on the intended purpose so long as the auxiliary memory device 103 can store various information. Examples of the auxiliary memory device 103 include a solid-state drive and a hard disk drive. As the auxiliary memory device 103, for example, portable memory devices such as a CD (Compact Disc) drive, a DVD (Digital Versatile Disc) drive, and a BD (Blu-ray (registered trademark) Disc) drive may also be used.

As the output device 104, for example, a display and a speaker can be used. The display is not particularly limited and an appropriate known display may be used. Examples of the display include a liquid crystal display and an organic EL display.

The input device 105 is not particularly limited and an appropriate known input device may be used so long as the input device can receive various requests to the preparator's skill evaluating device 100. Examples of the input device include a keyboard, a mouse, and a touch panel.

The communication interface (communication I/F) 106 is not particularly limited and an appropriate known communication interface may be used. Examples of the communication interface include a wireless or wired communication device.

The hardware configuration described above can realize the process functions of the preparator's skill evaluating device 100.

<Functional configuration of preparator's skill evaluating device>

FIG. 32 is a diagram illustrating an example of a functional configuration of the preparator's skill evaluating device 100.

As illustrated in FIG. 32, the preparator's skill evaluating device 100 includes an input unit 110, an output unit 120, a control unit 130, and a memory unit 140.

The control unit 130 includes a Ct value information obtaining unit 131 and a skill evaluating unit 132. The control unit 130 is configured to control the preparator's skill evaluating device 100 on the whole.

The memory unit 140 includes a Ct value information database 141 and a skill evaluation result database 142. Hereinafter, "database" may also be referred to as "DB".

The Ct value information obtaining unit 131 is configured to obtain information on a Ct value, using data representing the information on a Ct value stored in the Ct value information DB 141 of the memory unit 140. As described above, the Ct value information DB 141 stores, for example, data representing a Ct value previously obtained by an experiment. Note that information on a Ct value associated with the device may be stored in the Ct value information DB 141. Inputting to the DB may be performed via another information processing device coupled to the preparator's skill evaluating device 100 or performed by a human operator.

The skill evaluating unit 132 is configured to evaluate the skill of a preparator based on information on a Ct value. Examples of a specific method for evaluating the skill of a preparator include a method of calculating a standard deviation from the obtained Ct value and evaluating the skill of the preparator based on the calculated standard deviation.

The result of evaluating the skill of the preparator by the skill evaluating unit 132 is stored in the skill evaluation result DB 142 of the memory unit 140.

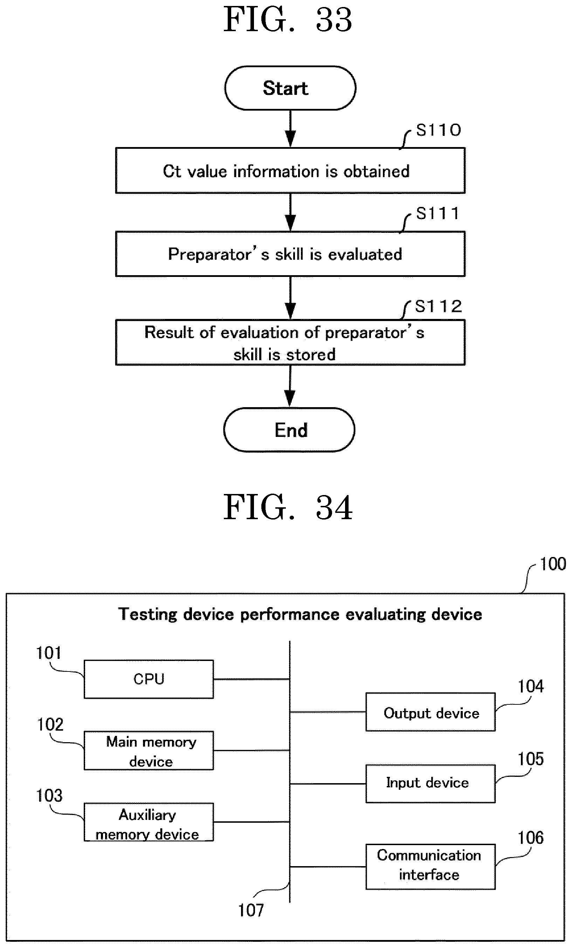

Next, the process procedures of the preparator's skill evaluating program of the present invention will be described. FIG. 33 is a flowchart illustrating the process procedures of the preparator's skill evaluating program by the control unit 130 of the preparator's skill evaluating device 100.

In the step S110, the Ct value information obtaining unit 131 of the control unit 130 of the preparator's skill evaluating device 100 obtains data representing the information on a Ct value stored in the Ct value information DB 141 of the memory unit 140, and moves the process to the step S111.

In the step S111, the skill evaluating unit 132 of the control unit 130 of the preparator's skill evaluating device 100 evaluates the skill of the preparator based on the obtained information on the Ct value, and moves the process to the step S112.

In the step S112, the control unit 130 of the preparator's skill evaluating device 100 stores the obtained result of evaluation of the skill of the preparator in the skill evaluation result DB 142 of the memory unit 140, and ends the process.

A testing device performance evaluating method, a testing device performance evaluating device, and a testing device performance evaluating program using the device of the fourth embodiment including a plurality of wells in each of which an amplifiable reagent is located in a specific copy number, and two or more groups into which the wells are divided to be varied in the specific copy number of the amplifiable reagent will be described.

[Testing device performance evaluating method, testing device performance evaluating device, and testing device performance evaluating program]

A testing device performance evaluating method is a testing device performance evaluating method for evaluating the performance of a testing device configured to test a testing target, includes a Ct value information obtaining step of obtaining information on a Ct value in the device of the present invention using the device of the present invention, and a performance evaluating step of evaluating the performance of the testing device based on the information on the Ct value, and further includes other steps as needed.

A testing device performance evaluating device is a testing device performance evaluating device configured to evaluate the performance of a testing device configured to test a testing target, and includes a Ct value information obtaining unit configured to obtain information on a Ct value in the device of the present invention using the device of the present invention, and a performance evaluating unit configured to evaluate the performance of the testing device based on the information on the Ct value, and further includes other units as needed.

A testing device performance evaluating program is a testing device performance evaluating program for evaluating the performance of a testing device configured to test a testing target, and causes a computer to execute a process including obtaining information on a Ct value in the device of the present invention using the device of the present invention and evaluating the performance of the testing device based on the information on the Ct value.

Control being performed by, for example, a control unit of the testing device performance evaluating device has the same meaning as the testing device performance evaluating method being carried out. Therefore, details of the testing device performance evaluating method will also be specified through description of the testing device performance evaluating device. Further, the testing device performance evaluating program realizes the testing device performance evaluating device with the use of, for example, computers as hardware resources. Therefore, details of the testing device performance evaluating program will also be specified through description of the testing device performance evaluating device.

<Ct value information obtaining step and Ct value information obtaining unit>

The Ct value information obtaining step is a step of obtaining information on a Ct value in the device of the present invention using the device of the present invention, and is performed by the Ct value information obtaining unit.

A Ct value can be obtained by performing real-time PCR using the device of the present invention.

Examples of the information on the Ct value include an average Ct value, standard deviation of Ct values, a CV value [(standard deviation/average Ct value)×100], and [(Ct value (Max)-Ct value (min))/2·average Ct value]×100.

Information on the Ct value may be obtained for each of two or more groups provided in the device and varied in the specific copy number of the amplifiable reagent.

<Performance evaluating step and performance evaluating unit>

The performance evaluating step is a step of evaluating the performance of the testing device based on the information on the Ct value, and is performed by the performance evaluating unit.

In a qualitative evaluation, real-time PCR is performed using the device of the present invention to measure Ct values and calculate the average Ct value. An in-plane property can be evaluated by grading a well as "○" when the Ct value in the well is within 10% of the average Ct value, and grading a well as "×" when the Ct value in the well is greater than 10% of the average Ct value.

By performing measurement for a certain period of time using the device of the present invention, it is possible to obtain temporal changes of the Ct values. Hence, like the in-plane property, when the Ct value of a well is greater than 10% of the average Ct value, it is possible to adopt a measure of calibrating the testing device or not using the measured position. Furthermore, because the specific copy number of the copies located is an absolute value, use of devices with copies located in the same specific copy number enables comparison of the performance between testing devices.

In a quantitative evaluation, by performing measurement for a certain period of time using the device of the present invention, it is possible to obtain temporal changes of the Ct values. Hence, like the in-plane property, when a value deviating from a quality control value is obtained, it is possible to adopt a measure of calibrating the testing device or not using the measured position. Furthermore, because the copy number of the copies located is an absolute value, use of devices with copies located in the same copy number enables comparison of the performance between testing devices.

In a quantitative evaluation, not a Ct value per se, but a copy number (or a concentration) corresponding to a Ct value can be obtained from a calibration curve and PCR efficiency. Therefore, the performance evaluation between testing devices may be performed using such values as a copy number (or a concentration), or a CV value converted to a copy number (or a concentration), and (Max-Min)/2·average value×100 calculated for a copy number (converted to a copy number or a concentration).

<Other steps and other units>

The other steps and the other units are not particularly limited and may be appropriately selected depending on the intended purpose. Examples of the other steps and the other units include a displaying step and a display unit.

The process according to the testing device performance evaluating program of the present invention can be executed using a computer including a control unit constituting the testing device performance evaluating device.

The hardware configuration and the functional configuration of the testing device performance evaluating device will be described below.

<Hardware configuration of testing device performance evaluating device>

FIG. 34 is a block diagram illustrating an example of a hardware configuration of a testing device performance evaluating device 100.

As illustrated in FIG. 34, the testing device performance evaluating device 100 includes a CPU (Central Processing Unit) 101, a main memory device 102, an auxiliary memory device 103, an output device 104, an input device 105, and a communication interface (communication I/F) 106. These units are coupled to one another via a bus 107.

The CPU 101 is a processing device configured to execute various controls and operations. The CPU 101 realizes various functions by executing an OS (Operating System) and programs stored in, for example, the main memory device 102. That is, in the present example, the CPU 101 functions as a control unit 130 of the testing device performance evaluating device 100 by executing the testing device performance evaluating program.

The CPU 101 also controls the operation of the testing device performance evaluating device 100 on the whole. In the present example, the CPU 101 is used as the device configured to control the operation of the testing device performance evaluating device 100 on the whole. However, this is non-limiting. For example, a FPGA (Field Programmable Gate Array) may be used.

The testing device performance evaluating program and various databases need not be stored in, for example, the main memory device 102 or the auxiliary memory device 103. The testing device performance evaluating program and various databases may be stored in, for example, any other information processing device that is coupled to the testing device performance evaluating device 100 via, for example, the Internet, a LAN (Local Area Network), and a WAN (Wide Area Network). The testing device performance evaluating device 100 may be configured to receive the testing device performance evaluating program and various databases from such another information processing device and execute the testing device performance evaluating program and various databases.

The main memory device 102 is configured to store various programs and store, for example, data needed for execution of the various programs.

The main memory device 102 includes a ROM (Read Only Memory) and a RAM (Random Access Memory) unillustrated.

The ROM stores, for example, various programs such as BIOS (Basic Input/Output System).

The RAM functions as a work area to be developed when the various programs stored in the ROM are executed by the CPU 101. The RAM is not particularly limited and may be appropriately selected depending on the intended purpose. Examples of the RAM include a DRAM (Dynamic Random Access Memory) and an SRAM (Static Random Access Memory).

The auxiliary memory device 103 is not particularly limited and may be appropriately selected depending on the intended purpose so long as the auxiliary memory device 103 can store various information. Examples of the auxiliary memory device 103 include a solid-state drive and a hard disk drive. As the auxiliary memory device 103, for example, portable memory devices such as a CD (Compact Disc) drive, a DVD (Digital Versatile Disc) drive, and a BD (Blu-ray (registered trademark) Disc) drive may also be used.

As the output device 104, for example, a display and a speaker can be used. The display is not particularly limited and an appropriate known display may be used. Examples of the display include a liquid crystal display and an organic EL display.

The input device 105 is not particularly limited and an appropriate known input device may be used so long as the input device can receive various requests to the testing device performance evaluating device 100. Examples of the input device include a keyboard, a mouse, and a touch panel.

The communication interface (communication I/F) 106 is not particularly limited and an appropriate known communication interface may be used. Examples of the communication interface include a wireless or wired communication device.

The hardware configuration described above can realize the process functions of the testing device performance evaluating device 100.

<Functional configuration of testing device performance evaluating device>

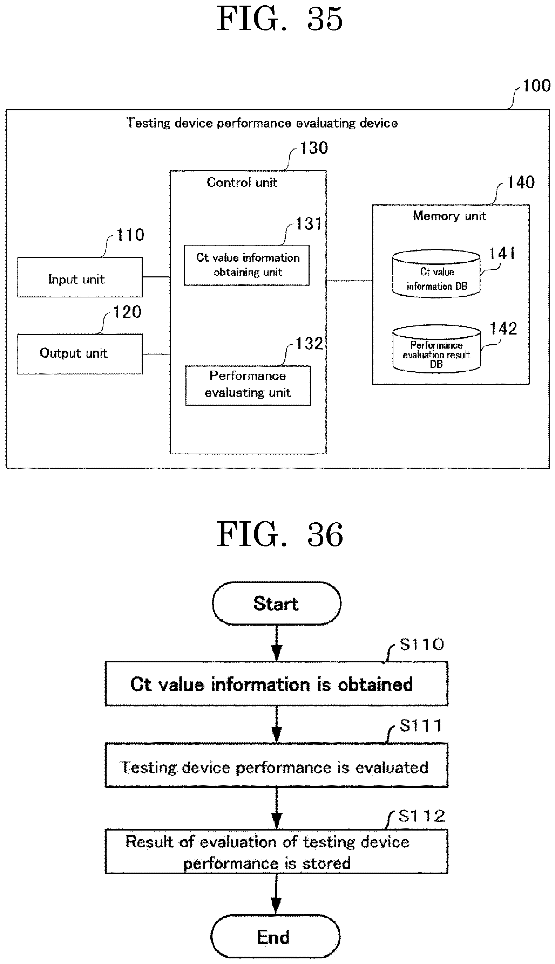

FIG. 35 is a diagram illustrating an example of a functional configuration of the testing device performance evaluating device 100.
As illustrated in FIG. 35, the testing device performance evaluating device 100 includes an input unit 110, an output unit 120, a control unit 130, and a memory unit 140.
The control unit 130 includes a Ct value information obtaining unit 131 and a performance evaluating unit 132. The control unit 130 is configured to control the testing device performance evaluating device 100 on the whole.
The memory unit 140 includes a Ct value information database 141 and a performance evaluation result database 142. Hereinafter, "database" may also be referred to as "DB".
The Ct value information obtaining unit 131 is configured to obtain information on a Ct value, using data representing the information on a Ct value stored in the Ct value information DB 141 of the memory unit 140. As described above, the Ct value information DB 141 stores, for example, data representing a Ct value previously obtained by an experiment. Note that information on a Ct value associated with the device may be stored in the Ct value information DB 141. Inputting to the DB may be performed via another information processing device coupled to the testing device performance evaluating device 100 or performed by a human operator.
The performance evaluating unit 132 is configured to evaluate the performance of a testing device based on information on a Ct value. The specific method for evaluating the performance of a testing device is as described above.
The result of evaluating the performance of the testing device by the performance evaluating unit 132 is stored in the performance evaluation result DB 142 of the memory unit 140.
Next, the process procedures of the testing device performance evaluating program of the present invention will be described. FIG. 36 is a flowchart illustrating the process procedures of the testing device performance evaluating program by the control unit 130 of the testing device performance evaluating device 100.
In the step S110, the Ct value information obtaining unit 131 of the control unit 130 of the testing device performance evaluating device 100 obtains data representing the information on a Ct value stored in the Ct value information DB 141 of the memory unit 140, and moves the process to the step S111.
In the step S111, the performance evaluating unit 132 of the control unit 130 of the testing device performance evaluating device 100 evaluates the performance of the testing device based on the obtained information on the Ct value, and moves the process to the step S112.
In the step S112, the control unit 130 of the testing device performance evaluating device 100 stores the obtained result of evaluation of the performance of the testing device in the performance evaluation result DB 142 of the memory unit 140, and ends the process.

<Device producing method>

A device producing method using cells including a specific nucleic acid as the amplifiable reagent will be described below.
The device producing method of the present invention includes a cell suspension producing step of producing a cell suspension containing a plurality of cells including a specific nucleic acid and a solvent, a liquid droplet landing step of discharging the cell suspension in the form of liquid droplets to sequentially land the liquid droplets in wells of a plate, a cell number counting step of counting the number of cells contained in the liquid droplets with a sensor after the liquid droplets are discharged and before the liquid droplets land in the wells, and a nucleic acid extracting step of extracting nucleic acids from cells in the wells, preferably includes a step of calculating uncertainty of each step, an outputting step, and a recording step, and further includes other steps as needed.

<<Cell suspension producing method>>

The cell suspension producing step is a step of producing a cell suspension containing a plurality of cells including a specific nucleic acid and a solvent.
The solvent means a liquid used for dispersing cells.
Suspension in the cell suspension means a state of cells being present dispersedly in the solvent.
Producing means a producing operation.

-Cell suspension-

The cell suspension contains a plurality of cells including a specific nucleic acid and a solvent, preferably contains an additive, and further contains other components as needed.

The plurality of cells including a specific nucleic acid are as described above.

--Solvent--

The solvent is not particularly limited and may be appropriately selected depending on the intended purpose. Examples of the solvent include water, a culture fluid, a separation liquid, a diluent, a buffer, an organic matter dissolving liquid, an organic solvent, a polymeric gel solution, a colloid dispersion liquid, an electrolytic aqueous solution, an inorganic salt aqueous solution, a metal aqueous solution, and mixture liquids of these liquids. One of these solvents may be used alone or two or more of these solvents may be used in combination. Among these solvents, water and a buffer are preferable, and water, a phosphate buffered saline (PBS), and a Tris-EDTA buffer (TE) are more preferable.

--Additive--

An additive is not particularly limited and may be appropriately selected depending on the intended purpose. Examples of the additive include a surfactant, a nucleic acid, and a resin. One of these additives may be used alone or two or more of these additives may be used in combination.

The surfactant can prevent mutual aggregation of cells and improve continuous discharging stability.

The surfactant is not particularly limited and may be appropriately selected depending on the intended purpose. Examples of the surfactant include ionic surfactants and nonionic surfactants. One of these surfactants may be used alone or two or more of these surfactants may be used in combination. Among these surfactants, nonionic surfactants are preferable because proteins are neither modified nor deactivated by nonionic surfactants, although depending on the addition amount of the nonionic surfactants.

Examples of the ionic surfactants include fatty acid sodium, fatty acid potassium, alpha-sulfo fatty acid ester sodium, sodium straight-chain alkyl benzene sulfonate, alkyl sulfuric acid ester sodium, alkyl ether sulfuric acid ester sodium, and sodium alpha-olefin sulfonate. One of these ionic surfactants may be used alone or two or more of these ionic surfactants may be used in combination. Among these ionic surfactants, fatty acid sodium is preferable and sodium dodecyl sulfonate (SDS) is more preferable.

Examples of the nonionic surfactants include alkyl glycoside, alkyl polyoxyethylene ether (e.g., BRIJ series), octyl phenol ethoxylate (e.g., TRITON X series, IGEPAL CA series, NONIDET P series, and NIKKOL OP series), polysorbates (e.g., TWEEN series such as TWEEN 20), sorbitan fatty acid esters, polyoxyethylene fatty acid esters, alkyl maltoside, sucrose fatty acid esters, glycoside fatty acid esters, glycerin fatty acid esters, propylene glycol fatty acid esters, and fatty acid monoglyceride. One of these nonionic surfactants may be used alone or two or more of these nonionic surfactants may be used in combination. Among these nonionic surfactants, polysorbates are preferable.

The content of the surfactant is not particularly limited, may be appropriately selected depending on the intended purpose, and is preferably 0.001% by mass or greater but 30% by mass or less relative to the total amount of the cell suspension. When the content of the surfactant is 0.001% by mass or greater, an effect of adding the surfactant can be obtained. When the content of the surfactant is 30% by mass or less, aggregation of cells can be suppressed, making it possible to strictly control the copy number of nucleic acid in the cell suspension.

The nucleic acid is not particularly limited and may be appropriately selected depending on the intended purpose so long as the nucleic acid does not affect detection of the detection target nucleic acid. Examples of the nucleic acid include ColE1 DNA. With such a nucleic acid, it is possible to prevent the nucleic acid having a target base sequence from adhering to the wall surface of a well.

The resin is not particularly limited and may be appropriately selected depending on the intended purpose. Examples of the resin include polyethyleneimide.

--Other materials--

Other materials are not particularly limited and may be appropriately selected depending on the intended purpose. Examples of the other materials include a crosslinking agent, a pH adjustor, an antiseptic, an antioxidant, an osmotic pressure regulator, a humectant, and a dispersant.

[Method for dispersing cells]

The method for dispersing the cells is not particularly limited and may be appropriately selected depending on the intended purpose. Examples of the method include a medium method such as a bead mill, an ultrasonic method such as an ultrasonic homogenizer, and a method using a pressure difference such as a French press. One of these methods may be used alone or two or more of these methods may be used in combination. Among these methods, the ultrasonic method is more preferable because the ultrasonic method has low damage on the cells. With the medium method, a high crushing force may destroy cellular membranes or cell walls, and the medium may mix as contamination.

[Method for screening cells]

The method for screening the cells is not particularly limited and may be appropriately selected depending on the intended purpose. Examples of the method include screening by wet classification, a cell sorter, and a filter. One of these methods may be used alone or two or more of these methods may be used in combination. Among these methods, screening by a cell sorter and a filter is preferable because the method has low damage on the cells.

It is preferable to estimate the number of nucleic acids having a target base sequence from the cell number contained in the cell suspension, by measuring the cell cycles of the cells.

Measuring the cell cycles means quantifying the cell number due to cell division.

Estimating the number of nucleic acids means obtaining the copy number of nucleic acids based on the cell number.

What is to be counted needs not be the cell number, but may be the number of target base sequences. Typically, it is safe to consider that the number of target base sequences is equal to the cell number, because the cells to be selected as the cells to be counted are cells each including one target base sequence (=one target base sequence per cell), or because one target base sequence is introduced per cell by gene recombination. However, nucleic acid replication occurs in cells in order for the cells to undergo cell division at specific cycles. Cell cycles are different depending on the kinds of cells. By extracting a predetermined amount of the solution from the cell suspension and measuring the cycles of a plurality of cells, it is possible to calculate an expected value of the number of target base sequences included in one cell and the uncertainty of the estimated value. This can be realized by, for example, observing nuclear stained cells with a flow cytometer.

Uncertainty means information on the variation in measurement results due to, for example, operations and instruments involved in production of a measurement target.

Calculation means deriving a needed value by a calculating operation.

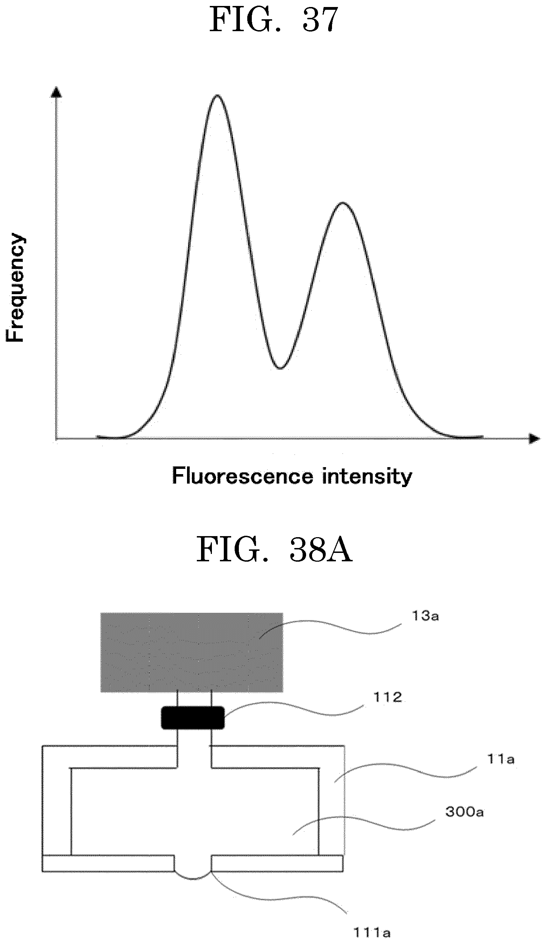

FIG. 37 is a graph plotting an example of a relationship between the frequency and the fluorescence intensity of cells in which target base sequence replication has occurred. As plotted in FIG. 37, based on presence or absence of target base sequence replication, two peaks appear on the histogram. Hence, the percentage of presence of cells in which target base sequence replication has occurred can be calculated. Based on this calculation result, the average target base sequence number included in one cell can be calculated. The estimated number of target base sequences can be calculated by multiplying the counted cell number by the obtained average target base sequence number.

It is preferable to perform an operation of controlling the cell cycles before producing the cell suspension. By preparing the cells uniformly to a state before replication occurs or a state after replication has occurred, it is possible to calculate the number of target base sequences based on the cell number more accurately.

It is preferable to calculate the uncertainty for the estimated specific copy number. By calculating the uncertainty, it is possible to express and output the uncertainty as a variance or a standard deviation based on these values. When adding up influences of a plurality of factors, it is possible use a square root of the sum of the squares of the standard deviation commonly used. For example, a correct answer percentage for the number of cells discharged, the number of DNA in a cell, and a landing ratio at which discharged cells land in wells can be used as the factors. A highly influential factor may be selected for calculation.

<<Liquid droplet landing step>>

The liquid droplet landing step is a step of discharging the cell suspension in the form of liquid droplets to sequentially land the liquid droplets in wells of a plate.

A liquid droplet means a gathering of a liquid formed by a surface tension.

Discharging means making the cell suspension fly in the form of liquid droplets.

"Sequentially" means "in order".

Landing means making liquid droplets reach the wells.

As a discharging unit, a unit configured to discharge the cell suspension in the form of liquid droplets (hereinafter may also be referred to as "discharging head") can be suitably used.

Examples of the method for discharging the cell suspension in the form of liquid droplets include an on-demand method and a continuous method that are based on the inkjet method. Of these methods, in the case of the continuous method, there is a tendency that the dead volume of the cell suspension used is high, because of, for example, empty discharging until the discharging state becomes stable, adjustment of the amount of liquid droplets, and continued formation of liquid droplets even during transfer between the wells. In the present invention, in terms of cell number adjustment, it is preferable to suppress influence due to the dead volume. Hence, of the two methods, the on-demand method is more preferable.

Examples of the on-demand method include a plurality of known methods such as a pressure applying method of applying a pressure to a liquid to discharge the liquid, a thermal method of discharging a liquid by film boiling due to heating, and an electrostatic method of drawing liquid droplets by electrostatic attraction to form liquid droplets. Among these methods, the pressure applying method is preferable for the reason described below.

In the electrostatic method, there is a need for disposing an electrode in a manner to face a discharging unit that is configured to retain the cell suspension and form liquid droplets. In the plate producing method of the present invention, a plate for receiving liquid droplets is disposed at the facing position. Hence, it is preferable not to provide an electrode, in order to increase the degree of latitude in the plate configuration.

In the thermal method, there are a risk of local heating concentration that may affect the cells, which are a biomaterial, and a risk of kogation to the heater portion. Influences by heat depend on the components contained or the purpose for which the plate is used. Therefore, there is no need for flatly rejecting the thermal method. However, the pressure applying method is preferable because the pressure applying method has a lower risk of kogation to the heater portion than the thermal method.

Examples of the pressure applying method include a method of applying a pressure to a liquid using a piezo element, and a method of applying a pressure using a valve such as an electromagnetic valve. The configuration example of a liquid droplet generating device usable for discharging liquid droplets of the cell suspension is illustrated in FIG. 38A to FIG. 38C.

FIG. 38A is an exemplary diagram illustrating an example of an electromagnetic valve-type discharging head. The electromagnetic valve-type discharging head includes an electric motor 13a, an electromagnetic valve 112, a liquid chamber 11a, a cell suspension 300a, and a nozzle 111a.

As the electromagnetic valve-type discharging head, for example, a dispenser available from Tech Elan LLC can be suitably used.

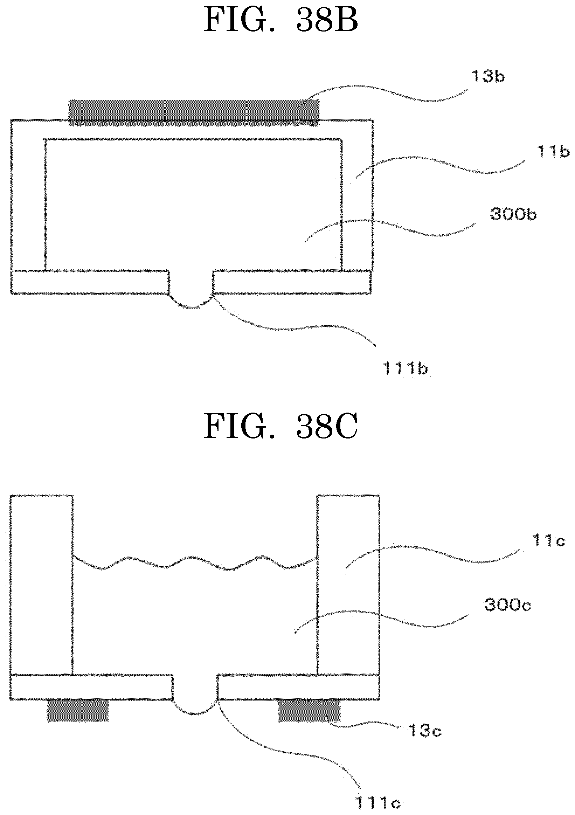

FIG. 38B is an exemplary diagram illustrating an example of a piezo-type discharging head. The piezo-type discharging head includes a piezoelectric element 13b, a liquid chamber 11b, a cell suspension 300b, and a nozzle 111b.

As the piezo-type discharging head, for example, a single cell printer available from Cytena GmbH can be suitably used.

Any of these discharging heads may be used. However, the pressure applying method by the electromagnetic valve is not capable of forming liquid droplets at a high speed repeatedly. Therefore, it is preferable to use the piezo method in order to increase the throughput of producing a plate. A piezo-type discharging head using a common piezoelectric element 13b may cause unevenness in the cell concentration due to settlement, or may have nozzle clogging.

Therefore, a more preferable configuration is the configuration illustrated in FIG. 38C. FIG. 38C is an exemplary diagram of a modified example of a piezo-type discharging head using the piezoelectric element illustrated in FIG. 38B. The discharging head of FIG. 38C includes a piezoelectric element 13c, a liquid chamber 11c, a cell suspension 300c, and a nozzle 111c.

In the discharging head of FIG. 38C, when a voltage is applied to the piezoelectric element 13c from an unillustrated control device, a compressive stress is applied in the horizontal direction of the drawing sheet. This can deform the membrane in the upward-downward direction of the drawing sheet.

Examples of any other method than the on-demand method include a continuous method for continuously forming liquid droplets. When pushing out liquid droplets from a nozzle by pressurization, the continuous method applies regular fluctuations using a piezoelectric element or a heater, to make it possible to continuously form minute liquid droplets. Further, the continuous method can select whether to land a flying liquid droplet into a well or to recover the liquid droplet in a recovery unit, by controlling the discharging direction of the liquid droplet with voltage application. Such a method is employed in a cell sorter or a flow cytometer. For example, a device named: CELL SORTER SH800 available from Sony Corporation can be used.

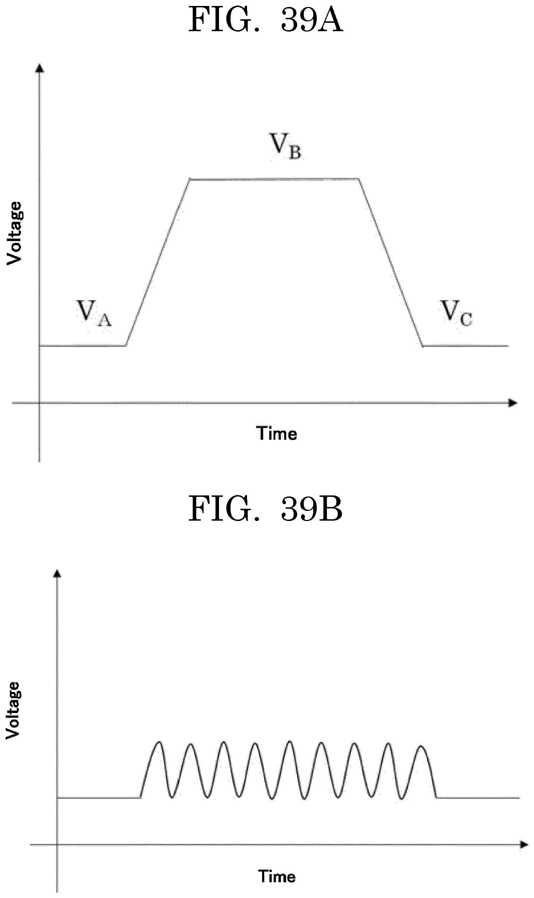

FIG. 39A is an exemplary graph plotting an example of a voltage applied to a piezoelectric element. FIG. 39B is an exemplary graph plotting another example of a voltage applied to a piezoelectric element. FIG. 7A plots a drive voltage for forming liquid droplets. Depending on the high or low level of the voltage ($V_A$, $V_B$, and $V_C$), it is possible to form liquid droplets. FIG. 39B plots a voltage for stirring the cell suspension without discharging liquid droplets.

During a period in which liquid droplets are not discharged, inputting a plurality of pulses that are not high enough to discharge liquid droplets enables the cell suspension in the liquid chamber to be stirred, making it possible to suppress occurrence of a concentration distribution due to settlement of the cells.

The liquid droplet forming operation of the discharging head that can be used in the present invention will be described below.

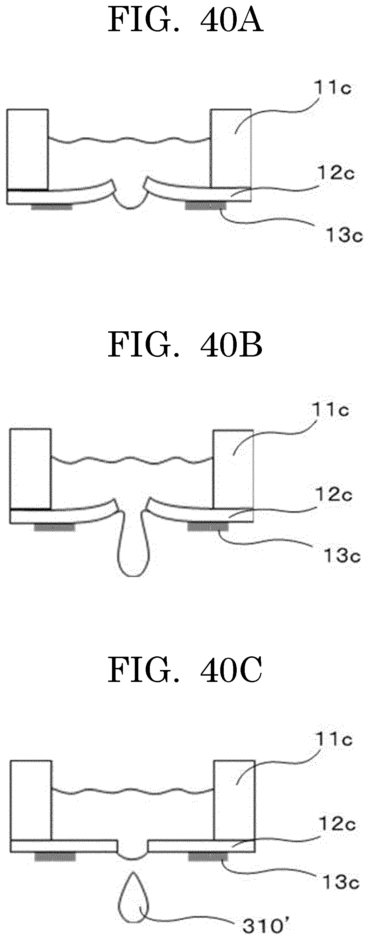

The discharging head can discharge liquid droplets with application of a pulsed voltage to the upper and lower electrodes formed on the piezoelectric element. FIG. 40A to FIG. 40C are exemplary diagrams illustrating liquid droplet states at the respective timings.

In FIG. 40A, first, upon application of a voltage to the piezoelectric element 13c, a membrane 12c abruptly deforms to cause a high pressure between the cell suspension retained in the liquid chamber 11c and the membrane 12c. This pressure pushes out a liquid droplet outward through the nozzle portion.

Next, as illustrated in FIG. 40B, for a period of time until when the pressure relaxes upward, the liquid is continuously pushed out through the nozzle portion, to grow the liquid droplet.

Finally, as illustrated in FIG. 40C, when the membrane 12c returns to the original state, the liquid pressure about the interface between the cell suspension and the membrane 12c lowers, to form a liquid droplet 310'.

In the device producing method, a plate in which wells are formed is secured on a movable stage, and by combination of driving of the stage with formation of liquid droplets from the discharging head, liquid droplets are sequentially landed in the concaves. A method of moving the plate along with moving the stage is described here. However, naturally, it is also possible to move the discharging head.

The plate is not particularly limited, and a plate that is commonly used in bio fields and in which wells are formed can be used.

The number of wells in the plate is not particularly limited and may be appropriately selected depending on the intended purpose. The number of wells may be a single number or a plural number.

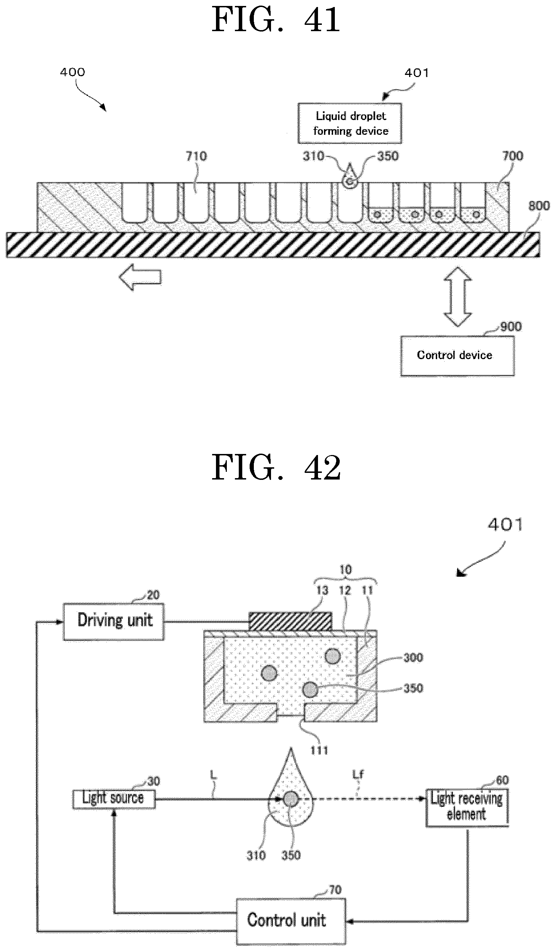

FIG. 41 is a schematic diagram illustrating an example of a dispensing device 400 configured to land liquid droplets sequentially into wells of a plate.

As illustrated in FIG. 41, the dispensing device 400 configured to land liquid droplets includes a liquid droplet forming device 401, a plate 700, a stage 800, and a control device 900.

In the dispensing device 400, the plate 700 is disposed over a movable stage 800. The plate 700 has a plurality of wells 710 (concaves) in which liquid droplets 310 discharged from a discharging head of the liquid droplet forming device 401 land. The control device 900 is configured to move the stage 800 and control the relative positional relationship between the discharging head of the liquid droplet forming device 401 and each well 710. This enables liquid droplets 310 containing fluorescent-stained cells 350 to be discharged sequentially into the wells 710 from the discharging head of the liquid droplet forming device 401.

The control device 900 may be configured to include, for example, a CPU, a ROM, a RAM, and a main memory. In this case, various functions of the control device 900 can be realized by a program recorded in, for example, the ROM being read out into the main memory and executed by the CPU. However, a part or the whole of the control device 900 may be realized only by hardware. Alternatively, the control device 900 may be configured with, for example, physically a plurality of devices.

When landing the cell suspension into the wells, it is preferable to land the liquid droplets to be discharged into the wells, in a manner that a plurality of levels are obtained.

A plurality of levels mean a plurality of references serving as standards.

As the plurality of levels, it is preferable that a plurality of cells including a specific nucleic acid have a predetermined concentration gradient in the wells. With a concentration gradient, the nucleic acid can be favorably used as a reagent for calibration curve. The plurality of levels can be controlled using values counted by a sensor.

As the plate, it is preferable to use, for example, a 1-well microtube, 8-series tubes, a 96-well plate, and a 384-well plate. When the number of wells are a plural number, it is possible to dispense the same number of cells into the wells of these plates, or it is also possible to dispense numbers of cells of different levels into the wells. There may be a well in which no cells are contained. Particularly, for producing a plate used for evaluating a real-time PCR device or digital PCR device configured to quantitatively evaluate an amount of nucleic acids, it is preferable to dispense numbers of nucleic acids of a plurality of levels. For example, it is conceivable to produce a plate into which cells (or nucleic acids) are dispensed at 7 levels, namely about 1 cell, 2 cells, 4 cells, 8 cells, 16 cells, 32 cells, and 64 cells. Using such a plate, it is possible to inspect, for example, quantitativity, linearity, and lower limit of evaluation of a real-time PCR device or digital PCR device.

<<Cell number counting step>>

The cell number counting step is a step of counting the number of cells contained in the liquid droplets with a sensor after the liquid droplets are discharged and before the liquid droplets land in the wells.

A sensor means a device configured to, by utilizing some scientific principles, change mechanical, electromagnetic, thermal, acoustic, or chemical properties of natural phenomena or artificial products or spatial information/temporal information indicated by these properties into signals, which are a different medium easily handleable by humans or machines.

Counting means counting of numbers.

The cell number counting step is not particularly limited and may be appropriately selected depending on the intended purpose, so long as the cell number counting step counts the number of cells contained in the liquid droplets with a sensor after the liquid droplets are discharged and before the liquid droplets land in the wells. The cell number counting step may include an operation for observing cells before discharging and an operation for counting cells after landing.

For counting the number of cells contained in the liquid droplets after the liquid droplets are discharged and before the liquid droplets land in the wells, it is preferable to observe cells in a liquid droplet at a timing at which the liquid droplet is at a position that is immediately above a well opening and at which the liquid droplet is predicted to enter the well in the plate without fail.

Examples of the method for observing cells in a liquid droplet include an optical detection method and an electric or magnetic detection method.

-Optical detection method-

With reference to FIG. 42, FIG. 46, and FIG. 47, an optical detection method will be described below.

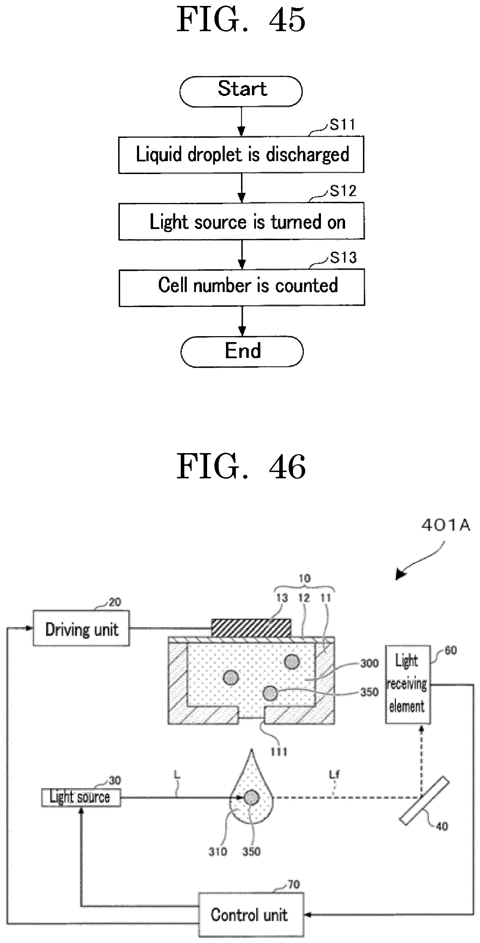

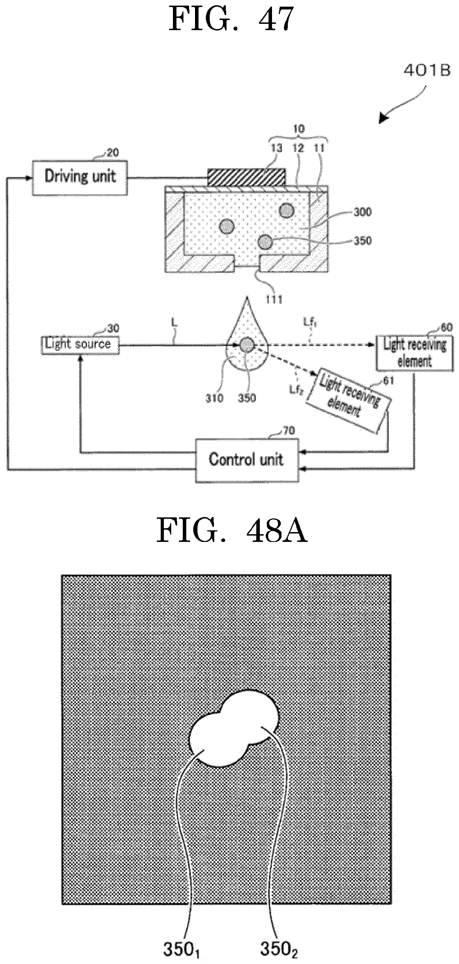

FIG. 42 is an exemplary diagram illustrating an example of a liquid droplet forming device 401. FIG. 46 and FIG. 47 are exemplary diagrams illustrating other examples of liquid droplet forming devices 401A and 401B. As illustrated in FIG. 42, the liquid droplet forming device 401 includes a discharging head (liquid droplet discharging unit) 10, a driving unit 20, a light source 30, a light receiving element 60, and a control unit 70.

In FIG. 42, a liquid obtained by dispersing cells in a predetermined solution after fluorescently staining the cells with a specific pigment is used as the cell suspension. Cells are counted by irradiating the liquid droplets formed by the discharging head with light having a specific wavelength and emitted from the light source and detecting fluorescence emitted by the cells with the light receiving element. Here, autofluorescence emitted by molecules originally contained in the cells may be utilized, in addition to the method of staining the cells with a fluorescent pigment. Alternatively, genes for producing fluorescent proteins (for example, GFP (Green Fluorescent Proteins)) may be previously introduced into the cells, in order that the cells may emit fluorescence.

Irradiation of light means application of light.

The discharging head 10 includes a liquid chamber 11, a membrane 12, and a driving element 13 and can discharge a cell suspension 300 suspending fluorescent-stained cells 350 in the form of liquid droplets.

The liquid chamber 11 is a liquid retaining portion configured to retain the cell suspension 300 suspending the fluorescent-stained cells 350. A nozzle 111, which is a through hole, is formed in the lower surface of the liquid chamber 11. The liquid chamber 11 may be formed of, for example, a metal, silicon, or a ceramic. Examples of the fluorescent-stained cells 350 include inorganic particles and organic polymer particles stained with a fluorescent pigment.

The membrane 12 is a film-shaped member secured on the upper end portion of the liquid chamber 11. The planar shape of the membrane 12 may be, for example, a circular shape, but may also be, for example, an elliptic shape or a quadrangular shape.

The driving element 13 is provided on the upper surface of the membrane 12. The shape of the driving element 13 may be designed to match the shape of the membrane 12. For example, when the planar shape of the membrane 12 is a circular shape, it is preferable to provide a circular driving element 13.

The membrane 12 can be vibrated by supplying a driving signal to the driving element 13 from a driving unit 20. The vibration of the membrane 12 can cause a liquid droplet 310 containing the fluorescent-stained cells 350 to be discharged through the nozzle 111.

When a piezoelectric element is used as the driving element 13, for example, the driving element 13 may have a structure obtained by providing the upper surface and the lower surface of the piezoelectric material with electrodes across which a voltage is to be applied. In this case, when the driving unit 20 applies a voltage across the upper and lower electrodes of the piezoelectric element, a compressive stress is applied in the horizontal direction of the drawing sheet, making it possible for the membrane 12 to vibrate in the upward-downward direction of the drawing sheet. As the piezoelectric material, for example, lead zirconate titanate (PZT) may be used. In addition, various piezoelectric materials can be used, such as bismuth iron oxide, metal niobate, barium titanate, or materials obtained by adding metals or different oxides to these materials.

The light source 30 is configured to irradiate a flying liquid droplet 310 with light L. A flying state means a state from when the liquid droplet 310 is discharged from a liquid droplet discharging unit 10 until when the liquid droplet 310 lands on the landing target. A flying liquid droplet 310 has an approximately spherical shape at the position at which the liquid droplet 310 is irradiated with the light L. The beam shape of the light L is an approximately circular shape.

It is preferable that the beam diameter of the light L be from about 10 times through 100 times as great as the diameter of the liquid droplet 310. This is for ensuring that the liquid droplet 310 is irradiated with the light L from the light source 30 without fail even when the position of the liquid droplet 310 fluctuates.

However, it is not preferable if the beam diameter of the light L is much greater than 100 times as great as the diameter of the liquid droplet 310. This is because the energy density of the light with which the liquid droplet 310 is irradiated is reduced, to lower the light volume of fluorescence Lf to be emitted upon the light L serving as excitation light, making it difficult for the light receiving element 60 to detect the fluorescence Lf.

It is preferable that the light L emitted by the light source 30 be pulse light. It is preferable to use, for example, a solid-state laser, a semiconductor laser, and a dye laser. When the light L is pulse light, the pulse width is preferably 10 microseconds or less and more preferably 1 microsecond or less. The energy per unit pulse is preferably roughly 0.1 microjoules or higher and more preferably 1 microjoule or higher, although significantly depending on the optical system such as presence or absence of light condensation.

The light receiving element 60 is configured to receive fluorescence Lf emitted by the fluorescent-stained cell 350 upon absorption of the light L as excitation light, when the fluorescent-stained cell 350 is contained in a flying liquid droplet 310. Because the fluorescence Lf is emitted to all directions from the fluorescent-stained cell 350, the light receiving element 60 can be disposed at an arbitrary position at which the fluorescence Lf is receivable. Here, in order to improve contrast, it is preferable to dispose the light receiving element 60 at a position at which direct incidence of the light L emitted by the light source 30 to the light receiving element 60 does not occur.

The light receiving element 60 is not particularly limited and may be appropriately selected depending on the intended purpose so long as the light receiving element 60 is an element capable of receiving the fluorescence Lf emitted by the fluorescent-stained cell 350. An optical sensor configured to receive fluorescence from a cell in a liquid droplet when the liquid droplet is irradiated with light having a specific wavelength is preferable. Examples of the light receiving element 60 include one-dimensional elements such as a photodiode and a photosensor. When high-sensitivity measurement is needed, it is preferable to use a photomultiplier tube and an Avalanche photodiode. As the light receiving element 60, two-dimensional elements such as a CCD (Charge Coupled Device), a CMOS (Complementary Metal Oxide Semiconductor), and a gate CCD may be used.

The fluorescence Lf emitted by the fluorescent-stained cell 350 is weaker than the light L emitted by the light source 30. Therefore, a filter configured to attenuate the wavelength range of the light L may be installed at a preceding stage (light receiving surface side) of the light receiving element 60. This enables the light receiving element 60 to obtain an extremely highly contrastive image of the fluorescent-stained cell 350. As the filter, for example, a notch filter configured to attenuate a specific wavelength range including the wavelength of the light L may be used.

As described above, it is preferable that the light L emitted by the light source 30 be pulse light. The light L emitted by the light source 30 may be continuously oscillating light. In this case, it is preferable to control the light receiving element 60 to be capable of receiving light at a timing at which a flying liquid droplet 310 is irradiated with the continuously oscillating light, to make the light receiving element 60 receive the fluorescence Lf.

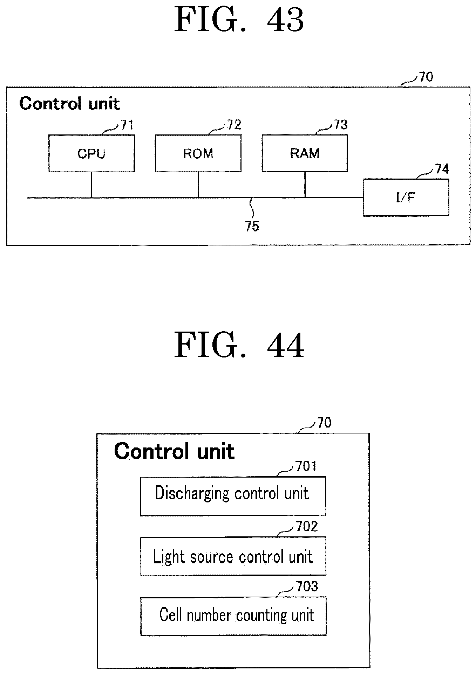

The control unit 70 has a function of controlling the driving unit 20 and the light source 30. The control unit 70 also has a function of obtaining information that is based on the light volume received by the light receiving element 60 and counting the number of fluorescent-stained cells 350 contained in the liquid droplet 310 (the case where the number is zero is also included). With reference to FIG. 43 to FIG. 48, an operation of the liquid droplet forming device 401 including an operation of the control unit 70 will be described below.

FIG. 43 is a diagram illustrating hardware blocks of the control unit of the liquid droplet forming device of FIG. 42. FIG. 44 is a diagram illustrating functional blocks of the control unit of the liquid droplet forming device of FIG. 42. FIG. 45 is a flowchart illustrating an example of the operation of the liquid droplet forming device.

As illustrated in FIG. 43, the control unit 70 includes a CPU 71, a ROM 72, a RAM 73, an I/F 74, and a bus line 75. The CPU 71, the ROM 72, the RAM 73, and the I/F 74 are coupled to one another via the bus line 75.

The CPU 71 is configured to control various functions of the control unit 70. The ROM 72 serving as a memory unit is configured to store programs to be executed by the CPU 71 for controlling the various functions of the control unit 70 and various information. The RAM 73 serving as a memory unit is configured to be used as, for example, the work area of the CPU 71. The RAM 73 is also configured to be capable of storing predetermined information for a temporary period of time. The I/F 74 is an interface configured to couple the liquid droplet forming device 401 to, for example, another device. The liquid droplet forming device 401 may be coupled to, for example, an external network via the I/F 74.

As illustrated in FIG. 44, the control unit 70 includes a discharging control unit 701, a light source control unit 702, and a cell number counting unit (cell number sensing unit) 703 as functional blocks.

With reference to FIG. 44 and FIG. 45, cell number (particle number) counting by the liquid droplet forming device 401 will be described. In the step S11, the discharging control unit 701 of the control unit 70 outputs an instruction for discharging to the driving unit 20. Upon reception of the instruction for discharging from the discharging control unit 701, the driving unit 20 supplies a driving signal to the driving element 13 to vibrate the membrane 12. The vibration of the membrane 12 causes a liquid droplet 310 containing a fluorescent-stained cell 350 to be discharged through the nozzle 111.

Next, in the step S12, the light source control unit 702 of the control unit 70 outputs an instruction for lighting to the light source 30 in synchronization with the discharging of the liquid droplet 310 (in synchronization with a driving signal supplied by the driving unit 20 to the liquid droplet discharging unit 10). In accordance with this instruction, the light source 30 is turned on to irradiate the flying liquid droplet 310 with the light L.

Here, the light is emitted by the light source 30, not in synchronization with discharging of the liquid droplet 310 by the liquid droplet discharging unit 10 (supplying of the driving signal to the liquid droplet discharging unit 10 by the driving unit 20), but in synchronization with the timing at which the liquid droplet 310 has come flying to a predetermined position in order for the liquid droplet 310 to be irradiated with the light L. That is, the light source control unit 702 controls the light source 30 to emit light at a predetermined period of time of delay from the discharging of the liquid droplet 310 by the liquid droplet discharging unit 10 (from the driving signal supplied by the driving unit 20 to the liquid droplet discharging unit 10).

For example, the speed v of the liquid droplet 310 to be discharged when the driving signal is supplied to the liquid droplet discharging unit 10 may be measured beforehand. Based on the measured speed v, the time t taken from when the liquid droplet 310 is discharged until when the liquid droplet 310 reaches the predetermined position may be calculated, in order that the timing of light irradiation by the light source 30 may be delayed from the timing at which the driving signal is supplied to the liquid droplet discharging unit 10 by the period of time of t. This enables a good control on light emission, and can ensure that the liquid droplet 310 is irradiated with the light from the light source 30 without fail.

Next, in the step S13, the cell number counting unit 703 of the control unit 70 counts the number of fluorescent-stained cells 350 contained in the liquid droplet 310 (the case where the number is zero is also included) based on information from the light receiving element 60. The information from the light receiving element 60 indicates the luminance (light volume) and the area value of the fluorescent-stained cell 350.

The cell number counting unit 703 can count the number of fluorescent-stained cells 350 by, for example, comparing the light volume received by the light receiving element 60 with a predetermined threshold. In this case, a one-dimensional element may be used or a two-dimensional element may be used as the light receiving element 60.

When a two-dimensional element is used as the light receiving element 60, the cell number counting unit 703 may use a method of performing image processing for calculating the luminance or the area of the fluorescent-stained cell 350 based on a two-dimensional image obtained from the light receiving element 60. In this case, the cell number counting unit 703 can count the number of fluorescent-stained cells 350 by calculating the luminance or the area value of the fluorescent-stained cell 350 by image processing and comparing the calculated luminance or area value with a predetermined threshold.

The fluorescent-stained cell 350 may be a cell or a stained cell. A stained cell means a cell stained with a fluorescent pigment or a cell that can express a fluorescent protein.

The fluorescent pigment for the stained cell is not particularly limited and may be appropriately selected depending on the intended purpose. Examples of the fluorescent pigment include fluoresceins, rhodamines, coumarins, pyrenes, cyanines, and azo pigments. One of these fluorescent pigments may be used alone or two or more of these fluorescent pigments may be used in combination. Among these fluorescent pigments, eosin, Evans blue, trypan blue, rhodamine 6G, rhodamine B, and Rhodamine 123 are more preferable.

Examples of the fluorescent protein include Sirius, EBFP, ECFP, mTurquoise, TagCFP, AmCyan, mTFP1, MidoriishiCyan, CFP, TurboGFP, AcGFP, TagGFP, Azami-Green, ZsGreen, EmGFP, EGFP, GFP2, HyPer, TagYFP, EYFP, Venus, YFP, PhiYFP, PhiYFP-m, TurboYFP, ZsYellow, mBanana, KusabiraOrange, mOrange, TurboRFP, DsRed-Express, DsRed2, TagRFP, DsRed-Monomer, AsRed2, mStrawberry, TurboFP602, mRFP1, JRed, KillerRed, mCherry, mPlum, PS-CFP, Dendra2, Kaede, EosFP, and KikumeGR. One of these fluorescent proteins may be used alone or two or more of these fluorescent proteins may be used in combination.

In this way, in the liquid droplet forming device 401, the driving unit 20 supplies a driving signal to the liquid droplet discharging unit 10 retaining the cell suspension 300 suspending fluorescent-stained cells 350 to cause the liquid droplet discharging unit 10 to discharge a liquid droplet 310 containing the fluorescent-stained cell 350, and the flying liquid droplet 310 is irradiated with the light L from the light source 30. Then, the fluorescent-stained cell 350 contained in the flying liquid droplet 310 emits the fluorescence Lf upon the light L serving as excitation light, and the light receiving element 60 receives the fluorescence Lf. Then, the cell number counting unit 703 counts the number of fluorescent-stained cells 350 contained in the flying liquid droplet 310, based on information from the light receiving element 60.

That is, the liquid droplet forming device 401 is configured for on-the-spot actual observation of the number of fluorescent-stained cells 350 contained in the flying liquid droplet 310. This can realize a better accuracy than hitherto obtained, in counting the number of fluorescent-stained cells 350. Moreover, because the fluorescent-stained cell 350 contained in the flying liquid droplet 310 is irradiated with the light L and emits the fluorescence Lf that is to be received by the light receiving element 60, an image of the fluorescent-stained cell 350 can be obtained with a high contrast, and the frequency of occurrence of erroneous counting of the number of fluorescent-stained cells 350 can be reduced.

FIG. 46 is an exemplary diagram illustrating a modified example of the liquid droplet forming device 401 of FIG. 42. As illustrated in FIG. 46, a liquid droplet forming device 401A is different from the liquid droplet forming device 401 (see FIG. 10) in that a mirror 40 is arranged at the preceding stage of the light receiving element 60. Description about components that are the same as in the embodiment already described may be skipped.

In the liquid droplet forming device 401A, arranging the mirror 40 at the perceiving stage of the light receiving element 60 can improve the degree of latitude in the layout of the light receiving element 60.

For example, in the layout of FIG. 42, when a nozzle 111 and a landing target are brought close to each other, there is a risk of occurrence of interference between the landing target and the optical system (particularly, the light receiving element 60) of the liquid droplet forming device 401. With the layout of FIG. 46, occurrence of interference can be avoided.

That is, by changing the layout of the light receiving element 60 as illustrated in FIG. 46, it is possible to reduce the distance (gap) between the landing target on which a liquid droplet 310 is landed and the nozzle 111 and suppress landing on a wrong position. As a result, the dispensing accuracy can be improved.

FIG. 47 is an exemplary diagram illustrating another modified example of the liquid droplet forming device 401 of FIG. 42. As illustrated in FIG. 47, a liquid droplet forming device 401B is different from the liquid droplet forming device 401 (see FIG. 42) in that a light receiving element 61 configured to receive fluorescence $Lf_2$ emitted by the fluorescent-stained cell 350 is provided in addition to the light receiving element 60 configured to receive fluorescence $Lf_1$ emitted by the fluorescent-stained cell 350. Description about components that are the same as in the embodiment already described may be skipped.

The fluorescences $Lf_1$ and $Lf_2$ represent parts of fluorescence emitted to all directions from the fluorescent-stained cell 350. The light receiving elements 60 and 61 can be disposed at arbitrary positions at which the fluorescence emitted to different directions by the fluorescent-stained cell 350 is receivable. Three or more light receiving elements may be disposed at positions at which the fluorescence emitted to different directions by the fluorescent-stained cell 350 is receivable. The light receiving elements may have the same specifications or different specifications.

With one light receiving element, when a plurality of fluorescent-stained cells 350 are contained in a flying liquid droplet 310, there is a risk that the cell number counting unit 703 may erroneously count the number of fluorescent-stained cells 350 contained in the liquid droplet 310 (a risk that a counting error may occur) because the fluorescent-stained cells 350 may overlap each other.

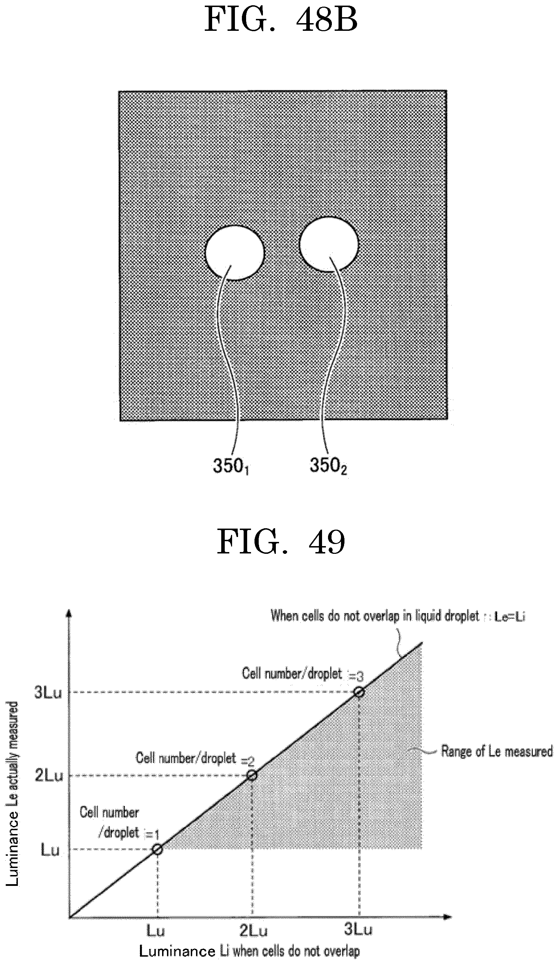

FIG. 48A and FIG. 48B are diagrams illustrating a case where two fluorescent-stained cells are contained in a flying liquid droplet. For example, as illustrated in FIG. 48A, there may be a case where fluorescent-stained cells $350_1$ and $350_2$ overlap each other, or as illustrated in FIG. 48B, there may be a case where the fluorescent-stained cells $350_1$ and $350_2$ do not overlap each other. By providing two or more light receiving elements, it is possible to reduce the influence of overlap of the fluorescent-stained cells.

As described above, the cell number counting unit 703 can count the number of fluorescent particles, by calculating the luminance or the area value of fluorescent particles by image processing and comparing the calculated luminance or area value with a predetermined threshold.

When two or more light receiving elements are installed, it is possible to suppress occurrence of a counting error, by adopting the data indicating the maximum value among the luminance values or area values obtained from these light receiving elements. This will be described in more detail with reference to FIG. 49.

FIG. 49 is a graph plotting an example of a relationship between a luminance Li when particles do not overlap each other and a luminance Le actually measured. As plotted in FIG. 49, when particles in the liquid droplet do not overlap each other, Le is equal to Li. For example, in the case where the luminance of one cell is assumed to be Lu, Le is equal to Lu when the number of cells per droplet is 1, and Le is equal to nLu when the number of particles per droplet is n (n: natural number).

However, actually, when n is 2 or greater, because particles may overlap each other, the luminance to be actually measured is $Lu \leqq Le \leqq nLu$ (the half-tone dot meshed portion in FIG. 16A). Hence, when the number of cells per droplet is n, the threshold may be set to, for example, $(nLu-Lu/2) \leqq threshold < (nLu+Lu/2)$. When a plurality of light receiving elements are installed, it is possible to suppress occurrence of a counting error, by adopting the maximum value among the data obtained from these light receiving elements. An area value may be used instead of luminance.

When a plurality of light receiving elements are installed, the number of particles may be determined according to an algorithm for estimating the number of cells based on a plurality of shape data to be obtained.

As can be understood, with the plurality of light receiving elements configured to receive fluorescence emitted to different directions by the fluorescent-stained cell 350, the liquid droplet forming device 401B can further reduce the frequency of occurrence of erroneous counting of the number of fluorescent-stained cells 350.

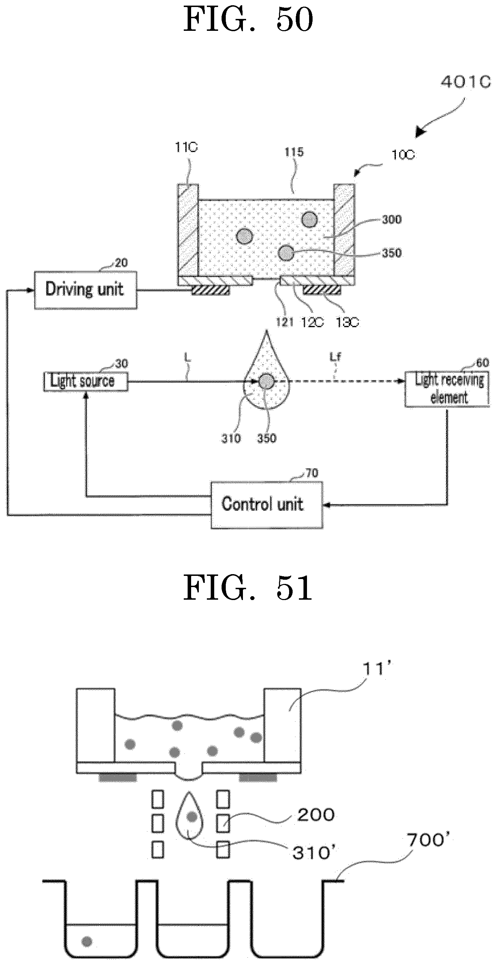

FIG. 50 is an exemplary diagram illustrating another modified example of the liquid droplet forming device 401 of FIG. 42. As illustrated in FIG. 50, a liquid droplet forming device 401C is different from the liquid droplet forming device 401 (see FIG. 42) in that a liquid droplet discharging unit 10C is provided instead of the liquid droplet discharging unit 10. Description about components that are the same as in the embodiment already described may be skipped.

The liquid droplet discharging unit 10C includes a liquid chamber 11C, a membrane 12C, and a driving element 13C. At the top, the liquid chamber 11C has an atmospherically exposed portion 115 configured to expose the interior of the liquid chamber 11C to the atmosphere, and bubbles mixed in the cell suspension 300 can be evacuated through the atmospherically exposed portion 115.

The membrane 12C is a film-shaped member secured at the lower end of the liquid chamber 11C. A nozzle 121, which is a through hole, is formed in approximately the center of the membrane 12C, and the vibration of the membrane 12C causes the cell suspension 300 retained in the liquid chamber 11C to be discharged through the nozzle 121 in the form of a liquid droplet 310. Because the liquid droplet 310 is formed by the inertia of the vibration of the membrane 12C, it is possible to discharge the cell suspension 300 even when the cell suspension 300 has a high surface tension (a high viscosity). The planer shape of the membrane 12C may be, for example, a circular shape, but may also be, for example, an elliptic shape or a quadrangular shape.

The material of the membrane 12C is not particularly limited. However, if the material of the membrane 12C is extremely flexible, the membrane 12C easily undergo vibration and is not easily able to stop vibration immediately when there is no need for discharging. Therefore, a material having a certain degree of hardness is preferable. As the material of the membrane 12C, for example, a metal material, a ceramic material, and a polymeric material having a certain degree of hardness can be used.

Particularly, when a cell is used as the fluorescent-stained cell 350, the material of the membrane is preferably a material having a low adhesiveness with the cell or proteins. Generally, adhesiveness of cells is said to be dependent on the contact angle of the material with respect to water. When the material has a high hydrophilicity or a high hydrophobicity, the material has a low adhesiveness with cells. As the material having a high hydrophilicity, various metal materials and ceramics (metal oxides) can be used. As the material having a high hydrophobicity, for example, fluororesins can be used.

Other examples of such materials include stainless steel, nickel, and aluminum, and silicon dioxide, alumina, and zirconia. In addition, it is conceivable to reduce cell adhesiveness by coating the surface of the material. For example, it is possible to coat the surface of the material with the metal or metal oxide materials described above, or coat the surface of the material with a synthetic phospholipid polymer mimicking a cellular membrane (e.g., LIPIDURE available from NOF Corporation).

It is preferable that the nozzle 121 be formed as a through hole having a substantially perfect circle shape in approximately the center of the membrane 12C. In this case, the diameter of the nozzle 121 is not particularly limited but is preferably twice or more greater than the size of the fluorescent-stained cell 350 in order to prevent the nozzle 121 from being clogged with the fluorescent-stained cell 350. When the fluorescent-stained cell 350 is, for example, an animal cell, particularly, a human cell, the diameter of the nozzle 121 is preferably 10 micrometers or greater and more preferably 100 micrometers or greater in conformity with the cell used, because a human cell typically has a size of about from 5 micrometers through 50 micrometers.

On the other hand, when a liquid droplet is extremely large, it is difficult to achieve an object of forming a minute liquid droplet. Therefore, the diameter of the nozzle 121 is preferably 200 micrometers or less. That is, in the liquid droplet discharging unit 10C, the diameter of the nozzle 121 is typically in the range of from 10 micrometers through 200 micrometers.

The driving element 13C is formed on the lower surface of the membrane 12C. The shape of the driving element 13C can be designed to match the shape of the membrane 12C. For example, when the planar shape of the membrane 12C is a circular shape, it is preferable to form a driving element 13C having an annular (ring-like) planar shape around the nozzle 121. The driving method for driving the driving element 13C may be the same as the driving method for driving the driving element 13.

The driving unit 20 can selectively (for example, alternately) apply to the driving element 13C, a discharging waveform for vibrating the membrane 12C to form a liquid droplet 310 and a stirring waveform for vibrating the membrane 12C to an extent until which a liquid droplet 310 is not formed.

For example, the discharging waveform and the stirring waveform may both be rectangular waves, and the driving voltage for the stirring waveform may be set lower than the driving voltage for the discharging waveform. This makes it possible for a liquid droplet 310 not to be formed by application of the stirring waveform. That is, it is possible to control the vibration state (degree of vibration) of the membrane 12C depending on whether the driving voltage is high or low.

In the liquid droplet discharging unit 10C, the driving element 13C is formed on the lower surface of the membrane 12C. Therefore, when the membrane 12 is vibrated by means of the driving element 13C, a flow can be generated in a direction from the lower portion to the upper portion in the liquid chamber 11C.

Here, the fluorescent-stained cells 350 move upward from lower positions, to generate a convection current in the liquid chamber 11C to stir the cell suspension 300 containing the fluorescent-stained cells 350. The flow from the lower portion to the upper portion in the liquid chamber 11C disperses the settled, aggregated fluorescent-stained cells 350 uniformly in the liquid chamber 11C.

That is, by applying the discharging waveform to the driving element 13C and controlling the vibration state of the membrane 12C, the driving unit 20 can cause the cell suspension 300 retained in the liquid chamber 11C to be discharged through the nozzle 121 in the form of a liquid droplet 310. Further, by applying the stirring waveform to the driving element 13C and controlling the vibration state of the membrane 12C, the driving unit 20 can stir the cell suspension 300 retained in the liquid chamber 11C. During stirring, no liquid droplet 310 is discharged through the nozzle 121.

In this way, stirring the cell suspension 300 while no liquid droplet 310 is being formed can prevent settlement and aggregation of the fluorescent-stained cells 350 over the membrane 12C and can disperse the fluorescent-stained cells 350 in the cell suspension 300 without unevenness. This can suppress clogging of the nozzle 121 and variation in the number of fluorescent-stained cells 350 in the liquid droplets 310 to be discharged. This makes it possible to stably discharge the cell suspension 300 containing the fluorescent-stained cells 350 in the form of liquid droplets 310 continuously for a long time.

In the liquid droplet forming device 401C, bubbles may mix in the cell suspension 300 in the liquid chamber 11C. Also in this case, with the atmospherically exposed portion 115 provided at the top of the liquid chamber 11C, the liquid droplet forming device 401C can be evacuated of the bubbles mixed in the cell suspension 300 to the outside air through the atmospherically exposed portion 115. This enables continuous, stable formation of liquid droplets 310 without a need for disposing of a large amount of the liquid for bubble evacuation.

That is, the discharging state is affected when mixed bubbles are present at a position near the nozzle 121 or when many mixed bubbles are present over the membrane 12C. Therefore, in order to perform stable formation of liquid droplets for a long time, there is a need for eliminating the mixed bubbles. Typically, mixed bubbles present over the membrane 12C move upward autonomously or by vibration of the membrane 12C. Because the liquid chamber 11C is provided with the atmospherically exposed portion 115, the mixed bubbles can be evacuated through the atmospherically exposed portion 115. This makes it possible to prevent occurrence of empty discharging even when bubbles mix in the liquid chamber 11C, enabling continuous, stable formation of liquid droplets 310.

At a timing at which a liquid droplet is not being formed, the membrane 12C may be vibrated to an extent until which a liquid droplet is not formed, in order to positively move the bubbles upward in the liquid chamber 11C.

-Electric or magnetic detection method-

In the case of the electric or magnetic detection method, as illustrated in FIG. 51, a coil 200 configured to count the number of cells is installed as a sensor immediately below a discharging head configured to discharge the cell suspension onto a plate 700' from a liquid chamber 11' in the form of a liquid droplet 310'. Cells are coated with magnetic beads that are modified with a specific protein and can adhere to the cells. Therefore, when the cells to which magnetic beads adhere pass through the coil, an induced current is generated to enable detection of presence or absence of the cells in the flying liquid droplet. Generally, cells have proteins specific to the cells on the surfaces of the cells. Modification of magnetic beads with antibodies that can adhere to the proteins enables adhesion of the magnetic beads to the cells. As such magnetic beads, a ready-made product can be used. For example, DYNABEADS (registered trademark) available from Veritas Corporation can be used.

[Operation for observing cells before discharging]

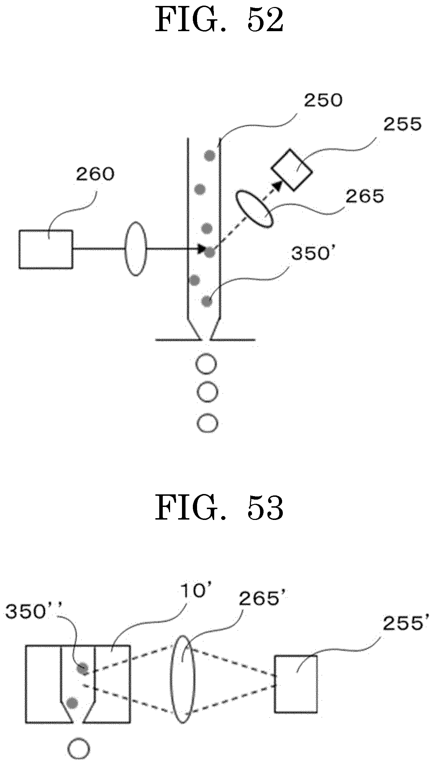

The operation for observing cells before discharging may be performed by, for example, a method for counting cells 350' that have passed through a micro-flow path 250 illustrated in FIG. 52 or a method for capturing an image of a portion near a nozzle portion of a discharging head illustrated in FIG. 53. The method of FIG. 52 is a method used in a cell sorter device, and, for example, CELL SORTER SH800 available from Sony Corporation can be used. In FIG. 52, a light source 260 emits laser light into the micro-flow path 250, and a detector 255 detects scattered light or fluorescence through a condenser lens 265. This enables discrimination of presence or absence of cells or the kind of the cells, while a liquid droplet is being formed. Based on the number of cells that have passed through the micro-flow path 250, this method enables estimation of the number of cells that have landed in a predetermined well.

As the discharging head 10' illustrated in FIG. 53, a single cell printer available from Cytena GmbH can be used. In FIG. 53, it is possible to estimate the number of cells that have landed in a predetermined well, by capturing an image of the portion near the nozzle portion with an image capturing unit 255' through a lens 265' before discharging and estimating based on the captured image that cells 350" present near the nozzle portion have been discharged, or by estimating the number of cells that are considered to have been discharged based on a difference between images captured before and after discharging. The method of FIG. 53 is more preferable because the method enables on-demand liquid droplet formation, whereas the method of FIG. 52 for counting cells that have passed through the micro-flow path generates liquid droplets continuously.

[Operation for counting cells after landing]

The operation for counting cells after landing may be performed by a method for detecting fluorescent-stained cells by observing the wells in the plate with, for example, a fluorescence microscope. This method is described in, for example, Sangjun et al., PLoS One, Volume 6(3), e17455.

Methods for observing cells before discharging a liquid droplet or after landing have the problems described below. Depending on the kind of the plate to be produced, it is the most preferable to observe cells in a liquid droplet that is being discharged. In the method for observing cells before discharging, the number of cells that are considered to have landed is counted based on the number of cells that have passed through a flow path and image observation before discharging (and after discharging). Therefore, it is not confirmed whether the cells have actually been discharged, and an unexpected error may occur. For example, there may be a case where because the nozzle portion is stained, a liquid droplet is not discharged appropriately but adheres to the nozzle plate, thus failing to make the cells in the liquid droplet land. Moreover, there may occur a problem that the cells stay behind in a narrow region of the nozzle portion, or a discharging operation causes the cells to move beyond assumption and go outside the range of observation.

The method for detecting cells on the plate after landing also have problems. First, there is a need for preparing a plate that can be observed with a microscope. As a plate that can be observed, it is common to use a plate having a transparent, flat bottom surface, particularly a plate having a bottom surface formed of glass. However, there is a problem that such a special plate is incompatible with use of ordinary wells. Further, when the number of cells is large, such as some tens of cells, there is a problem that correct counting is impossible because the cells may overlap with each other. Accordingly, it is preferable to perform the operation for observing cells before discharging and the operation for counting cells after landing, in addition to counting the number of cells contained in a liquid droplet with a sensor and a particle number (cell number) counting unit after the liquid droplet is discharged and before the liquid droplet lands in a well.

As the light receiving element, a light receiving element including one or a small number of light receiving portion(s), such as a photodiode, an Avalanche photodiode, and a photomultiplier tube may be used. In addition, a two-dimensional sensor including light receiving elements in a two-dimensional array formation, such as a CCD (Charge Coupled Device), a CMOS (Complementary Metal Oxide Semiconductor), and a gate CCD may be used.

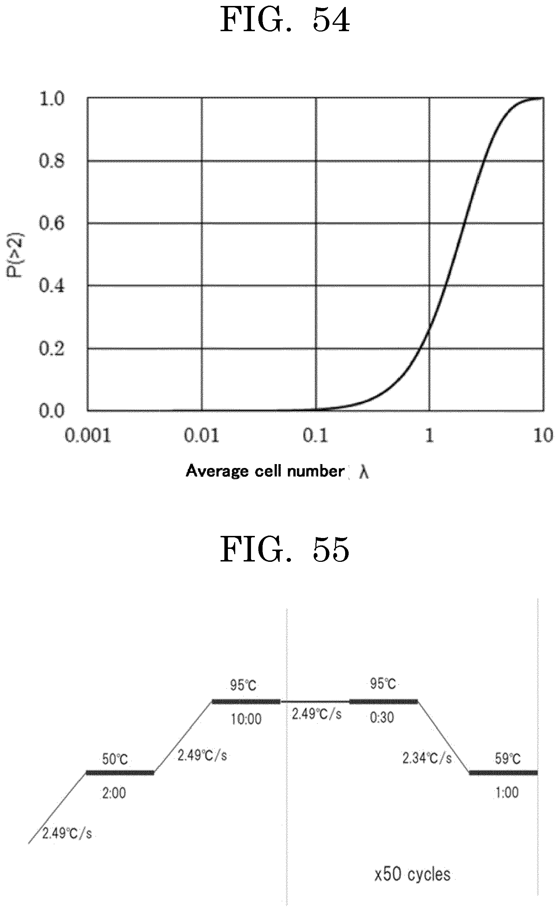

When using a light receiving element including one or a small number of light receiving portion(s), it is conceivable to determine the number of cells contained, based on the fluorescence intensity, using a calibration curve prepared beforehand. Here, binary detection of whether cells are present or absent in a flying liquid droplet is common. When the cell suspension is discharged in a state that the cell concentration is so sufficiently low that almost only 1 or 0 cell(s) will be contained in a liquid droplet, sufficiently accurate counting is available by the binary detection. On the premise that cells are randomly distributed in the cell suspension, the cell number in a flying liquid droplet is considered to conform to a Poisson distribution, and the probability P (>2) at which two or more cells are contained in a liquid droplet is represented by a formula (1) below. FIG. 54 is a graph plotting a relationship between the probability P (>2) and an average cell number. Here, $\lambda$ is a value representing an average cell number in a liquid droplet and obtained by multiplying the cell concentration in the cell suspension by the volume of a liquid droplet discharged.

$$P(>2) = 1-(1+\lambda)\times e^{-\lambda} \cdots \text{formula (1)}$$

When performing cell number counting by binary detection, in order to ensure accuracy, it is preferable that the probability P (>2) be a sufficiently low value, and that $\lambda$ satisfy: $\lambda<0.15$, at which the probability P (>2) is 1% or lower. The light source is not particularly limited and may be appropriately selected depending on the intended purpose, so long as the light source can excite fluorescence from cells. It is possible to use, for example, an ordinary lamp such as a mercury lamp and a halogen lamp to which a filter is applied for emission of a specific wavelength, a LED (Light Emitting Diode), and a laser. However, particularly when forming a minute liquid droplet of 1 nL or less, there is a need for irradiating a small region with a high light intensity. Therefore, use of a laser is preferable. As a laser light source, various commonly known lasers such as a solid-state laser, a gas laser, and a semiconductor laser can be used. The excitation light source may be a light source that is configured to continuously irradiate a region through which a liquid droplet passes or may be a light source that is configured for pulsed irradiation in synchronization with discharging of a liquid droplet at a timing delayed by a predetermined period of time from the operation for discharging the liquid droplet.

<<Step of calculating uncertainty>>

The step of calculating the uncertainty is a step of calculating the uncertainty in each of, for example, the cell suspension producing step, the liquid droplet landing step, and the cell number counting step.

The uncertainty can be calculated in the same manner as calculating the uncertainty in the cell suspension producing step.

The timing at which the uncertainties are calculated may be collectively in the next step to the cell number counting step, or may be at the end of each of, for example, the cell suspension producing step, the liquid droplet landing step, and the cell number counting step in order for the uncertainties to be synthesized in the next step to the cell number counting step. In other words, the uncertainties in these steps need only to be calculated at arbitrary timings by the time when synthesis is performed.

<<Outputting step>>

The outputting step is a step of outputting a counted value of the number of cells contained in the cell suspension that has landed in a well, counted by a particle number counting unit based on a detection result measured by a sensor.

The counted value means a number of cells contained in the well, calculated by the particle number counting unit based on the detection result measured by the sensor.

Outputting means sending a value counted by a device such as a motor, communication equipment, and a calculator upon reception of an input to an external server serving as a count result memory unit in the form of electronic information, or printing the counted value as a printed matter.

In the outputting step, an observed value or an estimated value obtained by observing or estimating the number of cells or the number of nucleic acids in each well of a plate during production of the plate is output to an external memory unit.

Outputting may be performed at the same time as the cell number counting step, or may be performed after the cell number counting step.

<<Recording step>>

The recording step is a step of recording the observed value or the estimated value output in the outputting step.

The recording step can be suitably performed by a recording unit.

Recording may be performed at the same time as the outputting step, or may be performed after the outputting step.

Recording means not only supplying information to a recording medium but also storing information in a memory unit.

<<Nucleic acid extracting step>>

The nucleic acid extracting step is a step of extracting nucleic acids from cells in the well.

Extracting means destroying, for example, cellular membranes and cell walls to pick out nucleic acids.

As the method for extracting nucleic acids from cells, there is known a method of thermally treating cells at from 90 degrees C through 100 degrees C. By a thermal treatment at 90 degrees C or lower, there is a possibility that DNA may not be extracted. By a thermal treatment at 100 degrees C or higher, there is a possibility that DNA may be decomposed. Here, it is preferable to perform thermal treatment with addition of a surfactant.

The surfactant is not particularly limited and may be appropriately selected depending on the intended purpose. Examples of the surfactant include ionic surfactants and nonionic surfactants. One of these surfactants may be used alone or two or more of these surfactants may be used in combination. Among these surfactants, nonionic surfactants are preferable because proteins are neither modified nor deactivated by nonionic surfactants, although depending on the addition amount of the nonionic surfactants.

Examples of the ionic surfactants include fatty acid sodium, fatty acid potassium, alpha-sulfo fatty acid ester sodium, sodium straight-chain alkyl benzene sulfonate, alkyl sulfuric acid ester sodium, alkyl ether sulfuric acid ester sodium, and sodium alpha-olefin sulfonate. One of these ionic surfactants may be used alone or two or more of these ionic surfactants may be used in combination. Among these ionic surfactants, fatty acid sodium is preferable and sodium dodecyl sulfate (SDS) is more preferable.

Examples of the nonionic surfactants include alkyl glycoside, alkyl polyoxyethylene ether (e.g., BRIJ series), octyl phenol ethoxylate (e.g., TRITON X series, IGEPAL CA series, NONIDET P series, and NIKKOL OP series), polysorbates (e.g., TWEEN series such as TWEEN 20), sorbitan fatty acid esters, polyoxyethylene fatty acid esters, alkyl maltoside, sucrose fatty acid esters, glycoside fatty acid esters, glycerin fatty acid esters, propylene glycol fatty acid esters, and fatty acid monoglyceride. One of these nonionic surfactants may be used alone or two or more of these nonionic surfactants may be used in combination. Among these nonionic surfactants, polysorbates are preferable.

The content of the surfactant is preferably 0.01% by mass or greater but 5.00% by mass or less relative to the total amount of the cell suspension in the well. When the content of the surfactant is 0.01% by mass or greater, the surfactant can be effective for DNA extraction. When the content of the surfactant is 5.00% by mass or less, inhibition against amplification can be prevented during PCR. As a numerical range in which both of these effects can be obtained, the range of 0.01% by mass or greater but 5.00% by mass or less is preferable.

The method described above may not be able to sufficiently extract DNA from a cell that has a cell wall. Examples of methods for such a case include an osmotic shock procedure, a freeze-thaw method, an enzymic digestive method, use of a DNA extraction kit, an ultrasonic treatment method, a French press method, and a homogenizer method. Among these methods, an enzymic digestive method is preferable because the method can save loss of extracted DNA.

<<Other steps>>

The other steps are not particularly limited and may be appropriately selected depending on the intended purpose. Examples of the other steps include an enzyme deactivating step.

-Enzyme deactivating step-

The enzyme deactivating step is a step of deactivating an enzyme. Examples of the enzyme include DNase, RNase, and an enzyme used in the nucleic acid extracting step in order to extract a nucleic acid. The method for deactivating an enzyme is not particularly limited and may be appropriately selected depending on the intended purpose. A known method can be suitably used.

The device of the present invention is widely used in, for example, biotechnology-related industries, life science industries, and health care industries, and can be used suitably for, for example, equipment configuration or generation of calibration curves, management of the accuracy of a testing device, and evaluation of the accuracy of a PCR device.

In the case of working the device for infectious diseases, the device is applicable to methods stipulated as official analytical methods or officially announced methods.

In the device of the present invention, when the amplifiable reagent contained in the device is amplified under stipulated amplification conditions, the standard deviation $\sigma$ of wells in which the Ct value is 30 or greater is 3 or less, preferably 2.5 or less, more preferably 2 or less, and particularly preferably 1.5 or less. It is preferable that among the wells in which the Ct value is 30 or greater, the wells with lower Ct values also have lower standard deviation.

For example, the stipulated amplification conditions are as described below.

<Amplification conditions>

- PCR device: QUANT STUDIO™ 12K FLEX REAL-TIME PCR SYSTEM
- Reagent: APPLIED BIOSYSTEMS™ TAQMAN™ UNIVERSAL MASTER MIX II
- Temperature: FIG. 24

It is preferable that the fourth embodiment further include a step of changing the composition except for the specific copy number of the amplifiable reagent.

<<Step of changing composition except for specific copy number of amplifiable reagent >>

What is meant by the step of changing the composition except for the specific copy number of the amplifiable reagent is that it is possible to provide combinations that may or may not contain any other primer or amplifying reagent than the nucleic acid serving as the amplifiable reagent. Specifically, one well includes (1) a composition containing a nucleic acid, a primer, and an amplifying reagent, another well includes (2) a composition containing a nucleic acid and a primer, and yet another well includes (3) a composition containing only a nucleic acid. A preparator who prepares reagent compositions adds a primer and an amplifying reagent to the compositions of (2) and (3) by a manual operation in preparation of the reagent compositions, and use the reagent compositions for a PCR reaction.

The step of changing the composition except for the specific copy number of the amplifiable reagent may be performed by machine dispensing instead of a manual operation. This makes it possible to accurately dispense the primer and the amplifying reagent.

Hence, the device enables, for example, evaluation of the skill of the preparator who prepares the reagent compositions.

Examples

The present invention will be described below by way of Examples. The present invention should not be construed as being limited to these Examples.

(Example 1)

<Production of device>

<<Preparation of nucleic acid sample>>

-Production of high-concentration nucleic acid sample dilution series-

As high-concentration nucleic acid samples, serially diluted samples were prepared using DNA600-G (available from National Institute of Advanced Industrial Science and Technology, NMIJ CRM 6205-A) as a dense nucleic acid sample and ULTRAPURE DNASE/RNASE-FREE-DISTILLED WATER (available from Thermo Fisher Scientific Inc., 10977-015, hereinafter referred to as "NFW") as a diluent solvent.

The concentrations of the serially diluted samples were determined based on gravimetry of the dense solution and the diluent solvent using an electronic balance (available from A&D company, Limited, BM-22).

-Production of yeast suspension for low-concentration nucleic acid sample series-

--Gene recombinant yeast--

For producing a recombinant, a budding yeast YIL015W BY4741 (available from ATCC, ATCC4001408) was used as a carrier cell for one copy of a specific nucleic acid sequence. The specific nucleic acid sequence was a dense nucleic acid sample DNA600-G (available from National Institute of Advanced Industrial Science and Technology, NMIJ CRM 6205-a, see SEQ ID NO. 1). In the form of a plasmid produced by arranging the specific nucleic acid sequence in tandem with URA3, which was a selectable marker, one copy of the specific nucleic acid sequence was introduced into yeast genome DNA by homologous recombination, targeting a BAR1 region of the carrier cell, to produce a gene recombinant yeast. As product information of DNA600-G, DNA600-G includes information on the uncertainty of the nucleic acid concentration.

---Culturing and cell-cycle control---

In an Erlenmeyer flask, a 90-mL fraction of the gene recombinant yeast cultured in 50 g/L of a YPD medium (available from Takara Bio Inc., CLN-630409) was mixed with 900 microliters of α1- MATING FACTOR ACETATE SALT (available from Sigma-Aldrich Co., LLC, T6901-5MG, hereinafter referred to as "α factor") prepared to 500 micrograms/mL with a Dulbecco's phosphate buffered saline (available from Thermo Fisher Scientific Inc., 14190-144, hereinafter referred to as "DPBS").

Next, the resultant was incubated with a bioshaker (available from Taitec Corporation, BR-23FH) at a shaking speed of 250 rpm at a temperature of 28 degrees C for 2 hours, to synchronize the yeast at a G0/G1 phase, to obtain a yeast suspension. For confirmation of the cell cycle of the synchronized cells, the cells were stained with SYTOX GREEN NUCLEIC ACID STAIN (device name: S7020, available from Thermo Fisher Scientific Inc.) and subjected to flow cytometry using a flow cytometer (device name: SH800Z, available from Sony Corporation) at an excitation wavelength of 488 nm. As a result, it was confirmed that the cells were synchronized at a G0/G1 phase. The ratio of cells at a G1 phase was 99.5% and the ratio of cells at a G2 phase was 0.5%.

---Fixing---

Forty-five milliliters of the synchronization-confirmed yeast suspension was transferred to a centrifuge tube (available from As One Corporation, VIO-50R) and centrifuged with a centrifugal separator (available from Hitachi, Ltd., F16RN,) at a rotation speed of 3,000 rpm for 5 minutes, with subsequent supernatant removal, to obtain yeast pellets.

Four milliliters of formalin (available from Wako Pure Chemical Industries, Ltd., 062-01661) was added to the obtained yeast pellets, and the resultant was left to stand still for 5 minutes, then centrifuged with subsequent supernatant removal, and suspended with addition of 10 mL of ethanol, to obtain a fixed yeast suspension.

---Nuclear staining---

Two hundred microliters of the fixed yeast suspension was fractionated, washed with DPBS once, and resuspended in 480 microliters of DPBS.

Next, to the resultant, 20 microliters of 20 mg/mL RNase A (available from Nippon Gene Co., Ltd., 318-06391) was added, followed by incubation with a bioshaker at 37 degrees C for 2 hours.

Next, to the resultant, 25 microliters of 20 mg/mL proteinase K (available from Takara Bio Inc., TKR-9034) was added, followed by incubation with PETIT COOL (available from Waken B Tech Co., Ltd., PETIT COOL MINI T-C) at 50 degrees C for 2 hours.

Finally, to the resultant, 6 microliters of 5 mM SYTOX GREEN NUCLEIC ACID STAIN (available from Thermo Fisher Scientific Inc., S7020) was added, followed by staining in a light-shielded environment for 30 minutes.

---Dispersing---

The stained yeast suspension was subjected to dispersion treatment using an ultrasonic homogenizer (available from Yamato Scientific Co., Ltd., LUH150,) at a power output of 30% for 10 seconds, and centrifuged for 5 minutes using a centrifuge at a rotation speed of 3,000 rpm. The supernatant was removed from the resultant. The resultant was washed with addition of DPBS (1 mL). Centrifugation and removal of the supernatant were performed a total of two times. The resultant was again suspended in DPBS (1 mL), to obtain a yeast suspension ink.

<<Filling of nucleic acid samples>>

-Filling of high-concentration nucleic acid sample dilution series-

Using a micropipetter (available from Eppendorf AG, 3120000011), the high-concentration nucleic acid sample dilution series were filled in a filling container (a 96-well flat bottom plate (available from Watson Co., Ltd., 4846-96-FS)) by 2.5 microliters per well.

-Extraction of nucleic acids from dispensed yeast cells-

For extraction of nucleic acids from the yeast cells, the filling container was incubated at 37 degrees C for 30 minutes, to dissolve the cell walls (extraction of nucleic acids), and then thermally treated at 95 degrees C for 2 minutes.

<<Pricing of samples filled>>

-Calculation of uncertainty of high-concentration nucleic acid sample series-

The high-concentration nucleic acid sample series filled had certain uncertainties for the respective specific copy number levels, due to the following factors of uncertainty.

Factor (1): Uncertainty relating to the concentration of the DNA600-G undiluted solution
This nucleic acid sample was priced with an uncertainty due to determination of the mass fraction of the total nucleic acid by nucleic acid base measurement according to isotopic dilution mass spectrometry (IDMS) and phosphorus measurement according to inductively-coupled plasma mass spectrometry (ICP-MS).
Factor (2): Uncertainty relating to the density of the diluent solvent and the dense nucleic acid sample solution
Factor (3): Uncertainty of the electronic balance in gravimetry
Factor (4): Uncertainty due to Poisson distribution
Factor (5): Uncertainty due to the micropipetter (available from Eppendorf AG) during filling of the nucleic acid samples

The uncertainty of each of these factors is presented in Table 4.
The uncertainties were synthesized by a common uncertainty synthesizing method, and the average specific copy number and the uncertainty were calculated for each level of the specific copy number finally filled. The results are presented in Table 5.

Table 4

| | | Average | Uncertainty=$1\sigma$ | Unit | Source |
|---|---|---|---|---|---|
| Factor (1) | DNA600G copy number concentration | 2.25E+09 | 1.87E+08 | Molecules /microliter | Reference material certificate of AIST |
| Factor (2) | DNA600G density | 0.997 | 1.00E-05 | Mg/ microliter | Reference material certificate 25°C of AIST |
| Factor (2) | Ultrapure water density | 0.99705 | 1.00E-05 | Mg/ microliter | 25°C |
| Factor (3) | Balance | x | 2.50E-03 | mg | AND homepage |
| Factor (4) | Poisson distribution | x | $\sqrt{x}$ | molecules | General formula of Poisson distribution |
| Factor (5) | Micropipetter | 2.5 | 1.00E-02 | microliter | Calibration certificate of micropipetter |
| *In Table 4, "X" in Factor (3) represents an arbitrary weighed value (weight), and "x" in Factor (4) represents an average specific copy number fractionated (average number of copies filled). | | | | | |

Table 5

| | Kind of liquid | Sample concentration | Weight | Predetermined number | | CV value |
|---|---|---|---|---|---|---|
| | | Average | Weighed value | Average | Uncertainty | % |
| | | copy/mioroliter | mg | copy | copy | |
| 2G | N FW | 0 | 18.60 | | | |
| | DNA600G undiluted solution | 2.25E+09 | 10.00 | | | |
| | 2G | | 28.60 | 1.96E+09 | 1.63E+08 | 8.3 |
| 200M | NFW | 0 | 91.00 | | | |
| | 2G | 7.85E+08 | 10.00 | | | |
| | 200M | | 101.00 | 1.94E+08 | 1.62E+07 | 8,. |
| 20M | NFW | 0 | 91.30 | | | |
| | 200M | 7.78E+07 | 10.00 | | | |
| | 20M | | 101.30 | 1.92E+07 | 1.60E+06 | 8.3 |
| 2M | NFW | 0 | 90.80 | | | |
| | 20M | 7.68E+06 | 9.70 | | | |
| | 2M | | 100.50 | 1.85E+06 | 1.54E+05 | 8.3 |
| 200 K | NFW | 0 | 91.00 | | | |
| | 2M | 7.41E+05 | 9.70 | | | |
| | 200K | | 100.70 | 1.78E+05 | 1.49E+04 | 8.3 |
| 20K | NFW | 0 | 136.50 | | | |
| | 200K | 7.14E+04 | 15.00 | | | |
| | 20K | | 151.50 | 1.77E+04 | 1.48E+03 | 8.4 |
| 4K | N FW | 0 | 75.90 | | | |
| | 20K | 7.07E+03 | 25.50 | | | |
| | 4K | | 101.40 | 4.44E+03 | 3.76E+02 | 8.5 |
| IK | NFW | 0 | 75.90 | | | |
| | 4K | 1.78E+03 | 25.40 | | | |
| | 1K | | 101.30 | L11E+03 | 9.87E+01 | 8.9 |
| 250 | NFW | 0 | 75.70 | | | |
| | 1K | 4.46E+02 | 25.00 | | | |
| | 250 | | 100.70 | 2.77E+02 | 2.86E+01 | 10.3 |

(continued)

| | Kind of liquid | Sample concentration | Weight | Predetermined number | | CV value |
|---|---|---|---|---|---|---|
| | | Average | Weighed value | Average | Uncertainty | % |
| | | copy/mioroliter | mg | copy | copy | |
| 70 | NFW | 0 | 75.70 | | | |
| | 250 | 1.11E+02 | 25.80 | | | |
| | 70 | | 101.50 | 7.03E+01 | 1.03E+01 | 14.7 |

*The abbreviations in Table 5 indicate the followings.
-2G: a dilute solution of the DNA600G undiluted solution
-200M: a dilute solution of 2G
-20M: a dilute solution of 200M
-2M: a dilute solution of 20M
-200k: a dilute solution of 2M
-20k: a dilute solution of 200k
-4k: a dilute solution of 20k
-1k: a dilute solution of 4k
-250: a dilute solution of 1k
-70: a dilute solution of 250
-NFW: ULTRAPURE DNASE/RNASE-FREE-DISTILLED WATER (available from Thermo Fisher Scientific Inc., 10977-015)

From the results of Table 5, the nucleic acid samples filled were successfully priced with average nucleic acid copy numbers and uncertainties for the average nucleic acid copy numbers.

-Pricing of each filling portion with uncertainty-

Each well was priced with the uncertainty calculated in the foregoing "Calculation of uncertainty of high-concentration nucleic acid sample series".
Hence, it was successful to calculate the average copy numbers of the high-concentration nucleic acid sample series and uncertainties for the average copy numbers, and price each well with the calculated values.
For filling the low-concentration nucleic acid sample series, a device can also be produced by an inkjet method as described below. The same methods as described above were employed in the steps up to fixing of the yeast suspension. Therefore, description about these steps will be skipped.

-Staining-

Five hundred microliters of the fixed yeast suspension was transferred to a 1.5 mL light-shielding tube (available from Watson, 131-915BR), centrifuged with a centrifugal separator at a rotation speed of 3,000 rpm for 5 minutes with subsequent supernatant removal, suspended sufficiently by pipetting with addition of 400 microliters of DPBS (1 mM EDTA) prepared to 1 mM EDTA (available from Tocris Bioscience, 200-449-4), then centrifuged with a centrifugal separator at a rotation speed of 3,000 rpm for 5 minutes with subsequent supernatant removal, to obtain yeast pellets. One milliliter of an Evans blue aqueous solution (available from Wako Pure Chemical Industries, Ltd., 054-04061) prepared to 1 mg/mL was added to the obtained pellets, and the resultant was stirred with a vortex for 5 minutes, then centrifuged with a centrifugal separator at a rotation speed of 3,000 rpm for 5 minutes with subsequent supernatant removal, and stirred with a vortex with addition of DPBS (1 mM EDTA), to obtain a stained yeast suspension.

-Dispersing-

The stained yeast suspension was subjected to dispersion treatment using an ultrasonic homogenizer (available from Yamato Scientific Co., Ltd., device name: LUH150) at a power output of 30% for 10 seconds, centrifuged with a centrifugal separator at a rotation speed of 3,000 rpm for 5 minutes with subsequent supernatant removal, and then washed with addition of 1 mL of DPBS. Centrifugal separation and supernatant removal were performed twice in total, and the resultant was again suspended in 1 mL of DPBS, to obtain a yeast suspension ink.

-Dispensing and cell counting-

A plate with known cell numbers was produced by counting the number of yeast cells in liquid droplets in the manner described below to discharge any of 1, 2, 4, 8, 16, 21, 64, and 128 cells as specific copy numbers per well. Specifically, with the use of the liquid droplet forming device illustrated in FIG. 47, the yeast suspension ink was sequentially discharged into each well of a 96 plate (product name: MICROAMP 96-WELL REACTION PLATE, available from Thermo Fisher Scientific Inc.), using a piezoelectricity applying-type discharging head (available in-house) as a liquid droplet discharging unit at 10 Hz.

An image of yeast cells in a liquid droplet discharged was captured using a high-sensitivity camera (available from Tokyo Instruments Inc., SCMOS PCO.EDGE) as a light receiving unit and using a YAG laser (available from Spectra-Physics, Inc., EXPLORER ONE-532-200-KE) as a light source, and the cell number was counted by image processing with image processing software IMAGE J serving as a particle number counting unit for the captured image. In this way, a plate with known cell numbers was produced.

-Extraction of nucleic acids-

With a Tris-EDTA (TE) buffer and ColE1 DNA (available from Wako Pure Chemical Industries, Ltd., 312-00434), ColE1/TE was prepared at 5 ng/microliter. With ColE1/TE, a Zymolyase solution of Zymolyase$^{(R)}$ 100T (available from Nacalai Tesque Inc., 07665-55) was prepared at 1 mg/mL.

Four microliters of the Zymolyase solution was added into each well of the produced plate with known cell numbers, incubated at 37.2 degrees C for 30 minutes, to dissolve cell walls (extraction of nucleic acids), and then thermally treated at 95 degrees C for 2 minutes, to produce a reference device.

Next, in order to consider the reliability of a result obtained from a plate with a known cell number, a plate with a known cell number was produced by dispensing cells in a specific copy number into wells and the uncertainty for a cell number of 1 was calculated. Note that it is possible to calculate uncertainties for various copy numbers, by using the method described below for each specific copy number.

-Calculation of uncertainty-

In the present Example, the number of cells in a liquid droplet, the copy number of amplifiable reagents in a cell, the number of cells in a well, and contamination were used as the factors for uncertainty.

As the number of cells in a liquid droplet, the number of cells in a liquid droplet, counted based on an analysis of an image of the liquid droplet discharged by a discharging unit, and the number of cells obtained based on microscopic observation of each liquid droplet landed on a glass slide among liquid droplets discharged by a discharging unit so as to be landed on the glass slide were used.

The copy number of nucleic acids in a cell (cell cycle) was calculated using the ratio of cells that were at a G1 phase of the cell cycle (99.5%) and the ratio of cells that were at a G2 phase (0.5%).

As the number of cells in a well, the number of discharged liquid droplets landed in a well was counted. However, in counting 96 samples in total, all of the samples were landed in the wells as liquid droplets. Therefore, as a factor, the number of cells in a well was excluded from calculation of the uncertainty.

To confirm contamination, a filtrate (4 microliters) of the ink was subjected to real-time PCR to see whether any other nucleic acid than the amplifiable reagents in the cell was mixed in the ink liquid. This was tried three times. The result was the limit of detection in all of the three tries. Therefore, as a factor, contamination was also excluded from calculation of the uncertainty.

For the uncertainty, standard deviation was calculated from the measured values of each factor and multiplied by a sensitivity coefficient, to obtain a standard uncertainty unified in the unit of the measured quantity. Based on such standard uncertainties, a synthesized standard uncertainty was calculated according to the sum-of-squares method. The synthesized standard uncertainty covered only the values in a range of about 68% of a normal distribution. Therefore, by doubling the synthesized standard uncertainty, it was possible to obtain an expanded uncertainty, which was an uncertainty that took into account a range of about 95% of the normal distribution. The results are presented in the budget sheet of Table 8 below.

Table 8

| Symbol | Factors of uncertainty | Value ($\pm$) | Probability distribution | Divisor | Standard uncertainty | Sensitivity coefficient | Standard uncertainty (in unit of measured quantity) |
|---|---|---|---|---|---|---|---|
| u1 | Number of cells in liquid droplet | 0.1037 cells | - | 1 | 0.1037 cells | 1.0290 copies/cell | 0.1067 copies |
| u2 | Number of nucleic acid molecules in cell (cell cycle) | 0.0709 copies | - | 1 | 0.0709 copies | - | 0.0709 copies |
| u3 | Number of cells in well | - | - | - | - | - | - |
| u4 | Contamination | - | - | - | - | - | - |
| uc | Synthesized standard uncertainty | | Normal distribution | | | | 0.1281 copies |
| U | Expanded uncertainty | | Normal distribution (k=2) | | | | 0.2562 copies |

In Table 8, "Symbol" means an arbitrary symbol associated with a factor of the uncertainty.

In Table 8, "Value ($\pm$)" indicates an experimental standard deviation in average value, obtained by dividing a calculated experimental standard deviation by the square root of the number of data.

In Table 8, "Probability distribution" is a probability distribution of a factor of the uncertainty. The field was left blank for type-A uncertainty evaluation, whereas either normal distribution or rectangular distribution was filled in the field for type-B uncertainty evaluation. In the present Example, only type-A uncertainty evaluation was performed. Therefore, the probability distribution field was left blank.

In Table 8, "Divisor" means a number that normalizes the uncertainty of each factor.

In Table 8, "Standard uncertainty" is a value obtained by dividing "Value ($\pm$)" by "Divisor".

In Table 8, "Sensitivity coefficient" means a value used for unification to the unit of the measured quantity.

Next, average specific copy numbers of nucleic acid samples filled in wells and uncertainties were calculated. The results are presented in Table 9. The coefficients of variation CV were calculated by dividing the uncertainty values by the average specific copy numbers.

Table 9

| Specific copy number | | Coefficient of variation CV |
|---|---|---|
| Average | Uncertainty | |
| Copy | Copy | % |
| 1.02E+00 | 1.28E-01 | 12.60 |
| 2.03E+00 | 1.81E-01 | 8.91 |
| 4.07E+00 | 2.56E-01 | 6.30 |
| 8.13E+00 | 3.62E-01 | 4.46 |
| 1.63E+01 | 5.12E-01 | 3.15 |
| 2.13E+01 | 5.87E-01 | 2.75 |
| 6.50E+01 | 1.02E+00 | 1.58 |
| 1.30E+02 | 1.45E+00 | 1.11 |

According to the inkjet method, the accuracy for dispensing a specific copy number of 1 of a nucleic acid sample, i.e., one copy of a nucleic acid sample (one yeast cell) per well was found to be $\pm 0.1281$ copies. In the case of filling one or more copies per well, the accuracy at which a specific copy number of nucleic acid samples would be filled would be determined by accumulation of this accuracy.

From the results described above, the obtained expanded uncertainty was stored as data for each device as the indicator of the variation in measurement. This would enable a user to use the indicator of the uncertainty as the reference for judging the reliability of a result of measurement in each well in an experiment. Use of the reference for judging the reliability would enable highly accurate evaluation of the performance of an analytical test.

<Confirmation of Ct value of device produced>

Using devices that were produced according to the above-described method on 0.2-mL 96-well plates and into which the reagent composition presented in Table 10 was dispensed, PCR devices (a1 and a2 models of Company A, b1 model of Company B, and c1 model of Company C) were subjected to qPCR on the conditions presented in FIG. 55, to evaluate the performance of the PCR devices to see whether the PCR devices were normal according to a Ct value management table of each model presented in Table 11. The results are presented in Table 12.

Table 10

| Cocktail | Ratio | Amount of liquid |
|---|---|---|
| TaqMan Universal PCR Master Mix | 10 | 1056.0 microliters |
| Forward Primer (10 micromoles) | 1 | 105.6 microliters |
| Reverse Primer (10 micromoles) | 1 | 105.6 microliters |
| TaqMan Probe (2 micromoles) | 2 | 211.2 microliters |
| NFW | 2 | 211.2 microliters |
| Cocktail Total volume | 16 | 1689.6 microliters |

Table 11

| Maker | Model name | Ct_Ave | Ct_Max | Ct_Min | $\sigma$ | Detection percentage |
|---|---|---|---|---|---|---|
| Company A | a1 model | 36 | 34 | 38 | 0.5 | 100% |
| Company A | a2 model | 35 | 32 | 38 | 0.5 | 100% |
| Company B | b1 model | 37 | 34 | 40 | 0.6 | 100% |
| Company C | c1 model | 36 | 33 | 39 | 0.6 | 100% |

Table 12

| Maker | Model name | Ct_Ave | Ct_Max | Ct_Min | $\sigma$ | Detection percentage | Judgment |
|---|---|---|---|---|---|---|---|
| Company A | a1 model | 35.6 | 33.9 | 37 | 0.4 | 100% | Pass |
| Company A | a2 model | 35.5 | 32.4 | 37.3 | 0.42 | 100% | Pass |
| Company B | b1 model | 36.6 | 35.3 | 38.5 | 0.41 | 100% | Pass |
| Company C | c1 model | 36.3 | 29.8 | 38.8 | 0.59 | 99.70% | Fail |

The numbers of cells located in the performance evaluation plates were of the same level, and the performance evaluation to see whether the PCR devices were normal was performed according to the Ct value management table specified for each model.

When the PCR devices were judged according to the judgment table, only the c1 model of Company C was judged as fail.

(Example 2)

<Analyte detection determination 1>

A sample was filled in an above-produced sample filling well filled with the nucleic acid serving as the amplifiable reagent. Subsequently, the testing target nucleic acid and the nucleic acid serving as the amplifiable reagent were allowed to undergo amplification reactions in the same well according to a PCR method.

Through the detection result obtaining unit and the detection result analyzing unit described above, presence or absence of the testing target was determined by the determining unit based on the result of amplification of the amplifiable reagent and the result of amplification of the testing target. When the case of (1) below was applicable, a "positive" determination was made. When the case of (2) below was applicable, a "negative" determination was made.

(1) When the amplifiable reagent was amplified and the testing target was amplified, the testing target was present and the detection result was positive.

(2) When the amplifiable reagent was amplified and the testing target was not amplified, the testing target was absent or at least less than the specific copy number of the amplifiable reagent, and the detection result was negative.

<Analyte detection determination 2>

<<Negative test for norovirus in shellfish>>

A method for testing norovirus in a shellfish will be described below as Example.

First, the mantle of the shellfish was cut away, and fatty parts attached to the mid-intestinal gland were carefully removed. The mid-intestinal gland was put in a sampling bag for a homogenizer, and crushed with addition of from 7-fold through 10-fold PBS (-). The crushed sample was cool-centrifuged at 10,000 rpm for 20 minutes. A 30% by mass sucrose solution was poured into a centrifuge tube for ultracentrifugation in an amount of about 10% by mass of the amount of the centrifuge tube. Onto the resultant, the supernatant of the cool-centrifuged sample was calmly stratified, followed by cool centrifugation at 35,000 rpm for 180 minutes. After the liquid phase was aspirated with an aspirator, the wall of the tube was quickly washed with PBS (-). Two hundred microliters of DDW was added to the sediment to float the sediment. The resultant was used as a sample to be used for extracting virus RNA.

Next, a reverse transcription reaction was performed using SUPER SCRIPT II (available from Invitrogen). A total of 15 microliters of a reaction liquid was prepared, using the sample (7.5 microliters), a 5X SSII buffer (2.25 microliters), 10 mM dNTPs (0.75 microliters), a random primer (1.0 microgram/microliter) (0.375 microliters), a ribonuclease inhibitor (33 units/microliter) (0.5 microliters), 100 mM DTT (0.75 microliters), SUPER SCRIPT II RT (200 units/microliter) (0.75 microliters), and distilled water (2.125 microliters). The reaction liquid was incubated at 42 degrees C for 1 hour. Next, the resultant was heated at 99 degrees C for 5 minutes, to deactivate the enzyme. Subsequently, the resultant was left to stand still on ice.

The sample that had undergone the reverse transcription reaction was filled in an amount of 5.0 microliters in a sample filling well filled with a nucleic acid produced in the same manner as in Example 1 to serve as the amplifiable reagent. Subsequently, the testing target nucleic acid and the nucleic acid serving as the amplifiable reagent were allowed to undergo amplification reactions in the same well according to a PCR method. The composition of the reaction liquid (total of 50 microliters) contained distilled water (33.75 microliters), a 10X EX TAQ™ buffer (5.0 microliters), dNTP (2.5 mM) (4.0 microliters), a NV primer F (50 micromoles) (0.5 microliters), a NV primer R (50 micromoles) (0.5 microliters), a primer F (50 micromoles) (0.5 microliters) for amplifying the nucleic acid serving as the amplifiable reagent, a primer R (50 micromoles) (0.5 microliters) for amplifying the nucleic acid serving as the amplifiable reagent, cDNA (sample) (5.0 microliters), and EX TAQ (5 units/microliter) (0.25 microliters).

Amplification of the amplifiable reagent was performed by PCR using T100™ THERMAL CYCLER (available from Bio-Rad Laboratories, Inc.). First, the amplifiable reagent was incubated at 50 degrees C for 2 minutes, and then 95 degrees C for 10 minutes. Subsequently, the resultant was subjected 35 times to a temperature cycle including 2 steps, namely 95 degrees C for 30 seconds and 61 degrees C for 2 minutes. Finally, the resultant was incubated at 61 degrees C for 2 minutes, and subsequently cooled to 4 degrees C, to terminate the reaction.

For the confirmation of the results, agarose electrophoresis was performed using MOTHER E-BASE™ DEVICE (available from Invitrogen™) and E-GEL™ 48 AGAROSE GELS, 4% (available from Invitrogen™). Electrophoresis was performed at 100 V for 20 minutes.

Based on the result of amplification of the testing target obtained as above, presence or absence of the testing target was determined. When the case of (1) below was applicable, a "positive" determination was made. When the case of (2) below was applicable, a "negative" determination was made.

(1) When the amplifiable reagent was amplified and the testing target was amplified, the testing target was present and the detection result was positive.

(2) When the amplifiable reagent was amplified and the testing target was not amplified, the testing target was absent or at least less than the specific copy number of the amplifiable reagent, and the detection result was negative.

In the present Example, (1) 10 cells (copies) of 600G yeast filled per well by an inkjet method (IJ) with a norovirus-containing sample added (Example of the present invention), (2) a norovirus-containing sample only, and (3) diluted 600G plasmid dispensed by a manual operation by an amount corresponding to 10 copies per well of a 96-well plate with a norovirus-containing sample added (Comparative Example to IJ) were prepared using 96-well plates.

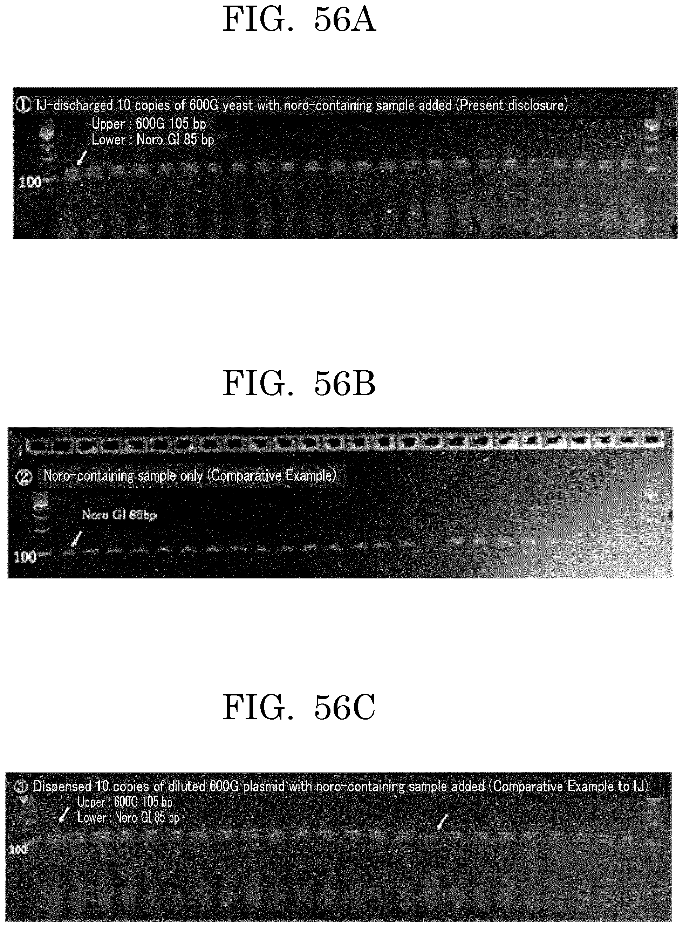

FIG. 56A is a diagram illustrating a result of agarose electrophoresis of a sample (1) performed after PCR amplification of the sample in a negative test for norovirus in a shellfish in Example 2, where the sample (1) was prepared by discharging 10 cells (copies) of 600G yeast by IJ and adding a norovirus-containing sample to the resultant (Example of the present invention).

FIG. 56B is a diagram illustrating a result of agarose electrophoresis of a sample (2) performed after PCR amplification of the sample, where the sample (2) was prepared to contain norovirus only.

FIG. 56C is a diagram illustrating a result of agarose electrophoresis of a sample (3) performed after PCR amplification of the sample in a negative test for norovirus in a shellfish in Example 2, where the sample (3) was prepared by diluting 600G plasmid, dispensing the resultant by an amount corresponding to 10 copies per well by a manual operation, and adding a norovirus-containing sample to the resultant (Comparative Example to IJ).

In (1), both of an amplification product (105 bp, upper) of 600G and an amplification product (85 bp, lower) of Noro GI were observed as two bands as illustrated in FIG. 56A to FIG. 56C.

In (2), only an amplification product (85 bp) of Noro GI was observed as a band as illustrated in FIG. 56A to FIG. 56C. The 16th sample was not observed in the band and was negative. However, it was impossible to determine definitely whether the detection target Noro GI was actually absent in the analyte sample, i.e., whether the negative determination was correct, or whether Noro GI was actually present but erroneously determined as absent (negative) due to failure of identifying, i.e., whether the determination was false-negative.

In (3), the determining method of the present invention was tried using a positive control sample produced by dilution. As illustrated in FIG. 56A to FIG. 56C, the 16th sample of the positive control sample was not observed in the band of a 600G amplification product (105 bp). This was an Example in which the positive control sample became false-negative due to variation caused by dilution. This means that in order to carry out the determining method of the present invention, it is preferable to have a high filling accuracy presented in the present invention.

(Example 3)

<Production of device for norovirus detection>

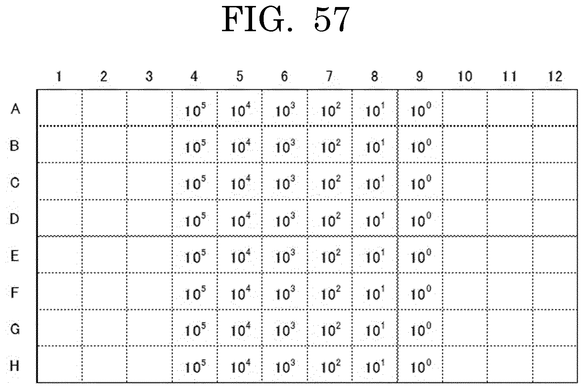

A device was produced in the manner described below. In the device, high-concentration nucleic acid sample dilution series ($1 \times 10^4$ copies/well, and $1 \times 10^5$ copies/well) containing greater than $1 \times 10^3$ copies of nucleic acid per well, and low-concentration nucleic acid sample dilution series ($1 \times 10^0$ copies/well, $1 \times 10^1$ copies/well, $1 \times 10^2$ copies/well, and $1 \times 10^3$ copies/well) containing less than or equal to $1 \times 10^3$ copies of nucleic acid per well were prepared on the device. The prepared nucleic acid dilution series were located on the position according to the positioning illustrated in FIG. 57.

-Production of high-concentration nucleic acid sample dilution series-

High-concentration nucleic acid sample dilution series were produced in the same manner as in Example 1, except that a sequence (synthesized by Fasmac Co., Ltd.) artificially synthesized by arranging the reference nucleic acid sample, which was changed from DNA600-G used in Example 1 to a partial sequence of GI-type norovirus genome (synthesized by Fasmac Co., Ltd., see SEQ ID NO. 4), in tandem with URA3 serving a selectable marker, was subjected to concentration measurement using a digital PCR (device name: QX200™ AUTODG™ DROPLET DIGITAL™ PCR SYSTEM, available from Bio-Rad Laboratories Inc.), to prepare desired concentrations (a copy number of $1 \times 10^4$/well, and a copy number of $1 \times 10^5$/well) by serial dilution. In this Example, only a partial sequence of GI-type norovirus genome was used as a specific nucleic acid sequence. However, it is also possible to locate any other specific nucleic acid sequence in each well to test a plurality of analytes simultaneously on one plate (device).

-Production of low-concentration nucleic acid sample dilution series-

--Gene recombinant yeast--

For producing a recombinant, a budding yeast YIL015W BY4741 (available from ATCC, ATCC4001408) was used as a carrier cell for one copy of a specific nucleic acid sequence.

In the form of a plasmid produced by arranging the specific nucleic acid sequence, which was a partial sequence of GI-type norovirus genome (synthesized by Fasmac Co., Ltd., see SEQ ID NO. 4) in tandem with URA3, which was a selectable marker, one copy of the specific nucleic acid sequence was introduced into yeast genome DNA by homologous recombination, targeting a BAR1 region of the carrier cell, to produce a gene recombinant yeast. In this Example, only a partial sequence of GI-type norovirus genome was used as a specific nucleic acid sequence. However, it is also possible to locate any other specific nucleic acid sequence in each well to test a plurality of analytes simultaneously on one plate (device).

--Culturing and cell-cycle control--

In an Erlenmeyer flask, a 90-mL fraction of the gene recombinant yeast cultured in 50 g/L of a YPD medium (available from Takara Bio Inc., CLN-630409) was mixed with 900 microliters of $\alpha$1- MATING FACTOR ACETATE SALT (available from Sigma-Aldrich Co., LLC, T6901-5MG, hereinafter referred to as "$\alpha$ factor") prepared to 500 micrograms/mL with a Dulbecco's phosphate buffered saline (available from Thermo Fisher Scientific Inc., 14190-144, hereinafter referred to as "DPBS").

Next, the resultant was incubated with a bioshaker (available from Taitec Corporation, BR-23FH) at a shaking speed of 250 rpm at a temperature of 28 degrees C for 2 hours, to synchronize the yeast at a G0/G1 phase, to obtain a yeast suspension.

-Fixing-

Forty-five milliliters of the synchronization-confirmed yeast suspension was transferred to a centrifuge tube (available from As One Corporation, VIO-50R) and centrifuged with a centrifugal separator (available from Hitachi, Ltd., F16RN,) at a rotation speed of 3,000 rpm for 5 minutes, with subsequent supernatant removal, to obtain yeast pellets.

Four milliliters of formalin (available from Wako Pure Chemical Industries, Ltd., 062-01661) was added to the obtained yeast pellets, and the resultant was left to stand still for 5 minutes, then centrifuged with subsequent supernatant removal, and suspended with addition of 10 mL of ethanol, to obtain a fixed yeast suspension.

-Nuclear staining-

Two hundred microliters of the fixed yeast suspension was fractionated, washed with DPBS once, and resuspended in 480 microliters of DPBS.

Next, to the resultant, 20 microliters of 20 mg/mL RNase A (available from Nippon Gene Co., Ltd., 318-06391) was added, followed by incubation with a bioshaker at 37 degrees C for 2 hours.

Next, to the resultant, 25 microliters of 20 mg/mL proteinase K (available from Takara Bio Inc., TKR-9034) was added, followed by incubation with PETIT COOL (available from Waken B Tech Co., Ltd., PETIT COOL MINI T-C) at 50 degrees C for 2 hours.

Finally, to the resultant, 6 microliters of 5 mM SYTOX GREEN NUCLEIC ACID STAIN (available from Thermo Fisher Scientific Inc., S7020) was added, followed by staining in a light-shielded environment for 30 minutes.

-Dispersing-

The stained yeast suspension was subjected to dispersion treatment using an ultrasonic homogenizer (available from Yamato Scientific Co., Ltd., LUH150,) at a power output of 30% for 10 seconds, to obtain a yeast suspension ink.

<Filling of nucleic acid samples>

-Filling of low-concentration nucleic acid sample dilution series-

--Dispensing of yeast suspension with number counting--

After a filling container (96-well flat bottom plate (available from Watson Co., Ltd., 4846-96-FS)) was filled with a dissolving liquid for dissolving cell walls in an amount of 4 microliters per well beforehand, a plate with a known cell number for the low-concentration nucleic acid sample dilution series was produced by counting the number of yeast cells in liquid droplets in the manner described below to discharge 1 cell per well. Specifically, with the use of the liquid droplet forming device illustrated in FIG. 47, the yeast suspension ink was sequentially discharged into each well of a 96 plate (product name: MICROAMP 96-WELL REACTION PLATE, available from Thermo Fisher Scientific Inc.), using a piezoelectricity applying-type discharging head (available in-house) as a liquid droplet discharging unit at 10 Hz.

An image of yeast cells in a liquid droplet discharged was captured using a high-sensitivity camera (available from Tokyo Instruments Inc., SCMOS PCO.EDGE) as a light receiving unit and using a YAG laser (available from Spectra-Physics, Inc., EXPLORER ONE-532-200-KE) as a light source, and the cell number was counted by image processing with image processing software IMAGE J serving as a particle number counting unit for the captured image. In this way, a plate with a known cell number of 1 was produced.

-Extraction of nucleic acids-

With a Tris-EDTA (TE) buffer and ColE1 DNA (available from Wako Pure Chemical Industries, Ltd., 312-00434), ColE1/TE was prepared at 5 ng/microliter. With ColE1/TE, a Zymolyase solution of Zymolyase(R) 100T (available from Nacalai Tesque Inc., 07665-55) was prepared at 1 mg/mL.

Four microliters of the Zymolyase solution was added into each well of the produced plate with a known cell number, incubated at 37.2 degrees C for 30 minutes, to dissolve cell walls (extraction of nucleic acids), and then thermally treated at 95 degrees C for 2 minutes, to produce a plate.

<Generation of calibration curve>

A PCR reaction liquid having the composition described below was added by 16 microliters per well in the produced device.

[PCR reaction liquid (Cocktail)]

| | | |
|---|---|---|
| -TAQMAN 2×UNIVERSAL PCR MASTER MIX*1: | | 10 microliters |
| -Forward primer (10 micromoles): | | 1 microliter |
| -Reverse primer (10 micromoles): | | 1 microliter |
| -TAQMAN PROBE*2: | | 2 microliters |
| -DW: | | 2 microliters |
| | Total (per well): | 16 microliters |

*1: available from Thermo Fisher Scientific Inc.
*2: modified with 5'FAM and 3'TAMRA

The primer/probe sequences used were primers and probes for GI detection having the same base sequences as in "Regarding norovirus detection methods" (annex of Food Safety Inspection issue No. 1105001 dated November 5, 2003) (final revision: Food Safety Inspection issue No. 1022, first issue, dated October 22, 2013) (hereinafter, referred to as "official analytical methods") notified by Inspection and Safety Division, Department of Food Safety, Pharmaceutical and Food Safety Bureau of Ministry of Health, Labour and Welfare.

Next, the prepared device was subjected to quantitative PCR reaction and measurement under the following conditions. The reaction and measurement were performed using QUANT STUDIO 5 available from Thermo Fisher Scientific Inc.

[Reaction conditions]

-Pre-heat-

- 50 degrees C for 2 minutes
- 95 degrees C for 10 minutes

- Cycle- (50 cycles)
- 95 degrees C for 30 seconds
- 61 degrees C for 1 minute

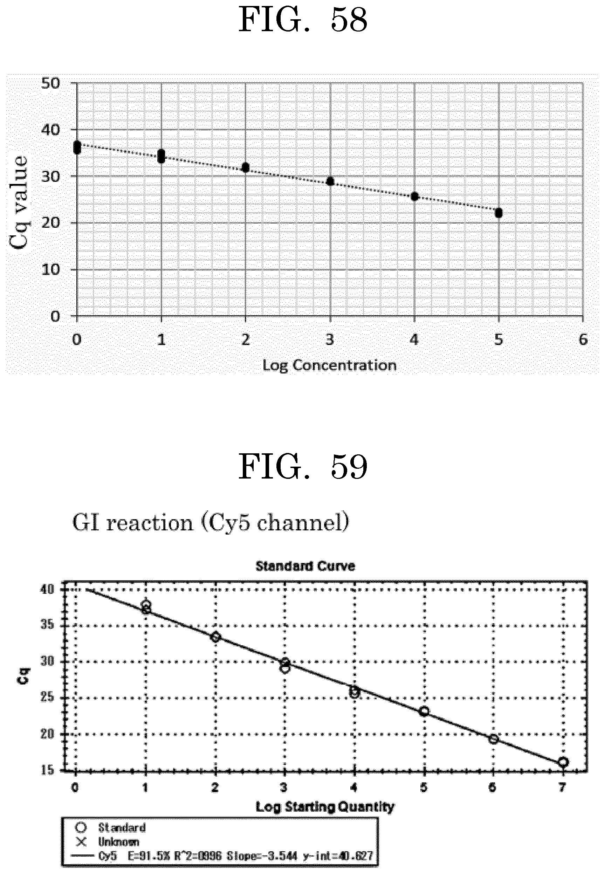

The results were analyzed with Auto setting without defining Baseline Threshold. The results are presented in FIG. 58.

With the device of the present invention, it was confirmed that dispensing of cells of the samples having the concentrations of lower than or equal to $10^3$ copies/well by an inkjet method with number counting enabled even one copy to be located in the wells accurately, and desired concentrations lower than or equal to 10 copies successfully fell on the same calibration curve on which desired concentrations higher than or equal to 10 copies fell.

(Comparative Example 3-1)

Next, a calibration curve was generated in the same manner as in Example, except that serial dilution was performed in the manner described below using GI-type nucleic acid and an attached control diluting fluid (hereinafter, may also be referred to as diluting fluid) of a commercially available norovirus quantification kit available from Toyobo Co., Ltd. (norovirus quantification kit G1/G2, undiluted solution: $2 \times 10^6$ copies/microliter). In this norovirus quantification kit, GI and GII were provided under concentration control to $2 \times 10^6$ copies/microliter in DNA states.

[Serial dilution of norovirus quantification kit]

1: $2 \times 10^6$ copies/microliter (undiluted solution)
2: $2 \times 10^5$ copies/microliter (solution of 1: 3 microliters+diluting fluid: 27 microliters)
3: $2 \times 10^4$ copies/microliter (solution of 2: 3 microliters+diluting fluid: 27 microliters)
4: $2 \times 10^3$ copies/microliter (solution of 3: 3 microliters+diluting fluid: 27 microliters)
5: $2 \times 10^2$ copies/microliter (solution of 4: 3 microliters+diluting fluid: 27 microliters)
6: $2 \times 10^1$ copies/microliter (solution of 5: 3 microliters+diluting fluid: 27 microliters)
7: $2 \times 10^0$ copies/microliter (solution of 6: 3 microliters+diluting fluid: 27 microliters)
8: $2 \times 10^{-1}$ copies/microliter (solution of 7: 3 microliters+diluting fluid: 27 microliters)

The prepared dilute solutions 1 to 8 were each prepared as a PCR reaction liquid having the composition described below, using the reagents (a reaction liquid, an enzyme solution, and a diluting fluid) attached to the norovirus quantification kit. For each dilute solution, three samples were prepared as the PCR reaction liquid.

[PCR reaction liquid]

| | | |
|---|---|---|
| -Reaction liquid: | | 10 microliters |
| -Enzyme solution: | | 5 microliters |
| -Primer liquid[*3] : | | 2.5 microliters |
| -Probe liquid[*3] : | | 2.5 microliters |
| -Diluting fluid: | | 5 microliters |
| | Total (per well): | 25 microliters |
| *3: The same ones as used in Example were used as the primer liquid and the probe liquid. | | |

The theoretical nucleic acid copy numbers in the PCR reaction liquids of the dilute solutions 1 to 8 are as presented in Table 13.

Table 13

| | Dilution series (copy number/microliter) | Theoretical value (copy number/5 microliters) |
|---|---|---|
| 1 | $2 \times 10^6$ | $1 \times 10^7$ |
| 2 | $2 \times 10^5$ | $1 \times 10^6$ |

(continued)

|   | Dilution series (copy number/microliter) | Theoretical value (copy number/5 microliters) |
|---|---|---|
| 3 | $2\times10^4$ | $1\times10^5$ |
| 4 | $2\times10^3$ | $1\times10^4$ |
| 5 | $2\times10^2$ | $1\times10^3$ |
| 6 | $2\times10^1$ | $1\times10^2$ |
| 7 | $2\times10^0$ | $1\times10^1$ |
| 8 | $2\times10^{-1}$ | $1\times10^0$ |

Next, the prepared norovirus nucleic acid dilute solutions were subjected to quantitative PCR reaction and measurement under the following conditions. The reaction and measurement were performed using CFX96 TOUCH™ DEEP WELL available from Bio-Rad Laboratories, Inc.

[Reaction conditions]

- Reverse transcription reaction: 50 degrees C for 5 minutes
- Pre-modification: 95 degrees C for 30 seconds
- Cycle-(40 cycles)
- Modification: 95 degrees C for 5 seconds
- Association/elongation: 54 degrees C for 30 seconds (detection)

The results were analyzed with User Defined of Baseline Threshold set to 20.0. The results are presented in FIG. 59.

From the results of Comparative Example, it was revealed that the results of the respective dilution series (concentrations) were not considerably dispersed, but one copy was unmeasurable and not observed to be amplified. Therefore, in Comparative Example, it was unsuccessful to determine whether the calibration curve of the concentrations lower than or equal to 10 copies was on the same straight line as the calibration curve of the concentrations higher than or equal to 10 copies.

As compared with Comparative Example in which the number of samples was 3 (N=3), Example according to the device of the present invention was analyzed based on N=8 and nevertheless turned out to have a lower variation.

From the above results, it was revealed that because a Ct value for one copy that could not be achieved with the commercially available kit of Comparative Example could be expressed on a calibration curve to be obtained with the device of the present invention, a calibration curve for lower than or equal to 10 copies could be reliably generated with the device of the present invention. Because Example and Comparative Example were carried out with the same primer and the same probe, a measuring system having the capacity of amplifying one copy was used in the same manner in both of Example and Comparative Example. Nevertheless, only the commercially available kit failed to amplify one copy. This is considered to be because the target nucleic acid molecule was not contained in the serially diluted solution of 1 copy/5 microliters of the commercially available kit. That is, concentration errors that occurred in the repeating steps of dilution accumulated, to make the concentrations in the very low copy region fall out of the assumed concentrations (desired nucleic acid concentrations). On the other hand, the method of the present invention of defining the copy number to be dispensed by the inkjet method with number counting enabled dispensing of one copy. Therefore, even when N=8 samples were tested, all of the samples were positive, making it possible to obtain a Ct value having a high accuracy.

Hence, use of the present invention makes it possible to determine whether testing was carried out in an appropriate manner, when a target of lower than or equal to 10 copies was added in one quantitative PCR reaction. Further, the device of the present invention can also be used as an effective accuracy management tool for testing in which mixing of even a very trace pathogen should not be overlooked, such as norovirus. In this Example, only a partial sequence of GI-type norovirus genome was used as a specific nucleic acid sequence. However, it is also possible to locate any other specific nucleic acid sequence in each well to test a plurality of analytes simultaneously on one plate (device).

(Example 4)

A device (plate) with a known cell number was produced in the same manner as described above in Example 1, except that the number of cells to be dispensed per well was changed to 10 cells.

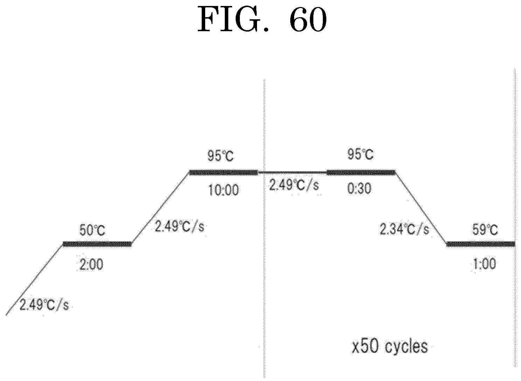

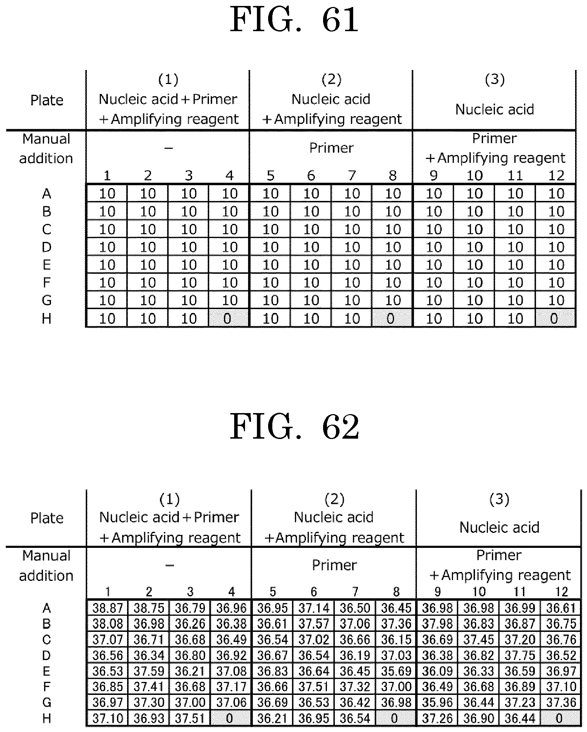

On the produced plate, amplification and detection by real-time PCR was performed according to the protocol illustrated in FIG. 60. First, for measurement by real-time PCR, at least any one of a primer and an amplifying reagent was added by a manual operation in order to obtain (1) an addition completed group (the first to the fourth columns in FIG. 61, composition: a nucleic acid, a primer, and an amplifying reagent), (2) a group for addition of a primer (the fifth to eighth columns in FIG. 61, composition: a nucleic acid and an amplifying reagent), and (3) a group for addition of a primer and an amplifying reagent (the ninth to twelfth columns in FIG. 61, composition: a nucleic acid). Note that "0" in FIG. 61 indicates negative controls, i.e., wells in which no nucleic acid was contained.

The composition of the amplifying reagent was as follows.

- Master Mix (available from Thermo Fisher Scientific Inc., TAQMAN UNIVERSAL PCR MASTER MIX): 1 microliter
- Forward primer and reverse primer for amplifying a specific base sequence (10 micromoles): 0.5 nmol
- TaqMan Probe (available from Thermo Fisher Scientific Inc., product name: TAQMAN UNIVERSAL PCR MASTER MIX): 0.4 nmol
- Yeast cell wall lytic enzyme (available from MC Food Specialties Inc., product name: ZYMOLYASE): 4 microliters
- NFW (available from Thermo Fisher Scientific Inc., product name: ULTRAPURE DNASE/RNASE-FREE DISTILLED WATER): 2 microliters

After the preparation of the plate, amplification and detection was performed using a real-time PCR device (available from Thermo Fisher Scientific Inc., QUANTSTUDIO 7FLEX). The results are presented in FIG. 62 to FIG. 64.

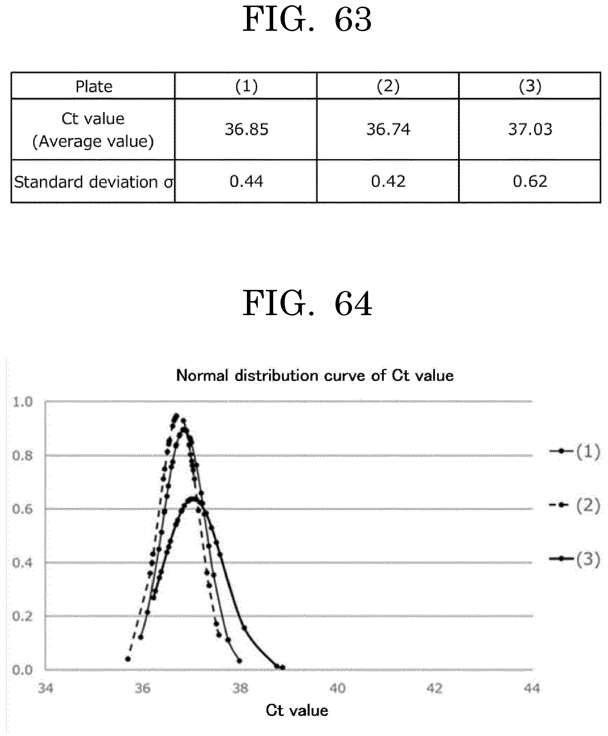

FIG. 62 is a diagram indicating information on Ct values in the respective wells of the plate illustrated in FIG. 61. FIG. 63 is a diagram indicating the average value and standard deviation of the Ct values of the composition of each of (1) to (3) obtained from FIG. 62. FIG. 64 is a graph plotting a normal distribution curve of the Ct values generated based on the information on the Ct values obtained from FIG. 62. From the results indicated in FIG. 62 to FIG. 64, it was found that the composition of (3) to which the primer and the amplifying reagent were added by the preparator achieved a greater average value and a greater standard deviation of the Ct values than the compositions of (1) and (2). As a result, it was found possible to evaluate the skill of the preparator who prepared reagent compositions, by using the device of the present invention.

As presented in Examples 1 to 4, the nucleic acid is located in the wells of the device accurately in a number (copy number) as true as possible to the desired number. This makes it possible to perform, for example, false-negative determination of an analyte, particularly norovirus, evaluation of the skill of a preparator, and evaluation of the performance of a testing device accurately. Combination of these Examples also makes it possible to perform, for example, false-negative determination of an analyte, particularly norovirus, evaluation of the skill of a preparator, and evaluation of the performance of a testing device even more accurately.

Aspects of the present invention are, for example, as follows.

<A1> A detection determining device used in a detection determining method including, in detection of a testing target in a sample by amplification of the testing target and an amplifiable reagent, determining that the testing target is present and a detection result is positive when the amplifiable reagent is amplified and the testing target is amplified, and determining that the testing target is absent or at least less than the specific copy number of the amplifiable reagent and a detection result is negative when the amplifiable reagent is amplified and the testing target is not amplified, the detection determining device including
at least one well,
wherein the amplifiable reagent in the at least one well is contained in a specific copy number.
<A2> The detection determining device according to <A1>, including
information on the specific copy number,
wherein the information on the specific copy number is at least any one selected from the group consisting of information on uncertainty of the specific copy number and a coefficient of variation CV of the specific copy number.
<A3> The detection determining device according to <A1> to <A2>,
wherein the amplifiable reagent is a nucleic acid of a partial base sequence of norovirus genome.
<A4> The detection determining device according to any one of <A1> to <A3>, including
two or more groups into which the at least one well is divided to be varied in composition in the at least one well.
<A5> The detection determining device according to <A4>, including
two or more groups into which the at least one well in which a reagent composition containing the amplifiable reagent in the specific copy number is located is divided, to have the amplifiable reagent located in the same specific copy number but to be varied in composition of the reagent composition except for the specific copy number, or two or more groups into which the at least one well in which the amplifiable reagent is located in the specific copy number is divided, to be varied in the specific copy number of the amplifiable reagent.

<B1> A device including:

at least one well,
an amplifiable reagent contained in a specific copy number in the at least one well.

<B2> The device according to <B1>, including
information on the specific copy number of the amplifiable reagent.
<B3> The device according to <2>, including
information on uncertainty as the information on the specific copy number,
wherein the information on the uncertainty includes information on a coefficient of variation CV of the amplifiable reagent, and
wherein the coefficient of variation CV satisfies $CV < 1/\sqrt{x}$, which is a relational expression with respect to an average specific copy number x of the amplifiable reagent.
<B4> The device according to any one of <1> to <3>, including
a plurality of wells in each of which the amplifiable reagent is contained,
wherein the amplifiable reagent is contained in the wells in the same specific copy number.
<B5> The device according to any one of <B1> to <B4>, including:
a plurality of wells in each of which the amplifiable reagent is contained; and
information on uncertainty of the device as a whole, the uncertainty being based on the specific copy number of the amplifiable reagent contained in each of the wells.
<B6> The device according to any one of <B1> to <B5>,
wherein the specific copy number is 1 or greater but 1,000 or less.
<B7> The device according to any one of <B1> to <B6>, further including
an identifier unit configured to enable identifying information on the specific copy number.
<B8> The device according to any one of <B1> to <B7>,
wherein the amplifiable reagent is encapsulated in a carrier.
<B9> The device according to any one of <B1> to <B8>,
wherein the amplifiable reagent is a nucleic acid.
<B10> The device according to <B9>,
wherein the nucleic acid is introduced in a nucleic acid in a nucleus of a cell.
<B11> The device according to any one of <B1> to <B10>, further including
at least any one selected from the group consisting of a primer and an amplifying reagent in the at least one well.
<B12> The device according to any one of <1> to <11>,
wherein the device is used for evaluation of accuracy of a PCR device.
<B13> A device including:

at least one well; and
an amplifiable reagent contained in the at least one well,
wherein when the amplifiable reagent is amplified under a stipulated amplification condition, any of the at least one well with a Ct value of 30 or greater have a standard deviation $\sigma$ of 3 or less.

<B14> A device including:

at least one well;
an amplifiable reagent contained in the at least one well; and
information on a number of the amplifiable reagent.

<B15> The device according to <B14>, including
information on uncertainty as the information on the number.
<B16> A testing method including
using the device according to any one of <B1> to <B15>.
<C1> A detection determining method used in detection of a testing target in a sample by amplification of the testing target and an amplifiable reagent, a specific copy number of the amplifiable reagent being 200 or less, the detection determining method including
determining that the testing target is present and a detection result is positive when the amplifiable reagent is amplified and the testing target is amplified, and determining that the testing target is absent or at least less than the specific copy number of the amplifiable reagent and a detection result is negative when the amplifiable reagent is amplified and the testing target is not amplified.

<C2> The detection determining method according to <C1>, wherein the amplifiable reagent is a nucleic acid.

<C3> The detection determining method according to <C2>, wherein the nucleic acid serving as the amplifiable reagent is introduced in a nucleic acid in a nucleus of a cell.

<C4> The detection determining method according to <3>, wherein the cell is a yeast.

<C5> The detection determining method according to any one of <C1> to <C4>, wherein a limit of detection of the testing target is equivalent to a limit of detection of the amplifiable reagent.

<C6> The detection determining method according to any one of <C1> to <C5>, wherein the amplifiable reagent is filled in a sample filling well to be filled with a sample, and the testing target and the amplifiable reagent are amplified in a same sample filling well.

<C7> The detection determining method according to any one of <C2> to <C6>, wherein a base sequence of the testing target and a base sequence of the amplifiable reagent are different from each other.

<C8> The detection determining method according to <7>, wherein positive control having a same base sequence as the base sequence of the testing target is filled in a predetermined amount in a well different from the sample filling well, and subjected to amplification treatment.

<C9> The detection determining method according to <C8>, wherein the detection determining method is a genetic test in which the testing target is a virus, a bacterium, or animal species identification for meat.

<C10> The detection determining method according to any one of <C1> to <C9>, wherein the specific copy number of the amplifiable reagent is a known number.

<C11> A detection determining device used in detection of a testing target in a sample by amplification of the testing target and an amplifiable reagent, a specific copy number of the amplifiable reagent being 200 or less, the detection determining device including:

a determining unit configured to determine that the testing target is present and a detection result is positive when the amplifiable reagent is amplified and the testing target is amplified, and determine that the testing target is absent or at least less than the specific copy number of the amplifiable reagent and a detection result is negative when the amplifiable reagent is amplified and the testing target is not amplified.

<C12> A detection determining program used in detection of a testing target in a sample by amplification of the testing target and an amplifiable reagent, a specific copy number of the amplifiable reagent being 200 or less, the detection determining program causing a computer to execute a process including

determining that the testing target is present and a detection result is positive when the amplifiable reagent is amplified and the testing target is amplified, and determining that the testing target is absent or at least less than the specific copy number of the amplifiable reagent and a detection result is negative when the amplifiable reagent is amplified and the testing target is not amplified.

<C13> A device used in the detection determining method according to any one of <C1> to <C10>, the device including

at least one sample filling well to be filled with a sample,

wherein the at least one sample filling well further includes an amplifiable reagent in a specific copy number, and

wherein the specific copy number of the amplifiable reagent is 200 or less.

<C14> The device according to <C13>, wherein a relationship between a coefficient of variation CV for the specific copy number and an average specific copy number x of the amplifiable reagent satisfies the following formula: $CV < 1/\sqrt{x}$.

<C15> The device according to <C13> or <C14>, wherein the specific copy number of the amplifiable reagent is 100 or less.

<C16> The device according to any one of <13> to <15>, wherein the at least one sample filling well includes information on the specific copy number of the amplifiable reagent and uncertainty of the specific copy number of the amplifiable reagent.

<C17> The device according to any one of <C13> to <C16>, including a sealing member configured to seal an opening of the at least one sample filling well.

<C18> The device according to any one of <C13> to <C17>, wherein the amplifiable reagent is a nucleic acid.

<C19> The device according to <C18>, wherein the nucleic acid is incorporated into a nucleic acid in a nucleus of a cell.

<C20> The device according to <C19>,
wherein the cell is a yeast.
<C21> The device according to any one of <C18> to <C20>,
wherein a base sequence of the testing target and a base sequence of the amplifiable reagent are different from each other.
<C22> The device according to any one of <C13> to <C21>,
wherein the at least one sample filling well includes a pair of primers for amplifying the testing target and a pair of primers for amplifying the amplifiable reagent.
<C23> A device used in the detection determining method according to any one of <C1> to <C10>.
<D1> A device including:

at least one well in which an amplifiable reagent is contained in a specific copy number of less than 100; and
at least one well in which the amplifiable reagent is contained in a specific copy number of 100 or greater,
wherein for at least one well in which the specific copy number of the amplifiable reagent is less than 100, a formula: $CV < 1/\sqrt{x}$ is satisfied, where CV represents a coefficient of variation for the specific copy number and x represents average specific copy number of the amplifiable reagent.

<D2> The device according to <D1>,
wherein a coefficient of variation CV of the at least one well in which the specific copy number of the amplifiable reagent is 100 or greater is 20% or lower.
<D3> The device according to <D1> or <D2>,
wherein a coefficient of variation CV of the at least one well in which the specific copy number of the amplifiable reagent is less than 100 is 10% or lower.
<D4> The device according to any one of <D1> to <D3>,
wherein for the specific copy number of the amplifiable reagent of 100 or greater, a formula: $CV > 1/\sqrt{x}$ is satisfied, where CV represents a coefficient of variation for the specific copy number and x represents average specific copy number of the amplifiable reagent.
<D5> The device according to any one of <D1> to <D4>,
wherein each of the specific copy numbers of the amplifiable reagent contained in the at least one well includes two or more levels.
<D6> The device according to any one of <D1> to <D5>, further including
for a specific copy number of the amplifiable reagent in the at least one well, information on uncertainty of the specific copy number.
<D7> The device according to <D6>, further including
an identifier unit configured to enable identifying at least any one of information on the coefficient of variation CV of the at least one well in which the specific copy number of the amplifiable reagent is less than 100, information on the coefficient of variation CV of the at least one well in which the specific copy number of the amplifiable reagent is 100 or greater, and the information on the uncertainty of the specific copy number.
<D8> The device according to any one of <D1> to <D7>, further including
a sealing member configured to seal an opening of the at least one well.
<D9> The device according to <D8>,
wherein the amplifiable reagent is a nucleic acid.
<D10> The device according to <D9>,
wherein the nucleic acid is introduced in a nucleic acid in a nucleus of a cell.
<D11> The device according to <10>,
wherein the cell is a yeast cell.
<D12> The device according to any one of <D1> to <D11>,
wherein the at least one well contains at least any one of a primer and an amplifying reagent.
<D13> The device according to any one of <D6> to <D12>,
wherein the at least one well includes a plurality of wells, and
wherein the device further includes a specific copy number of the nucleic acid in each of the plurality of wells and the uncertainty of the specific copy number of the nucleic acid, as information for each of the plurality of wells.
<D14> The device according to any one of <D8> to <D13>, further including
a base material provided with the at least one well,
wherein the identifier unit is placed between the sealing member and the base material.
<D15> A device including:

at least one well; and

information on a specific copy number of a nucleic acid in the at least one well and uncertainty of the specific copy number of the nucleic acid,
wherein the device is used for evaluation of a PCR device capable of amplifying a nucleic acid.

<D16> The device according to <D15>,
wherein a result of amplification of a nucleic acid by the device and the information on the specific copy number of the nucleic acid and the uncertainty of the specific copy number of the nucleic acid are used for management of the PCR device.
<E1> A device including
a well provided in a number of at least one,
wherein a nucleic acid of norovirus is contained in a specific copy number in at least one well of the well, and
wherein the specific copy number of the nucleic acid of the norovirus is 1,000 or less.
<E2> The device according to <E1>,
wherein the nucleic acid of the norovirus is contained in a carrier.
<E3> The device according to <E2>,
wherein the carrier is at least any one selected from the group consisting of cells, phages, and viruses.
<E4> The device according to <E3>,
wherein the cells are selected from the group consisting of yeast, animals, and plants.
<E5> The device according to any one of <E1> to <E4>,
wherein the norovirus contains at least any one selected from the group consisting of a nucleic acid that expresses a GI type and a nucleic acid that expresses a GII type.
<E6> The device according to any one of <E1> to <E5>,
wherein the nucleic acid of the norovirus includes base sequences of two or more selected from the group consisting of SEQ ID NOS. 8, 9, 10, and 11, and includes a base sequence having a total length of 50 bases or more.
<E7> The device according to any one of <E1> to <E6>,
wherein the nucleic acid of the norovirus includes at least any base sequence selected from the group consisting of a base sequence having a homology of 80% or higher with respect to a base sequence of SEQ ID NO. 4. and a base sequence having a homology of 80% or higher with respect to a base sequence of SEQ ID NO. 5.
<E8> The device according to any one of <E1> to <E6>,
wherein the nucleic acid of the norovirus includes at least any base sequence selected from the group consisting of (i) a base sequence X including: a base sequence of SEQ ID NO. 4; and a base sequence having an arbitrary length less than or equal to 1,000 bases at a 5' terminal side or a 3' terminal side, and a base sequence having a homology of 80% or higher with respect to the base sequence X, and (ii) a base sequence Y including: a base sequence of SEQ ID NO. 5; and abase sequence having an arbitrary length less than or equal to 1,000 bases at a 5' terminal side or a 3' terminal side, and a base sequence having a homology of 80% or higher with respect to the base sequence Y.
<E9> The device according to any one of <E1> to <E8>,
wherein the at least one well in which the nucleic acid of the norovirus is contained in the specific copy number contains at least any one selected from the group consisting of a primer and an amplifying reagent.
<E10> The device according to any one of <1> to <9>, including
a sealing member configured to seal an opening of the at least one well in which the nucleic acid of the norovirus is contained in the specific copy number.
<E11> The device according to any one of <E1> to <E10>,
wherein the at least one well in which the nucleic acid of the norovirus is contained in the specific copy number includes two or more wells, and
wherein the specific copy number of the nucleic acid of the norovirus in one of the at least one well is different from the specific copy number of the nucleic acid of the norovirus in another of the at least one well.
<E12> The device according to any one of <E1> to <E11>,
wherein the well which is different from the at least one well containing the nucleic acid of the norovirus in the specific copy number and in which a nucleic acid of a testing target norovirus is located contains an amplifiable reagent different from the nucleic acid of the testing target norovirus.
<E13> The device according to any one of claims 1 to 12,
wherein the specific copy number of the nucleic acid of the norovirus is a known number.
<E14> A norovirus testing method including
using the device according to any one of <E1> to <E13> and subjecting a nucleic acid of a testing target norovirus and a nucleic acid of a norovirus in a specific copy number to amplification reaction to detect the nucleic acid of the testing target norovirus.
<E15> The norovirus testing method according to <E14>, including:

determining that the nucleic acid of the testing target norovirus is present and a detection result is positive when the nucleic acid of the norovirus in the specific copy number and the nucleic acid of the testing target norovirus are both amplified; and

determining that the nucleic acid of the testing target norovirus is absent or less than or equal to a limit of detection and a detection result is negative when the nucleic acid of the norovirus in the specific copy number is amplified and the nucleic acid of the testing target norovirus is not amplified.

<E16> The norovirus testing method according to <E14> or <E15>, including

filling the well in which the nucleic acid of the testing target norovirus is located, except the at least one well in which the nucleic acid of the norovirus is contained in the specific copy number, with an amplifiable reagent different from the nucleic acid of the testing target norovirus, and subjecting the nucleic acid of the norovirus in the specific copy number, the nucleic acid of the testing target norovirus, and the amplifiable reagent to amplification reaction;

determining that the nucleic acid of the testing target norovirus is present and a detection result is positive when all of the nucleic acid of the norovirus in the specific copy number, the amplifiable reagent, and the nucleic acid of the testing target norovirus are amplified as a result of the subjecting to amplification reaction; and

determining that the nucleic acid of the testing target norovirus is absent or less than or equal to a limit of detection and a detection result is negative when the nucleic acid of the norovirus in the specific copy number and the amplifiable reagent are amplified but the nucleic acid of the testing target norovirus is not amplified as a result of the subjecting to amplification reaction.

<E17> The norovirus testing method according to any one of <E14> to <E16>,

wherein the at least one well in which the nucleic acid of the norovuris is contained in the specific copy number in the device includes one well in which the nucleic acid of the norovirus is contained in a predetermined specific copy number and another one well in which the nucleic acid of the norovirus is contained in a specific copy number different from the specific copy number in the one well,

wherein the norovirus testing method includes:

subjecting the nucleic acids of the norovirus contained in the one well and the another one well varied in the specific copy number, and the nucleic acid of the testing target norovirus to amplification reaction; and

comparing results of amplification of the nucleic acids of the norovirus contained in respective specific copy numbers with a result of amplification of the nucleic acid of the testing target norovirus to determine an amount of the nucleic acid of the testing target norovirus.

<E18> A norovirus testing program used in detection of a testing target norovirus in a sample by subjecting a nucleic acid of a norovirus in a specific copy number and a nucleic acid of the testing target norovirus to amplification reaction, the norovirus testing program causing a computer to execute a process including:

determining that the nucleic acid of the testing target norovirus is present and a detection result is positive when the nucleic acid of the norovirus in the specific copy number and the nucleic acid of the testing target norovirus are both amplified; and

determining that the nucleic acid of the testing target norovirus is absent or less than or equal to a limit of detection and a detection result is negative when the nucleic acid of the norovirus in the specific copy number is amplified but the nucleic acid of the testing target norovirus is not amplified.

<E19> A norovirus testing device used in detection of a testing target norovirus in a sample by subjecting a nucleic acid of a norovirus in a specific copy number and a nucleic acid of the testing target norovirus to amplification reaction, the norovirus testing device including

a determining unit configured to determine that the nucleic acid of the testing target norovirus is present and a detection result is positive when the nucleic acid of the norovirus in the specific copy number and the nucleic acid of the testing target norovirus are both amplified, and determine that the nucleic acid of the testing target norovirus is absent or less than or equal to a limit of detection and a detection result is negative when the nucleic acid of the norovirus in the specific copy number is amplified but the nucleic acid of the testing target norovirus is not amplified.

<F1> A device including:

a plurality of wells; and

two or more groups into which the wells are divided to be varied in composition in the wells.

<F2> A device including:

a plurality of wells;

a reagent composition located in each of the wells and containing an amplifiable reagent in a specific copy number; and

two or more groups into which the wells are divided to have the amplifiable reagent located in the same specific copy number but to be varied in composition of the reagent composition except for the specific copy number.

<F3> The device according to <F2>, including
groups varied in the specific copy number.

<F4> A device including:

a plurality of wells;

an amplifiable reagent located in a specific copy number in each of the wells; and

two or more groups into which the wells are divided to be varied in the specific copy number of the amplifiable reagent.

<F5> The device according to any one of <F2> to <F4>,
wherein at least one group of the groups into which the wells are divided is a group in which the specific copy number of the amplifiable reagent is close to a limit of detection.

<F6> The device according to any one of <F2> to <F4>,
wherein at least one group of the groups into which the wells are divided is a group in which the specific copy number of the amplifiable reagent is a copy number greater than a limit of quantification.

<F7> The device according to any one of <F2> to <F6>,
wherein at least one group of the groups into which the wells are divided is a negative control group in which the specific copy number of the amplifiable reagent is 0.

<F8> The device according to any one of <F2> to <F7>,
wherein at least one group of the groups into which the wells are divided is a positive control group in which the specific copy number of the amplifiable reagent is 100 or greater.

<F9> The device according to any one of <F2> to <F8>,
wherein at least one group of the groups into which the wells are divided is a group having the least copy number except for the negative control group, the at least one group being positioned at least at wells that are approximately at a periphery of the device.

<F10> The device according to any one of <F2> to <F9>,
wherein each of the wells contains at least any one selected from the group consisting of a primer and an amplifying reagent.

<F11> The device according to any one of <F2> to <F10>, including
an identifier unit configured to enable identifying information on the specific copy number of the amplifiable reagent in the wells.

<F12> The device according to any one of <F2> to <F11>,
wherein the amplifiable reagent is a nucleic acid.

<F13> The device according to <F12>,
wherein the nucleic acid is introduced in a nucleic acid in a nucleus of a cell.

<F14> The device according to <F13>,
wherein the cell is a yeast cell.

<F15> The device according to any one of <F2> to <F14>,
wherein the specific copy number is a counted copy number of the amplifiable reagent.

<F16> The device according to any one of <F13> to <F15>,
wherein the cell is discharged by an inkjet method.

<F17> A preparator's skill evaluating method for evaluating skill of a preparator who prepares a reagent composition, the preparator's skill evaluating method including:
obtaining information on a Ct value in the device according to any one of <F1> to <F16>; and
evaluating the skill of the preparator based on the obtained information on the Ct value.

<F18> A preparator's skill evaluating program for evaluating skill of a preparator who prepares a reagent composition, the preparator's skill evaluating program causing a computer to execute a process including:

obtaining information on a Ct value in the device according to any one of <F1> to <F16>; and
evaluating the skill of the preparator based on the obtained information on the Ct value.

<F19> A preparator's skill evaluating device configured to evaluate skill of a preparator who prepares a reagent composition, the preparator's skill evaluating device including:

a Ct value information obtaining unit configured to obtain information on a Ct value in the device according to any one of <F1> to <F16>; and
a skill evaluating unit configured to evaluate the skill of the preparator based on the obtained information on the Ct value.

<F20> A testing device performance evaluating method for evaluating performance of a testing device configured to test a testing target, the testing device performance evaluating method including:

obtaining information on a Ct value in the device according to any one of <F1> to <F16>; and
evaluating the performance of the testing device based on the information on the Ct value.

<F21> A testing device performance evaluating program for evaluating performance of a testing device configured to test a testing target, the testing device performance evaluating program causing a computer to execute a process including:

obtaining information on a Ct value in the device according to any one of <F1> to <F16>; and
evaluating the performance of the testing device based on the information on the Ct value.

<F22> A testing device performance evaluating device configured to evaluate performance of a testing device configured to test a testing target, the testing device performance evaluating device including:

a Ct value information obtaining unit configured to obtain information on a Ct value in the device according to any one of <F1> to <F16>; and
a performance evaluating unit configured to evaluate the performance of the testing device based on the information on the Ct value.

The detection determining device according to any one of <A1> to <A5>, the device according to any one of <B1> to <B15> and the testing method according to <B16>, the detection determining method according to any one of <C1> to <C10>, the detection determining device according to <C11>, the detection determining program according to <C12>, and the device according to any one of <C13> to <C23>, the device according to any one of <D1> to <D14> and the device according to <D15> or <D16>, the device according to any one of <E1> to <E13>, the norovirus testing method according to any one of <E14> to <E17>, the norovirus testing program according to <E18>, and the norovirus testing device according to <E19>, and the device according to any one of <F1> to <F16>, the preparator's skill evaluating program according to <F17>, the preparator's skill evaluating method according to <F18>, the preparator's skill evaluating device according to <F19>, the testing device performance evaluating method according to <F20>, the testing device performance evaluating program according to <F21>, and the testing device performance evaluating device according to <F22> can solve the various problems in the related art and can achieve the object of the present invention.

Description of the Reference Numeral

1:    device
2:    base material
3:    well
4:    amplifiable reagent
5:    sealing member

[Sequence Listing]

N-RC015-18P

**Claims**

1. A detection determining device used in a detection determining method including, in detection of a testing target in a sample by amplification of the testing target and an amplifiable reagent, determining that the testing target is present and a detection result is positive when the amplifiable reagent is amplified and the testing target is amplified, and determining that the testing target is absent or at least less than a specific copy number of the amplifiable reagent and a detection result is negative when the amplifiable reagent is amplified and the testing target is not amplified, the detection determining device comprising
   at least one well,
   wherein the amplifiable reagent in the at least one well is contained in a specific copy number.

2. The detection determining device according to claim 1, comprising
   information on the specific copy number,
   wherein the information on the specific copy number is at least any one selected from the group consisting of information on uncertainty of the specific copy number and a coefficient of variation CV of the specific copy number.

3. The detection determining device according to claim 1 or 2,
   wherein the amplifiable reagent is a nucleic acid of a partial base sequence of norovirus genome.

4. The detection determining device according to any one of claims 1 to 3, comprising
   two or more groups into which the at least one well is divided to be varied in composition in the at least one well.

5. The detection determining device according to claim 4, comprising
   two or more groups into which the at least one well in which a reagent composition containing the amplifiable reagent in the specific copy number is located is divided, to have the amplifiable reagent located in the same specific copy number but to be varied in composition of the reagent composition except for the specific copy number, or
   two or more groups into which the at least one well in which the amplifiable reagent is located in the specific copy number is divided, to be varied in the specific copy number of the amplifiable reagent.

# FIG. 1

# FIG. 2

# FIG. 3

# FIG. 4

# FIG. 5

Wells filled with amplifiable reagent → ← Wells filled with sample →

| 1 | 1 | | | | | | |
|---|---|---|---|---|---|---|---|
| 3 | 3 | | | | | | |
| 5 | 5 | | | | | | |
| 7 | 7 | | | | | | |
| 9 | 9 | | | | | | |

# FIG. 6

Portions filled with amplifiable reagent

FIG. 7

FIG. 8

# FIG. 9

# FIG. 10

# FIG. 11

Wells filled with amplifiable reagent

Filled with sample

May be filled with positive control

# FIG. 12

100

Detection determining device

101 CPU

102 Main memory device

103 Auxiliary memory device

104 Output device

105 Input device

106

# FIG. 13

100

**Detection determining device**

130

**Control unit**

131 Detection result obtaining unit

132 Detection result analyzing unit

133 Determining unit

110 Input unit

120 Output unit

140

**Memory unit**

141 Detection result DB

142 Determination result DB

# FIG. 14

Start

S101 Obtain detection result — 141 Detection result DB

S102 Analyze detection result — 141 Detection result DB

S103 Has amplifiable reagent been amplified?
Yes / No

S104 Has testing target been amplified?
No / Yes

S107 Has testing target been amplified?
Yes / No

S106 Determine detection result as negative

S105 Determine detection result as positive

S108 Determine detection result as status unknown

S109 Determine detection result as failure of experiment

S110 Store determination result in determination result DB — 142 Determination result DB

End

# FIG. 15

# FIG. 16

# FIG. 17

Wells filled with
amplifiable reagent    Wells filled with sample
←————————→←————————————————————————→

| | | | | | | | |
|---|---|---|---|---|---|---|---|
| 1 | $10^2$ | | | | | | |
| 2 | $10^3$ | | | | | | |
| 3 | $10^4$ | | | | | | |
| 10 | $10^5$ | | | | | | |
| 50 | $10^6$ | | | | | | |

# FIG. 18

Wells filled with
amplifiable reagent    Wells filled with sample
←————————→←————————————————————————→

| | | | | | | | |
|---|---|---|---|---|---|---|---|
| 1 | $10^2$ | | | | | | |
| 3 | $10^3$ | | | | | | |
| 5 | $10^4$ | | | | | | |
| 10 | $10^5$ | | | | | | |
| 50 | $10^6$ | | | | | | |

# FIG. 19

# FIG. 20

# FIG. 21

|   | 1 | 2 | 3 | 4 | 5 | 6 | 7 | 8 | 9 | 10 | 11 | 12 |
|---|---|---|---|---|---|---|---|---|---|----|----|----|
| A | 37.2 | 37.4 | 36.1 | 36.9 | 37.5 | 39.1 | 36.9 | 41.8 | 36.9 | 37.2 | 36.4 | 32.7 |
| B | 37.2 | 36.1 | 37.0 | 37.0 | 36.9 | 37.2 | 37.3 | 37.0 | 36.3 | 36.4 | 37.0 | 37.3 |
| C | 37.0 | 38.5 | 37.6 | 37.7 | 39.8 | 37.4 | 37.1 | 39.9 | 36.2 | 36.2 | 37.3 | 36.1 |
| D | 37.6 | 36.6 | 37.0 | 38.2 | UD | 36.4 | 36.3 | 30.1 | 37.2 | 36.9 | 37.6 | 37.5 |
| E | 37.0 | 36.4 | 36.3 | 38.2 | UD | 37.0 | 35.8 | 30.3 | 36.5 | 37.0 | 36.1 | 36.5 |
| F | 36.6 | 37.0 | 36.1 | 36.8 | 37.1 | 37.1 | 37.1 | 37.0 | 36.5 | 36.9 | 36.3 | 37.4 |
| G | 37.0 | 36.4 | 37.0 | 37.3 | 37.3 | 37.0 | 38.2 | 37.0 | 36.7 | 37.8 | 36.3 | 36.6 |
| H | 37.0 | 36.9 | 38.2 | 36.1 | 37.0 | 36.4 | 37.9 | 36.4 | 36.8 | 37.0 | 37.4 | 38.4 |

# FIG. 22

|   | 1 | 2 | 3 | 4 | 5 | 6 | 7 | 8 | 9 | 10 | 11 | 12 |
|---|---|---|---|---|---|---|---|---|---|----|----|----|
| A | 3 | 3 | 3 | 3 | 3 | 3 | 3 | 3 | 3 | 3 | 3 | 3 |
| B | 3 | 3 | 3 | 3 | 3 | 3 | 3 | 3 | 3 | 3 | 3 | 3 |
| C | 3 | 3 | 3 | 3 | 3 | 3 | 3 | 3 | 3 | 3 | 3 | 3 |
| D | 3 | 3 | 3 | 3 | 0 | 3 | 3 | 0 | 3 | 3 | 3 | 3 |
| E | 3 | 3 | 3 | 3 | 0 | 3 | 3 | 0 | 3 | 3 | 3 | 3 |
| F | 3 | 3 | 3 | 3 | 3 | 3 | 3 | 3 | 3 | 3 | 3 | 3 |
| G | 3 | 3 | 3 | 3 | 3 | 3 | 3 | 3 | 3 | 3 | 3 | 3 |
| H | 3 | 3 | 3 | 3 | 3 | 3 | 3 | 3 | 3 | 3 | 3 | 3 |

# FIG. 23

|   | 1 | 2 | 3 | 4 | 5 | 6 | 7 | 8 | 9 | 10 | 11 | 12 |
|---|---|---|---|---|---|---|---|---|---|----|----|----|
| A | O | O | O | O | O | O | O | × | O | O | O | × |
| B | O | O | O | O | O | O | O | O | O | O | O | O |
| C | O | O | O | O | O | O | O | O | O | O | O | O |
| D | O | O | O | O | UD | O | O | UD | O | O | O | O |
| E | O | O | O | O | UD | O | O | UD | O | O | O | O |
| F | O | O | O | O | O | O | O | O | O | O | O | O |
| G | O | O | O | O | O | O | O | O | O | O | O | O |
| H | O | O | × | O | O | O | O | O | O | O | O | × |

# FIG. 24

|   | 1 | 2 | 3 | 4 | 5 | 6 | 7 | 8 | 9 | 10 | 11 | 12 |
|---|---|---|---|---|---|---|---|---|---|----|----|----|
| A | 2 | 2 | 2 | 2 | 2 | 2 | 2 | 2 | 2 | 2 | 2 | 2 |
| B | 2 | 3 | 5 | 10 | 20 | 100 | 3 | 5 | 10 | 20 | 100 | 2 |
| C | 2 | 2 | 2 | 2 | 2 | 2 | 2 | 2 | 2 | 2 | 2 | 2 |
| D | 2 | 2 | 2 | 2 | PC | 0 | 0 | 2 | 2 | 2 | 2 | 2 |
| E | 2 | 2 | 2 | 2 | 2 | 0 | 0 | PC | 2 | 2 | 2 | 2 |
| F | 2 | 2 | 2 | 2 | 2 | 2 | 2 | 2 | 2 | 2 | 2 | 2 |
| G | 2 | 3 | 5 | 10 | 20 | 100 | 3 | 5 | 10 | 20 | 100 | 2 |
| H | 2 | 2 | 2 | 2 | 2 | 2 | 2 | 2 | 2 | 2 | 2 | 2 |

# FIG. 25

# FIG. 26

# FIG. 27

|   | 1 | 2 | 3 | 4 | 5 | 6 | 7 | 8 | 9 | 10 | 11 | 12 |
|---|---|---|---|---|---|---|---|---|---|----|----|----|
| A | 2 | 2 | 2 | 2 | 2 | 2 | 2 | 2 | 2 | 2 | 2 | 2 |
| B | 2 | 10 | 10 | 10 | 10 | 10 | 10 | 10 | 10 | 10 | 10 | 2 |
| C | 2 | 2 | 2 | 2 | 2 | 2 | 0 | 2 | 2 | 2 | 2 | 2 |
| D | 2 | 100 | 100 | 100 | 100 | 100 | 100 | 100 | 100 | 100 | 100 | 2 |
| E | 2 | 10 | 10 | 10 | 10 | 10 | 10 | 10 | 10 | 10 | 10 | 2 |
| F | 2 | 2 | 2 | 2 | 2 | 2 | 0 | 2 | 2 | 2 | 2 | 2 |
| G | 2 | 100 | 100 | 100 | 100 | 100 | 100 | 100 | 100 | 100 | 100 | 2 |
| H | 2 | 2 | 2 | 2 | 2 | 2 | 2 | 2 | 2 | 2 | 2 | 2 |

# FIG. 28

|   | 1 | 2 | 3 | 4 | 5 | 6 | 7 | 8 | 9 | 10 | 11 | 12 |
|---|---|---|---|---|---|---|---|---|---|----|----|----|
| A | 2 | 2 | 2 | 2 | 2 | 2 | 2 | 2 | 2 | 2 | 2 | 2 |
| B | 2 | 5 | 10 | 5 | 100 | 2 | 2 | 100 | 5 | 10 | 5 | 2 |
| C | 2 | 3 | 10 | 3 | 0 | 2 | 2 | PC | 3 | 10 | 3 | 2 |
| D | 2 | 2 | 2 | 2 | 2 | 2 | 2 | 2 | 2 | 2 | 2 | 2 |
| E | 2 | 2 | 2 | 2 | 2 | 2 | 2 | 2 | 2 | 2 | 2 | 2 |
| F | 2 | 3 | 10 | 3 | PC | 2 | 2 | 0 | 3 | 10 | 3 | 2 |
| G | 2 | 5 | 10 | 5 | 100 | 2 | 2 | 100 | 5 | 10 | 5 | 2 |
| H | 2 | 2 | 2 | 2 | 2 | 2 | 2 | 2 | 2 | 2 | 2 | 2 |

# FIG. 29

| | 1 | 2 | 3 | 4 | 5 | 6 | 7 | 8 | 9 | 10 | 11 | 12 |
|---|---|---|---|---|---|---|---|---|---|---|---|---|
| A | 2 | 2 | 2 | 2 | 2 | 2 | 2 | 2 | 2 | 2 | 2 | 2 |
| B | 2 | 100 | 2 | 2 | 2 | 2 | 2 | 2 | 2 | 2 | 100 | 2 |
| C | 2 | 2 | 10 | 2 | 0 | 2 | 2 | 0 | 2 | 10 | 2 | 2 |
| D | 2 | 2 | 2 | 5 | 2 | 2 | 2 | 2 | 5 | 2 | 2 | 2 |
| E | 2 | 2 | 2 | 5 | 2 | 2 | 2 | 2 | 5 | 2 | 2 | 2 |
| F | 2 | 2 | 10 | 2 | PC | 2 | 2 | PC | 2 | 10 | 2 | 2 |
| G | 2 | 100 | 2 | 2 | 2 | 2 | 2 | 2 | 2 | 2 | 100 | 2 |
| H | 2 | 2 | 2 | 2 | 2 | 2 | 2 | 2 | 2 | 2 | 2 | 2 |

# FIG. 30

| | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 |
|---|---|---|---|---|---|---|---|---|---|---|---|---|---|---|---|---|---|---|---|---|---|---|---|---|
| A | 2 | 2 | 2 | 2 | 2 | 2 | 2 | 2 | 2 | 2 | 2 | 2 | 2 | 2 | 2 | 2 | 2 | 2 | 2 | 2 | 2 | 2 | 2 | 2 |
| B | 2 | 2 | 2 | 2 | 2 | 2 | 2 | 2 | 2 | 2 | 2 | 2 | 2 | 2 | 2 | 2 | 2 | 2 | 2 | 2 | 2 | 2 | 2 | 2 |
| C | 2 | 2 | 2 | 2 | 2 | 2 | 2 | 2 | 2 | 2 | 2 | 2 | 2 | 2 | 2 | 2 | 2 | 2 | 2 | 2 | 2 | 2 | 2 | 2 |
| D | 2 | 2 | 2 | 2 | 2 | 2 | 2 | 2 | 2 | 2 | 2 | 2 | 2 | 2 | 2 | 2 | 2 | 2 | 2 | 2 | 2 | 2 | 2 | 2 |
| E | 2 | 2 | 2 | 2 | 2 | 2 | 2 | 2 | 2 | 2 | 2 | 2 | 2 | 2 | 2 | 2 | 2 | 2 | 2 | 2 | 2 | 2 | 2 | 2 |
| F | 2 | 2 | 2 | 2 | 2 | 2 | 2 | 2 | 2 | 2 | 2 | 2 | 2 | 2 | 2 | 2 | 2 | 2 | 2 | 2 | 2 | 2 | 2 | 2 |
| G | 2 | 2 | 2 | 2 | 2 | 2 | 2 | 2 | 2 | 10 | 2 | 100 | 100 | 2 | 10 | 2 | 2 | 2 | 2 | 2 | 2 | 2 | 2 | 2 |
| H | 2 | 2 | 2 | 2 | 2 | 2 | 2 | 2 | 2 | 10 | 0 | 100 | 100 | PC | 10 | 2 | 2 | 2 | 2 | 2 | 2 | 2 | 2 | 2 |
| I | 2 | 2 | 2 | 2 | 2 | 2 | 2 | 2 | 2 | 10 | 0 | 100 | 100 | PC | 10 | 2 | 2 | 2 | 2 | 2 | 2 | 2 | 2 | 2 |
| J | 2 | 2 | 2 | 2 | 2 | 2 | 2 | 2 | 2 | 10 | 2 | 100 | 100 | 2 | 10 | 2 | 2 | 2 | 2 | 2 | 2 | 2 | 2 | 2 |
| K | 2 | 2 | 2 | 2 | 2 | 2 | 2 | 2 | 2 | 2 | 2 | 2 | 2 | 2 | 2 | 2 | 2 | 2 | 2 | 2 | 2 | 2 | 2 | 2 |
| L | 2 | 2 | 2 | 2 | 2 | 2 | 2 | 2 | 2 | 2 | 2 | 2 | 2 | 2 | 2 | 2 | 2 | 2 | 2 | 2 | 2 | 2 | 2 | 2 |
| M | 2 | 2 | 2 | 2 | 2 | 2 | 2 | 2 | 2 | 2 | 2 | 2 | 2 | 2 | 2 | 2 | 2 | 2 | 2 | 2 | 2 | 2 | 2 | 2 |
| N | 2 | 2 | 2 | 2 | 2 | 2 | 2 | 2 | 2 | 2 | 2 | 2 | 2 | 2 | 2 | 2 | 2 | 2 | 2 | 2 | 2 | 2 | 2 | 2 |
| O | 2 | 2 | 2 | 2 | 2 | 2 | 2 | 2 | 2 | 2 | 2 | 2 | 2 | 2 | 2 | 2 | 2 | 2 | 2 | 2 | 2 | 2 | 2 | 2 |
| P | 2 | 2 | 2 | 2 | 2 | 2 | 2 | 2 | 2 | 2 | 2 | 2 | 2 | 2 | 2 | 2 | 2 | 2 | 2 | 2 | 2 | 2 | 2 | 2 |

# FIG. 31

# FIG. 32

# FIG. 33

Start

S110

Ct value information is obtained

S111

Preparator's skill is evaluated

S112

Result of evaluation of preparator's skill is stored

End

# FIG. 34

100

**Testing device performance evaluating device**

101 CPU

102 Main memory device

103 Auxiliary memory device

104 Output device

105 Input device

106 Communication interface

107

# FIG. 35

Testing device performance evaluating device — 100

Control unit — 130

Input unit — 110

Output unit — 120

Ct value information obtaining unit — 131

Performance evaluating unit — 132

Memory unit — 140

Ct value information DB — 141

Performance evaluation result DB — 142

# FIG. 36

Start

Ct value information is obtained — S110

Testing device performance is evaluated — S111

Result of evaluation of testing device performance is stored — S112

End

# FIG. 37

Fluorescence intensity

# FIG. 38A

# FIG. 38B

13b
11b
300b
111b

# FIG. 38C

11c
300c
13c
111c

# FIG. 39A

# FIG. 39B

# FIG. 40A

11c

12c

13c

# FIG. 40B

11c

12c

13c

# FIG. 40C

11c

12c

13c

310'

# FIG. 41

400

401

Liquid droplet
forming device

710    310    350    700

800

Control device    900

# FIG. 42

401

20
Driving unit

10
13  12  11

300

350

111

30
Light source

L    310    350    Lf

60
Light receiving
element

70
Control unit

# FIG. 43

```
                                              70
┌──────────────────────────────────────────────────┐
│ Control unit                                       │
│              71              72              73    │
│        ┌──────────┐   ┌──────────┐   ┌──────────┐  │
│        │   CPU    │   │   ROM    │   │   RAM    │  │
│        └──────────┘   └──────────┘   └──────────┘  │
│                                               74   │
│                                          ┌────────┐│
│  ──────────────┴──────────────┴──────────│  I/F   ││
│                                          └────────┘│
│                      75                            │
└──────────────────────────────────────────────────┘
```

# FIG. 44

```
                                70
┌──────────────────────────────────┐
│ Control unit                      │
│                    701            │
│  ┌────────────────────────────┐   │
│  │  Discharging control unit  │   │
│  └────────────────────────────┘   │
│                    702            │
│  ┌────────────────────────────┐   │
│  │  Light source control unit │   │
│  └────────────────────────────┘   │
│                    703            │
│  ┌────────────────────────────┐   │
│  │  Cell number counting unit │   │
│  └────────────────────────────┘   │
│                                   │
└──────────────────────────────────┘
```

# FIG. 45

```
        ┌─────────────┐
        │    Start     │
        └──────┬───────┘
               │        ⟋S11
    ┌──────────▼──────────────┐
    │ Liquid droplet is discharged │
    └──────────┬──────────────┘
               │        ⟋S12
    ┌──────────▼──────────────┐
    │  Light source is turned on  │
    └──────────┬──────────────┘
               │        ⟋S13
    ┌──────────▼──────────────┐
    │  Cell number is counted     │
    └──────────┬──────────────┘
               │
        ┌──────▼───────┐
        │     End      │
        └──────────────┘
```

# FIG. 46

# FIG. 47

# FIG. 48A

# FIG. 48B

$350_1$        $350_2$

# FIG. 49

# FIG. 50

# FIG. 51

# FIG. 52

# FIG. 53

# FIG. 54

# FIG. 55

# FIG. 56A

# FIG. 56B

# FIG. 56C

# FIG. 57

|   | 1 | 2 | 3 | 4 | 5 | 6 | 7 | 8 | 9 | 10 | 11 | 12 |
|---|---|---|---|---|---|---|---|---|---|----|----|----|
| A |   |   |   | $10^5$ | $10^4$ | $10^3$ | $10^2$ | $10^1$ | $10^0$ |   |   |   |
| B |   |   |   | $10^5$ | $10^4$ | $10^3$ | $10^2$ | $10^1$ | $10^0$ |   |   |   |
| C |   |   |   | $10^5$ | $10^4$ | $10^3$ | $10^2$ | $10^1$ | $10^0$ |   |   |   |
| D |   |   |   | $10^5$ | $10^4$ | $10^3$ | $10^2$ | $10^1$ | $10^0$ |   |   |   |
| E |   |   |   | $10^5$ | $10^4$ | $10^3$ | $10^2$ | $10^1$ | $10^0$ |   |   |   |
| F |   |   |   | $10^5$ | $10^4$ | $10^3$ | $10^2$ | $10^1$ | $10^0$ |   |   |   |
| G |   |   |   | $10^5$ | $10^4$ | $10^3$ | $10^2$ | $10^1$ | $10^0$ |   |   |   |
| H |   |   |   | $10^5$ | $10^4$ | $10^3$ | $10^2$ | $10^1$ | $10^0$ |   |   |   |

# FIG. 58

# FIG. 59

GI reaction (Cy5 channel)

# FIG. 60

# FIG. 61

| Plate | (1) Nucleic acid + Primer + Amplifying reagent | | | | (2) Nucleic acid + Amplifying reagent | | | | (3) Nucleic acid | | | |
|---|---|---|---|---|---|---|---|---|---|---|---|---|
| Manual addition | − | | | | Primer | | | | Primer + Amplifying reagent | | | |
| | 1 | 2 | 3 | 4 | 5 | 6 | 7 | 8 | 9 | 10 | 11 | 12 |
| A | 10 | 10 | 10 | 10 | 10 | 10 | 10 | 10 | 10 | 10 | 10 | 10 |
| B | 10 | 10 | 10 | 10 | 10 | 10 | 10 | 10 | 10 | 10 | 10 | 10 |
| C | 10 | 10 | 10 | 10 | 10 | 10 | 10 | 10 | 10 | 10 | 10 | 10 |
| D | 10 | 10 | 10 | 10 | 10 | 10 | 10 | 10 | 10 | 10 | 10 | 10 |
| E | 10 | 10 | 10 | 10 | 10 | 10 | 10 | 10 | 10 | 10 | 10 | 10 |
| F | 10 | 10 | 10 | 10 | 10 | 10 | 10 | 10 | 10 | 10 | 10 | 10 |
| G | 10 | 10 | 10 | 10 | 10 | 10 | 10 | 10 | 10 | 10 | 10 | 10 |
| H | 10 | 10 | 10 | 0 | 10 | 10 | 10 | 0 | 10 | 10 | 10 | 0 |

# FIG. 62

| Plate | (1) Nucleic acid + Primer + Amplifying reagent | | | | (2) Nucleic acid + Amplifying reagent | | | | (3) Nucleic acid | | | |
|---|---|---|---|---|---|---|---|---|---|---|---|---|
| Manual addition | − | | | | Primer | | | | Primer + Amplifying reagent | | | |
| | 1 | 2 | 3 | 4 | 5 | 6 | 7 | 8 | 9 | 10 | 11 | 12 |
| A | 38.87 | 38.75 | 36.79 | 36.96 | 36.95 | 37.14 | 36.50 | 36.45 | 36.98 | 36.98 | 36.99 | 36.61 |
| B | 38.08 | 36.98 | 36.26 | 36.38 | 36.61 | 37.57 | 37.06 | 37.36 | 37.98 | 36.83 | 36.87 | 36.75 |
| C | 37.07 | 36.71 | 36.68 | 36.49 | 36.54 | 37.02 | 36.66 | 36.15 | 36.69 | 37.45 | 37.20 | 36.76 |
| D | 36.56 | 36.34 | 36.80 | 36.92 | 36.67 | 36.54 | 36.19 | 37.03 | 36.38 | 36.82 | 37.75 | 36.52 |
| E | 36.53 | 37.59 | 36.21 | 37.08 | 36.83 | 36.64 | 36.45 | 35.69 | 36.09 | 36.33 | 36.59 | 36.97 |
| F | 36.85 | 37.41 | 36.68 | 37.17 | 36.66 | 37.51 | 37.32 | 37.00 | 36.49 | 36.68 | 36.89 | 37.10 |
| G | 36.97 | 37.30 | 37.00 | 37.06 | 36.69 | 36.53 | 36.42 | 36.98 | 35.96 | 36.44 | 37.23 | 37.36 |
| H | 37.10 | 36.93 | 37.51 | 0 | 36.21 | 36.95 | 36.54 | 0 | 37.26 | 36.90 | 36.44 | 0 |

# FIG. 63

| Plate | (1) | (2) | (3) |
|---|---|---|---|
| Ct value (Average value) | 36.85 | 36.74 | 37.03 |
| Standard deviation σ | 0.44 | 0.42 | 0.62 |

# FIG. 64

Normal distribution curve of Ct value

## INTERNATIONAL SEARCH REPORT

| | International application No. |
|---|---|
| | PCT/JP2018/042044 |

**A. CLASSIFICATION OF SUBJECT MATTER**
Int.Cl. C12M1/00(2006.01)i, C12N15/10(2006.01)n, C12Q1/686(2018.01)n

According to International Patent Classification (IPC) or to both national classification and IPC

**B. FIELDS SEARCHED**

Minimum documentation searched (classification system followed by classification symbols)
Int.Cl. C12M1/00, C12N15/10, C12Q1/686

Documentation searched other than minimum documentation to the extent that such documents are included in the fields searched
Published examined utility model applications of Japan 1922–1996
Published unexamined utility model applications of Japan 1971–2019
Registered utility model specifications of Japan 1996–2019
Published registered utility model applications of Japan 1994–2019

Electronic data base consulted during the international search (name of data base and, where practicable, search terms used)
JSTPlus/JMEDPlus/JST7580 (JDreamIII),
CAplus/REGISTRY/MEDLINE/EMBASE/BIOSIS/WPIDS (STN)

**C. DOCUMENTS CONSIDERED TO BE RELEVANT**

| Category* | Citation of document, with indication, where appropriate, of the relevant passages | Relevant to claim No. |
|---|---|---|
| X<br>Y | PUGLIESE, N. et al., "Validation of a seminested PCR approach for rapid detection of Salmonella enterica subsp. enterica serovar Gallinarum", Journal of Microbiological Methods, 2011, vol. 85, pp. 22-27, materials and methods | 1, 4, 5<br>1-5 |
| X<br>Y | JP 11-69999 A (ORTHO-CLINICAL DIAGNOSTICS, INC.) 16 March 1999, example 4, paragraph [0055] & US 2001/0019822 A1, example 4, paragraph [0060] & EP 887425 A2 | 1, 4, 5<br>1-5 |

☒ Further documents are listed in the continuation of Box C. ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 08 February 2019 (08.02.2019) | 26 February 2019 (26.02.2019) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

**INTERNATIONAL SEARCH REPORT**

International application No.

PCT/JP2018/042044

C (Continuation).    DOCUMENTS CONSIDERED TO BE RELEVANT

| Category* | Citation of document, with indication, where appropriate, of the relevant passages | Relevant to claim No. |
|---|---|---|
| X<br>Y | 白土佳子ら, COBAS AMPLICOR(TM)法における Mycobacterium 属菌検出に関する各種核酸抽出法の検討, 臨床病理, 2001, Vol. 49, no. 6, pp. 608-612, entire text, in particular, page 608, right column, line 2 to page 609, left column, line 4, page 609, right column, lines 14-33, (SHIRATSUCHI, Yoshiko et al., "Significance of Nucleic Acid Extraction for the Performance of COBAS AMPLICOR Mycobacterium-PCR Assay", The Japanese journal of clinical pathology) | 1, 4, 5<br>1-5 |
| X<br>Y | 日和佐 敬一, PCR 検査用日動測定装置「コバスアンプリコア」による Neisseria gonorrhoeae 測定の基礎的検討, 医学検査, 1998, vol. 47, no. 5, pp. 52-55, entire text, in particular, table 3, page 55, left column, paragraph [0002], (HIWASA, Keiichi, "Basic evaluation of automated (COBASAMPLICOR) PCR system for detection of Neisseria gonorrhoeae.", Japanese Journal of Medical Technology) | 1, 4, 5<br>1-5 |
| Y | JP 2009-515531 A (GEN-PROBE INCORPORATED) 16 April 2009, paragraphs [0007], [0103], [0104], examples & US 2007/0111246 A1 & WO 2007/059179 A1, pp. 2-8, pp. 37-51, tables 1-6 & EP 2273404 A1 | 1-5 |
| Y<br>A | 大河内恵ら, ノロウイルス遺伝子検査＜糞便直接 RT-PCR 法について＞, 広島県臨床検査技師会誌, 2009, no. 104, pp. 33-36, entire text, in particular, page 33, "measurement principle, features" to page 34, "result", page 36, fig. 1, non-official translation (OKOUCHI, Megumi et al., "Norovirus genetic test <About stool using direct RT-PCR method>", Journal of Hiroshima Association of Medical Technologists) | 3<br>1, 2, 4, 5 |
| Y<br>A | JP 2016-19495 A (TOYOBO CO., LTD.) 04 February 2016, claims, examples (Family: none) | 3<br>1, 2, 4, 5 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014033658 A
- JP 2015195735 A
- JP 2008141965 A
- JP 2011223940 A
- JP 9224699 A
- WO 20020349439 A

**Non-patent literature cited in the description**

- Guidance for Industry: Bioanalytical Method Validation. U.S. Food and Drug Administration, 05 September 2014
- Guideline on Bioanalytical Method Validation. European Medicines Agency, 05 September 2014
- **SANGJUN et al.** *PLoS One,* vol. 6 (3), e17455

SEQUENCE LISTING

<110>  Ricoh Company, Ltd.

<120>  Device for detection determination

<130>  N-RC015-18P

<150>  JP 2017-218552
<151>  2017-11-13

<150>  JP 2017-224014
<151>  2017-11-21

<150>  JP 2017-226081
<151>  2017-11-24

<150>  JP 2017-226082
<151>  2017-11-24

<150>  JP 2017-226084
<151>  2017-11-24

<150>  JP 2017-226086
<151>  2017-11-24

<160>  11

<210>  1
<211>  600
<212>  DNA
<213>  Artificial Sequence

<400>  1
attcgaaggg tgattggatc ggagatagga tgggtcaatc gtagggacaa tcgaagccag     60
aatgcaaggg tcaatggtac gcagaatgga tggcacttag ctagccagtt aggatccgac    120
tatccaagcg tgtatcgtac ggtgtatgct tcggagtaac gatcgcacta agcatggctc    180
aatcctaggc tgataggttc gcacatagca tgccacatac gatccgtgat tgctagcgtg    240
attcgtaccg agaactcacg ccttatgact gcccttatgt caccgcttat gtctcccgag    300
atcacacccg ttatctcagc cctaatctct gcggtttagt ctggccttaa tccatgcctc    360
atagctaccc tcataccatc gctcatacct tccgacattg catccgtcat tccaaccctg    420
attcctacgg tctaacctag cctctatcct acccagttag gttgcctctt agcatccctg    480
ttacgtacgc tcttaccatg cgtcttacct tggcactatc gatgggagta tggtagcgag    540
tatggaacgg actaacgtag gcagtaagct agggtgtaag gttgggacta aggatgccag    600

<210>  2
<211>  85
<212>  DNA
<213>  Norwalk virus(GI)

<400>  2
cgctggatgc gcttccatga cctcggattg tggacaggag atcgcgatct tctgcccgaa     60
ttcgtaaatg atgatggcgt ctaag                                           85

<210>  3
<211>  98
<212>  DNA
<213>  Norwalk virus(GII)

<400>  3
caagagccaa tgttcagatg gatgagattc tcagatctga gcacgtggga gggcgatcgc     60

```
aatctggctc ccagctttgt gaatgaagat ggcgtcga                                98


<210>   4
<211>   85
<212>   DNA
<213>   Norwalk virus(GI)


<400>   4
cgctggatgc gcttccatga cctcggattg tggacaggag atcgcgatct tctgcccgaa        60
ttcgtaaatg atgatggcgt ctaag                                              85


<210>   5
<211>   98
<212>   DNA
<213>   Norwalk virus(GII)


<400>   5
caagagccaa tgttcagatg gatgagattc tcagatctga gcacgtggga gggcgatcgc        60
aatctggctc ccagctttgt gaatgaagat ggcgtcga                                98


<210>   6
<211>   130
<212>   DNA
<213>   Artificial Sequence


<400>   6
atgtatgtcc caggatggca ggccatgttc cgctggatgc gcttccatga cctcggattg        60
tggacaggag atcgcgatct tctgcccgaa ttcgtaaatg atgatggcgt ctaaggacgc        120
tacatcaagc                                                               130


<210>   7
<211>   143
<212>   DNA
<213>   Artificial Sequence


<400>   7
ttttacgtgc ccagacaaga gccaatgttc agatggatga gattctcaga tctgagcacg        60
tgggagggcg atcgcaatct ggctcccagc tttgtgaatg aagatggcgt cgaatgacgc        120
caacccatct gatgggtccg cag                                                143


<210>   8
<211>   20
<212>   DNA
<213>   Artificial Sequence


<400>   8
cgytggatgc gnttycatga                                                    20


<210>   9
<211>   22
<212>   DNA
<213>   Artificial Sequence


<400>   9
cttagacgcc atcatcatty ac                                                 22


<210>   10
```

```
<211>   26
<212>   DNA
<213>   Artificial Sequence

<400>   10
cargarbcna tgttyagrtg gatgag                                                    26


<210>   11
<211>   21
<212>   DNA
<213>   Artificial Sequence

<400>   11
tcgacgccat cttcattcac a                                                         21
```